(19) Europäisches Patentamt
European Patent Office
Office européen des brevets

(11) **EP 4 356 750 A2**

(12) # EUROPEAN PATENT APPLICATION

(43) Date of publication:
**24.04.2024 Bulletin 2024/17**

(21) Application number: **23213974.1**

(22) Date of filing: **29.07.2022**

(51) International Patent Classification (IPC):
**A23K 50/15** [(2016.01)]

(52) Cooperative Patent Classification (CPC):
**A23K 50/10; A23K 10/18; A23K 10/30;
A23K 10/33; A23K 20/158; A23K 20/189;
A23K 20/20; A23K 20/22; A23K 20/24;
A23K 40/35; A23K 50/15;** Y02P 60/22

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.07.2021 GB 202111040**

(62) Document number(s) of the earlier application(s) in
accordance with Art. 76 EPC:
**22754891.4 / 4 351 357**

(71) Applicant: **Glasport Bio Limited
Galway (IE)**

(72) Inventors:
- **O'FLAHERTY, Vincent
  Galway (IE)**
- **THORN, Camilla
  Galway (IE)**
- **LEE, Chui Sang
  Galway (IE)**
- **FRIEL, Ruairi
  Galway (IE)**

(74) Representative: **Carpmaels & Ransford LLP
One Southampton Row
London WC1B 5HA (GB)**

Remarks:
This application was filed on 12-01-2024 as a
divisional application to the application mentioned
under INID code 62.

(54) **COMPOSITIONS AND METHODS**

(57) The invention generally relates to compositions and methods for reducing methane production in animals, in particular by controlling oxidation-reduction potential in the digestive tract.

EP 4 356 750 A2

**Description**

**Technical Field**

This invention relates to compositions and methods for reducing greenhouse gas production from animals. In particular, the invention generally relates to new methods of reducing methane production from animals by controlling oxidation-reduction potential in the digestive tract of animals, and compositions useful in such methods comprising oxidising agents.

**Background and Related Art**

There is significant concern in the agricultural industry regarding the high production of greenhouse gases, in particular methane, from the rearing of animals, for example from ruminant animals and in particular from cattle for the beef and dairy industries. Methanogens such as methanogenic microorganisms (e.g. methanogenic archaea), residing in the rumen of these animals produce methane from the breakdown of ingested food. According to a 2006 United Nations' Food and Agricultural Organization report, the livestock sector, a large proportion of which is cows, generates 18% more greenhouse gas emissions (as measured in $CO_2$ equivalent) than the transport sector.

Therefore, there is a need reduce the carbon footprint of the agricultural industry.

Compositions and their use in methods of reducing methane production of animals are known in the art, for instance in WO 2020/074672, but such efforts have focused on different compositions and different methods, e.g. involving iodide. Some previous efforts have also focused on aggressive approaches e.g. defaunation and/or high concentrations of peroxide and/or iodide, sometimes generating or relying on reactive species. However, defaunation methods can have unfavourably negative effects on digestibility (and can have negative impacts on animal performance and feed efficiency). There remains a need for new compositions and new methods for reducing the greenhouse gas production from animals.

Moreover, in general in the agricultural industry, it is advantageous if residues of contaminants and prohibited substances are either not present in animal products destined for the human food chain, or are present at such a level that adverse effects on the health of consumers cannot occur. This includes meat, meat products, milk derived from the main food species (e.g. bovines, sheep/goats, pigs and poultry). For example, in the case of chemical residues, such as organochlorine compounds, bromoform and organophosphorus compounds, animals are only suitable for food production if the levels of residues of such contaminants in edible products are below levels that could pose a health risk for consumers. Accordingly, maximum residue limits/levels (MRLs) in meat and dairy products are defined for various contaminants by regulatory bodies, for example under EU law (e.g. Directive 96/23/EC).

For example, one of the issues with the use of seaweeds, or seaweed extracts in the feed of animals to reduce methane emissions is the presence of bromoform residues, which are toxic. Other ingredients in seaweeds or plant extracts may also negatively affect the food value or acceptability of animal products destined for the human food chain.

Some food ingredients do not have MRLs but must still be considered. Iodine, for example, is an essential nutrient for humans, but there is some evidence that excess iodine consumption can have negative health impacts. Consequently, daily dietary intake guidelines have been developed for iodine. It is advantageous for animal products destined for the human food chain to not have elevated iodine levels, to the extent that consumption of normal amounts of the products would exceed daily intake guidelines. There remains a need for new compositions and new methods for reducing the greenhouse gas production from animals which permit such ingredients to be absent.

**Definitions**

The term "comprising" encompasses "including" as well as "consisting" e.g. a composition "comprising" X may consist exclusively of X or may include something additional e.g. X + Y. The term "comprising" used herein also encompasses "consisting essentially of", e.g. a composition "comprising" X may consist of X and any other components that do not materially affect the essential characteristics of the composition. Alternatively or in addition "consisting essentially of" can mean, for example, making up 95% or more of a component.

The term "ORP" means oxidation-reduction potential which can be measured by conventional means, e.g. using an ORP meter. See below for an illustrative assay method in animals.

The terms "methanogenesis" and "methane production" are used interchangeably and in the present context both mean the bioproduction of methane ($CH_4$), for example by microorganisms e.g. archaea or other organisms. The term "methanogen" refers to an organism (e.g. microorganisms, such as methanogenic archaea) which produces methane ($CH_4$).

The term "source of" can mean a substance or composition which directly or indirectly provides/releases a chemical entity (e.g. an ion or a molecule such as peroxide), for example by chemical or physical dissociation, dissolution and/or chemical or biochemical decomposition (e.g. in the digestive tract of an animal, such as the rumen of a ruminant). For example, hydrogen peroxide, urea hydrogen peroxide, magnesium peroxide, calcium peroxide, and combinations thereof are all sources of oxygen and in particular, sources of peroxide. The term "source of" may also encompass the substance itself, for example "source of X" may be "X" *per se.*

The term "active ingredient" means the component primarily responsible for the biological effect (e.g. the methane reduction). It generally does not include excipients, fillers, food and the like, which do not have a methane reducing effect (at least as their main function).

The term "iodide" can mean iodide ions (I⁻) and can also mean a source of iodide ions, for example an iodide salt (e.g. KI).

The term "defaunation" means a reduction in the population or elimination of fauna in an ecological system, for example a substantial reduction in the population or elimination of microorganisms (e.g. protozoa) present in the stomach e.g. rumen of an animal. This normally refers to a substantial reduction in the population rather than a minor reduction which is secondary to another effect.

The term "digestibility" means the proportion, for example by weight, of foodstuff taken into the digestive tract of an animal which is absorbed into the body. Digestibility can be measured by conventional means. For example digestibility can be calculated from the difference in weight of foodstuff taken in by an animal and the residual foodstuff which is excreted having not been metabolised and/or absorbed.

The term "oxidising agent" can mean anything (e.g. any compound, composition, or biological system) that increases ORP. Various oxidising agents are known in the art.

The term "source of oxygen" means a compound or composition which releases oxygen, for example by decomposition of peroxide or peroxide ions in solution. Release of oxygen from a peroxide or a source of peroxide may optionally be catalysed by an organism (e.g. a yeast) an enzyme (e.g. a peroxidase or catalase) or a metal (e.g. iron). Various sources of oxygen are known in the art.

The term "peroxide" can mean "peroxide ions" ($O_2^{2-}$) and can also mean a source of peroxide ions (e.g. hydrogen peroxide, urea hydrogen peroxide, magnesium peroxide, calcium peroxide, sodium percarbonate, sodium peroxide, lithium peroxide and combinations thereof).

The term "nitrate" can mean nitrate ions ($NO_3^-$) and can also mean a source of nitrate ions, for example a nitrate salt (e.g. NaNOs).

The terms "digestive tract" in the context of the present invention normally means the single component of the digestive tract where most methane production occurs. This is normally the stomach (for example the rumen in ruminants), but can be the large intestine in some animals. This might be the first chamber of the alimentary canal. The terms "digestive tract" (and "stomach" and "rumen") in the context of the present invention do not normally include the mouth or the oesophagus. For example when discussing an increase in ORP this may concern only effects in a stomach and/or large intestine and/or small intestine. Throughout the specification, preferred embodiments are the methods and compositions described, where the term "digestive tract" is replaced with "stomach", or is replaced with the term "rumen". In some embodiments for non-ruminant animals, the methods are as described herein except that the "stomach" is replaced with "large intestine". In the most preferred embodiment, the invention is adapted to act in the rumen of a ruminant.

The terms "elevating ORP" and "increasing ORP" are used interchangeably and mean raising ORP to be more oxidising than beforehand. The normal level could for example be measured in a system (e.g. in the digestive tract of an animal) prior to administration of a composition of the invention or in a reference system in the absence of a composition of the invention. Increasing ORP can mean increasing $E_h$ to a more positive value relative to normal. Further target increases are described herein.

The term "$C_{max}$" means the maximum (or peak) concentration of a substance, compound, ion, etc. achieved, normally in the present context in the digestive tract of the animal or in a specific part of the digestive tract (e.g. the rumen of a ruminant, or a specific part of the rumen) and $C_{max}$ can be measured for any fluid (e.g. rumen fluid) from that part of the body.

The term "without reducing digestibility" can mean no reduction in digestibility but can also encompass a slight reduction in digestibility e.g. without substantially reducing digestibility.

Generally throughout this application the term "substantially" can mean a deviation of not more than about 25%; preferably not more than about 20%; preferably not more than about 15%; preferably not more than about 10%; most preferably not more than about 5%. For example, the term "substantially" can mean that deviation from a particular value is not more than about 15%.

Generally the term "about" can mean a deviation of not more than 25%; preferably not more than 20%; preferably not more than 15%; preferably not more than 10%; most preferably not more than 5%. For example, the term "about" can mean that deviation from a particular value is not more than 15%.

"Administration" is normally oral, and includes feeding animals, the animals feeding themselves, and in some embodiments administration of a particular dosage form, e.g. a bolus.

## Brief Description of the Figures

**Figure 1** shows the effect of different compositions on gas production and in particular methane production in a simulated rumen model.

**Figure 2** shows the effect of different compositions on methane production volume in a simulated rumen model.

**Figure 3** shows the effect of different compositions on the total volume of gas produced in a simulated rumen model.

**Figure 4** shows the effect of different compositions on the daily amount of methane produced in a simulated rumen model.

**Figure 5** shows the effect of different compositions on the amount of ammonia produced in a simulated rumen model.

**Figure 6** shows the effect of different compositions on the acidity of rumen fluids in a simulated rumen model.

**Figure 7** shows the effect of different compositions on dry-matter digestibility in a simulated rumen model.

**Figure 8** shows the effect of different compositions on dry-matter digestibility of forage-based feed in a simulated rumen model.

**Figure 9** shows the effect of different compositions on dry-matter digestibility of the feed composition in a simulated rumen model.

**Figure 10** shows the effect of different compositions on average dry-matter digestibility across feed compositions and forage-based feed in a simulated rumen model.

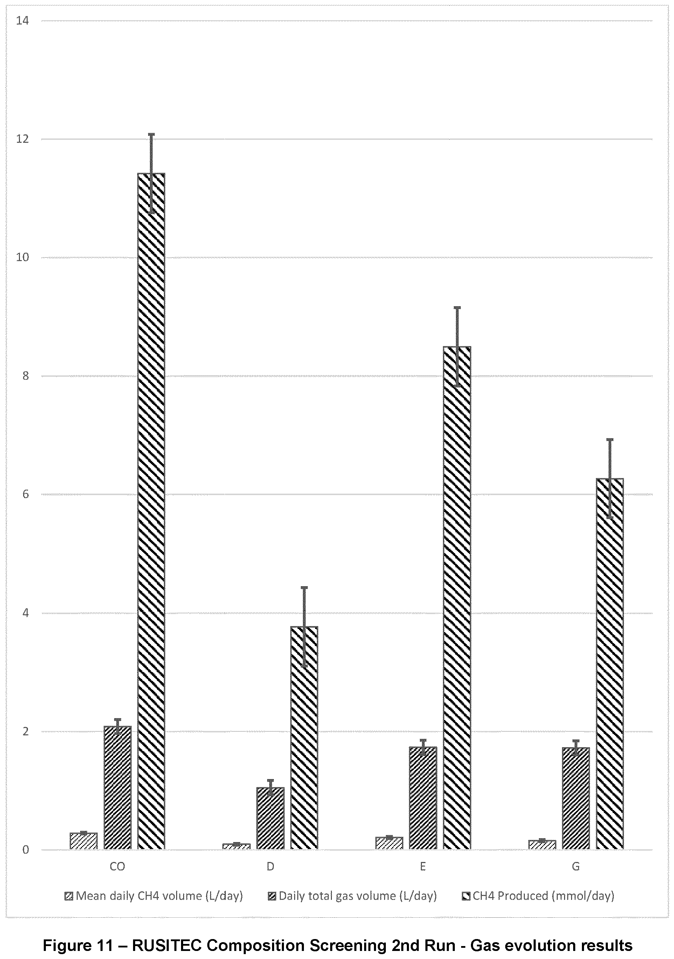

**Figure 11** shows the effect of different compositions on gas production and in particular methane production in a simulated rumen model.

**Figure 12** shows the effect of different compositions on methane production volume in a simulated rumen model.

**Figure 13** shows the effect of different compositions on the total volume of gas produced in a simulated rumen model.

**Figure 14** shows the effect of different compositions on the daily amount of methane produced in a simulated rumen model.

**Figure 15** shows the effect of different compositions on the acidity of rumen fluids in a simulated rumen model.

**Figure 16** shows the effect of different compositions on dry-matter digestibility in a simulated rumen model.

**Figure 17** shows the effect of different compositions on dry-matter digestibility of forage-based feed in a simulated rumen model.

**Figure 18** shows the effect of different compositions on dry-matter digestibility of the feed composition in a simulated rumen model.

**Figure 19** shows the effect of different compositions on average dry-matter digestibility across feed compositions and forage-based feed in a simulated rumen model.

**Figure 20** shows the *in vivo* ORP level over time in the rumen of cows in control, low-dose $CaO_2$, and high-dose $CaO_2$ groups.

**Figure 21** shows the measured rumen ORP levels in studied cattle as a function of time from 0 to 50 hours.

**Figure 22** shows the measured rumen ORP levels in studied cattle as a function of time from 50 to 100 hours.

**Figure 23** shows the effect of different oxidising agents on ORP as a function of time.

The figures are described in more detail in the examples of this application which discuss the relevant experimental details and results.

## Summary of the invention

The inventors have discovered new compositions and new methods which reduce methane production while not having a substantial negative impact on digestibility in animals. In particular the new compositions and new methods reduce methane production without significant defaunation of the digestive tract e.g. rumen. Some protozoa harbour methanogenic archaea as endosymbionts and some methane is produced by these archaea. Defaunation may reduce the population of protozoa. However, it has surprisingly been found that increasing oxygen supply and ORP can decrease methane production despite protozoa being known to consume oxygen. Compositions and methods of the invention might also improve feed efficiency in animals by inhibiting methanogenesis and making more of the ingested food available to the animal rather than methanogens in the digestive tract.

In one aspect the invention relates to a method of reducing methane production from an animal by increasing the oxidation-reduction potential in the stomach of the animal, the method comprising orally administering a composition comprising an oxidising agent to the animal. The inventors have found maintaining oxidation-reduction potential (ORP) within a favourable range to be a completely new and surprisingly effective approach for reducing methane production, without negatively affecting digestive functions. Previous efforts have focused on aggressive approaches e.g. defaunation and/or high concentrations of peroxide, or producing reactive species, without intention to control ORP.

In a further aspect the invention relates to a method of reducing methane production from an animal while increasing ammonia production in the animal, the method comprising orally administering a composition comprising an oxidising agent to the animal, wherein the oxidising agent comprises urea hydrogen peroxide. It was surprisingly discovered that the method simultaneously reduces methane production in ruminants whilst also promoting ammonia production. In particular, the inventors have observed that the oxidising agent urea hydrogen peroxide (UHP) is particularly effective in reducing methane production from animals while also increasing ammonia production in the animal.

In a further aspect, the invention relates to a method of reducing methane production from an animal, the method comprising orally administering a composition comprising an oxidising agent to the animal, wherein the composition is administered to the animal in a dose which provides an increase in the oxidation-reduction potential of the stomach of the animal of at least + 100 mV for at least 1 hours. The inventors have discovered that these doses are particularly effective or optimal for the methods of the invention (e.g. reducing methane production in a ruminant) these doses can reduce methane production without negatively impacting digestibility.

In a further aspect, the invention relates to a composition in the form of a bolus for oral administration to an animal comprising: (i) an oxidising agent; (ii) excipients; and (iii) a coating. The inventors have surprisingly discovered that these compositions can be orally administered on a regular basis to achieve the desired reduction in methane production from animals (e.g. a sustained reduction in methane production from ruminants for a period of days).

In a further aspect, the invention relates to a composition in the form of pellets comprising: (i) urea hydrogen peroxide or magnesium peroxide or a combination thereof; (ii) food components; and (iii) a coating. The inventors have discovered that a coating can protective the active ingredients from environmental factors before use and/or from saliva during oral administration and can sustain release of the oxidising agent.

## Detailed Description

This application has been drafted into sections. However, the sections should not be read in isolation. Unless otherwise specified, each section is to be read in combination with the other sections i.e. taking the entire application as a whole. This means, for example, that all of the compositions described in the "Compositions" section are intended to be read in combination with the "Methods" section (i.e. preferred compositions are suitable for the methods and preferred methods use the compositions described herein). The various optional and preferred features can also be combined, even when taken from different parts of the specification. Likewise all "aspects" and "embodiments" can be combined. No separation of embodiments is intended, unless explicitly stated.

The invention generally provides a method of increasing ORP. In particular, methods of the invention may comprise elevating ORP in the digestive tract of an animal, for example in the stomach, for example, in the rumen of a ruminant. This is normally achieved using oxidising agents. For example the invention provides a method of increasing oxygen supply in the digestive tract to increase ORP and achieve a desired ORP range. The methods of the invention reduce methane production from animals.

Methods of the invention may generally involve inhibiting methanogens present in the digestive tract of an animal (e.g. ruminant) and in particular, inhibiting the methanogenic activity (i.e. methane production) of methanogens (e.g. methanogens present in the rumen of a ruminant). Without wishing to be bound by theory, elevating ORP in the digestive tract of an animal, in particular elevating ORP to be within a desired range, may inhibit methanogens (i.e. may inhibit the methanogenic activity of microorganisms), for example by disfavouring methanogenic metabolic pathways. In some embodiments, the methanogens are methanogenic archaea.

The methods of the invention are normally non-therapeutic. They are not therefore principally aimed at prophylactic or therapeutic treatment of disease (although the invention does not preclude beneficial health effects alongside the desired effect of reducing methane production).

### *ORP levels:*

Methanogenesis usually requires an ORP or redox of about -200 mV or lower. A normal average ORP in a rumen is well below about -100 mV, for example in dairy cows the average rumen ORP may be about -200 mV, and methanogenesis therefore occurs, particularly when there are transient fluctuations down to lower ORP, e.g. in the period after eating. The methods of the invention may increase average ORP and thus reduce the frequency of fluctuations which reach methanogenic levels of ORP (and thus reduce methanogenesis). The methods of the invention may also reduce fluctuations to methanogenic levels in the period after eating.

Increasing ORP generally means increasing average ORP above the naturally occurring level (e.g. above about -200 mV in ruminants). The increase should desirably be not too high such that it has negative effects on digestibility. For instance it may be desirable to avoid a sustained (e.g. for at least 1 hour) increase of above a threshold of about +500mV over baseline. However, a transient increase above this threshold may be tolerated.

A transient increase lasts only a short period of time, e.g. less than about 1 hour; preferably less than about 30 minutes; preferably less than about 15 minutes.

The invention may elevate ORP above baseline by at least about +10mV; at least about +50mV; at least about +100 mV; at least about +150 mV; at least about +200 mV; at least about +250 mV; and/or or at least about +300 mV. For example, the invention may elevate ORP in the digestive tract of an animal (e.g. the rumen of a ruminant) by at least about +100 mV (e.g. from about -200 mV at baseline to about -100mV after administration).

In some embodiments, the invention elevates ORP above a particular potential in the digestive tract of an animal (e.g. the rumen of a ruminant). In particular, the invention may increase ORP to at least about -200 mV; at least about -150 mV; at least about -100 mV; at least about -50 mV; at least about 0 mV; at least about 50 mV; and/or at least about 100 mV. For example, the invention may preferably increase ORP in the digestive tract of an animal (e.g. the rumen of a ruminant) to at least about -100 mV.

In preferred embodiments, the invention controls ORP in the digestive tract by increasing ORP to be within a particular desired range over a sustained period. In particular, the invention may increase average ORP in the digestive tract to a range of from about -200 mV to about +250 mV for a period of time; preferably from about -200 mV to about +200 mV; preferably from about -200 mV to about +150 mV; preferably from about -150 mV to about +150 mV; preferably from about -100 mV to about +150 mV; preferably from about -100 mV to about +100 mV; preferably from about -100 mV to about +50 mV; preferably from about -100 mV to about 0 mV. For instance, the invention may preferably control ORP in the digestive tract by increasing ORP to a range from about -100 mV to about +150 mV for a period of time. The period of time is described elsewhere herein, and is normally sustained, e.g. it can be for several hours, days or even weeks.

In further preferred embodiments, the invention may increase average ORP in the digestive tract to a range which is not above 0mV. For instance in some embodiments the invention increases average ORP in the digestive tract to a range which is from about -300 mV to about 0 mV for a period of time; preferably from about -250 mV to about 0 mV; preferably from about -200 mV to about 0 mV; preferably from about -150 mV to about 0 mV; preferably from about -100 mV to about 0 mV. preferably from about -50 mV to about 0 mV; preferably from about -100 mV to about +50 mV; preferably from about -100 mV to about 0 mV. In some embodiments the invention increases average ORP in the digestive tract to a range which is from about -300 mV to about -50 mV for a period of time; preferably from about -250 mV to about -50 mV; preferably from about -200 mV to about -50 mV; preferably from about -150 mV to about -50 mV; preferably from about -100 mV to about -50 mV. preferably from about -50 mV to about -50 mV; preferably from about -100 mV to about +50 mV; preferably from about -100 mV to about -50 mV. In some embodiments the invention increases average ORP in the digestive tract to a range which is from about -300 mV to about -100 mV for a period of time; preferably from about -250 mV to about -100 mV; preferably from about - 200 mV to about -100 mV; preferably from about -150 mV to about -100 mV. As an example, the invention may increase average ORP in the digestive tract to a range which is from about -200 mV to about -100 mV for at least 1 hour. The period of time is described elsewhere herein, and is normally sustained, e.g. it can be for several hours, days or even weeks.

In some embodiments, the average ORP or baseline ORP in the rumen of a ruminant might be from about -500 mV to about -200 mV. For example, the average ORP or baseline ORP in the rumen of a dairy cow or beef cow might be from about -500 mV to about -200mV. However, in some embodiments the average ORP or baseline ORP might vary, depending on for example, the particular species and/or breed of ruminant and/or the diet of the ruminant. ORP in the rumen can be measured by conventional means, e.g. using an ORP meter. See below for an illustrative assay method in animals.

*ORP profile in use:*

Methods and compositions of the invention are generally capable of elevating ORP in the digestive tract for a sustained period of time. For example, the period can be at least about 1 hour; preferably at least about 2 hours; preferably at least about 3 hours; preferably at least about 4 hours, preferably at least about 5 hours; preferably at least about 6 hours; preferably at least about 12 hours. For example the period can be at least about 1 hour. In some embodiments the period of time is even longer, e.g. at least about 1 day; at least about 1 week; at least about 2 weeks; or at least about 1 month. For example the period can be at least about 1 week. Longer periods are particularly applicable using coated compositions or bolus compositions. Longer periods can, for example, have the advantage of avoiding the need for daily feeding

It is often favourable to increase ORP for at least about 2 hours, particularly when the composition is given at the same time as food, since this can coincide with the period of when methanogenesis is highest in the day (i.e. in the 2 hours after feeding).

In some embodiments, the invention maintains the desired ORP range in the digestive tract of an animal (e.g. the rumen of a ruminant) for a sustained period (e.g. for at least about 1 hour, preferably at least about 2 hours). In particular, the invention may maintain an ORP in a range of from about -200mV to about +300mV; from about -200 mV to about +250 mV; from about -200 mV to about +200 mV; from about -200 mV to about +150 mV; from about -150 mV to about +150 mV; from about -100 mV to about +150 mV; from about -100 mV to about +50 mV; and/or from about -100 mV to about 0 mV; preferably from about -100 mV to about +150 mV. For example, the invention may maintain an ORP range in the digestive tract of an animal (e.g. the rumen of a ruminant) in a range of from about -100 mV to about +150 mV for a sustained period (e.g. for at least 1 hour).

The ORP might be sustained within a window which is the maximum ORP +/- about 100mV, for a period of time, e.g. for at least about 1 hour.

The ORP in the digestive tract of the animal is normally elevated for a period of time after administration. Normally it is elevated by at least +100 mV. This is normally for at least about 1 hour, preferably for at least about 2 hours, preferably for at least about 3 hours. For example, the ORP may be elevated by at least about +100 mV for at least about 2 hours. The ORP may be elevated in a particular component of the digestive tract, for example the main stomach component of the digestive tract, e.g. the rumen in ruminants. The increase could be measured at a single point in time or over a period of time, e.g. taking an average. This can be measured by conventional means for example by the method described in Example 3.

In some embodiments, the invention provides a preferable ORP profile in the digestive tract of an animal. The invention might increase ORP by about +50 mV (from initial to the peak ORP) and the ORP may remain elevated by about +50 mV for an initial period of at least about 1 hour; preferably the invention increases ORP by about +100 mV (from initial to the peak ORP) and the ORP remains elevated by about +100 mV for an initial period of at least about 1 hour; preferably the invention increases ORP by about +150 mV (from initial to the peak ORP) and the ORP remains elevated by about +150 mV for an initial period of at least 1 hour; preferably the invention increases ORP by about +200 mV (from initial to the peak ORP) and the ORP remains elevated by about +200 mV for an initial period of at least about 1 hour; preferably the invention increases ORP by about +250 mV (from initial to the peak ORP) and the ORP remains elevated by about +250 mV for an initial period of at least about 1 hour; preferably the invention increases ORP by about +300 mV (from initial to the peak ORP) and the ORP remains elevated by about +300 mV for an initial period of at least about 1 hour; preferably the invention increases ORP by about +350 mV (from initial to the peak ORP) and the ORP remains elevated by about +350 mV for an initial period of at least about 1 hour. For example the invention might increase ORP (e.g. in the rumen of a ruminant) by about +100 mV (from initial to the peak ORP) and the ORP may remain elevated by about +100 mV for an initial period of at least about 1 hour.

In some embodiments, the invention provides a ORP profile in the digestive tract of an animal which is suitable for reducing methane production. The invention might increase ORP by about +50 mV (from initial to the peak ORP) and the ORP may remain elevated by about +50 mV for an initial period of at least about 2 hours; preferably the invention increases ORP by about +100 mV (from initial to the peak ORP) and the ORP remains elevated by about +100 mV for an initial period of at least about 2 hours; preferably the invention increases ORP by about +150 mV (from initial to the peak ORP) and the ORP remains elevated by about +150 mV for an initial period of at least about 2 hours; preferably the invention increases ORP by about +200 mV (from initial to the peak ORP) and the ORP remains elevated by about +200 mV for an initial period of at least about 2 hours; preferably the invention increases ORP by about +250 mV (from initial to the peak ORP) and the ORP remains elevated by about +250 mV for an initial period of at least about 2 hours; preferably the invention increases ORP by about +300 mV (from initial to the peak ORP) and the ORP remains elevated by about +300 mV for an initial period of at least about 2 hours; preferably the invention increases ORP by about +350 mV (from initial to the peak ORP) and the ORP remains elevated by about +350 mV for an initial period of at least about 2 hours. For example the invention might increase ORP (e.g. in the rumen of a ruminant) by about +100 mV (from initial to the peak ORP) and the ORP may remain elevated by about +100 mV for an initial period of at least about 2 hours.

In some embodiments, the invention provides a preferable ORP profile in the digestive tract of an animal. The invention might increase ORP by about +50 mV (from initial to the peak ORP) and the ORP may remain elevated by about +50 mV for an initial period of at least about 3 hours; preferably the invention increases ORP by about +100 mV (from initial to the peak ORP) and the ORP remains elevated by about +100 mV for an initial period of at least about 3 hours; preferably the invention increases ORP by about +150 mV (from initial to the peak ORP) and the ORP remains elevated by about +150 mV for an initial period of at least about 3 hours; preferably the invention increases ORP by about +200 mV (from initial to the peak ORP) and the ORP remains elevated by about +200 mV for an initial period of at least about 3 hours; preferably the invention increases ORP by about +250 mV (from initial to the peak ORP) and the ORP remains elevated by about +250 mV for an initial period of at least about 3 hours; preferably the invention increases ORP by about +300 mV (from initial to the peak ORP) and the ORP remains elevated by about +300 mV for an initial period of at least about 3 hours; preferably the invention increases ORP by about +350 mV (from initial to the peak ORP) and the ORP remains elevated by about +350 mV for an initial period of at least about 3 hours. For example the invention might increase ORP (e.g. in the rumen of a ruminant) by about +100 mV (from initial to the peak ORP) and the ORP may remain elevated by about +100 mV for an initial period of at least about 3 hours.

In some embodiments the increase in ORP is not excessive. It can be advantageous to avoid excessive increases in ORP for several reasons, e.g. reducing defaunation effects, and reducing aerobic environments (in which other gases can form, e.g. $CO_2$).

For instance an increase in ORP of over about +500 mV may be avoided (e.g. peak ORP compared to average baseline ORP). Alternatively an increase in ORP of over about +400 mV may be avoided. Alternatively an increase in ORP of over about +300 mV may be avoided. Alternatively an increase in ORP of over about +200 mV may be avoided. Alternatively an increase in ORP of over about +150 mV may be avoided. In some embodiments any increase above these thresholds are transient only.

Alternatively or in addition the gradient of the ORP curve over time ("dORP/dT") may not be steep in the period after administration. For instance dORP/dT may be below about +500 mV/h for about 1 hour after administration.

An excessive increase in ORP may be avoided by the nature of the oxidising agent, which often provides oxidation in a controlled fashion. Alternatively or in addition slowing release of oxidising agent from the composition can be achieved by the formulation e.g. using a coated composition whereby the coating slows the release relative to an uncoated composition.

In preferred embodiments, the invention does not create an aerobic environment (e.g. an environment having an ORP of about 0mV or more positive) in the digestive tract (e.g. rumen of a ruminant) for a sustained period. However, in some embodiments a transient aerobic environment may be tolerated. Therefore, the invention may in some embodiments avoid a sustained ORP increase to an average of above about +300 mV (e.g. for at least 1 hour); preferably a sustained ORP increase to an average of above about +200 mV may be avoided; preferably a sustained ORP increase to an average of above about +150 mV may be avoided; preferably a sustained ORP increase to an average of above about +100 mV may be avoided; preferably a sustained ORP increase to an average of above about +50 mV may be avoided; preferably a sustained ORP increase to an average of above about 0 mV may be avoided. For instance, a sustained (e.g. at least 1 hour) increase in ORP to an average of above about 0 mV may be avoided.

In some embodiments the methods avoid a high peak concentration in peroxide ions following administration of the composition. For instance, $C_{max}$ of peroxide in the digestive tract might be below about 10 mM following administration of the composition. This may be achieved by administration of a composition comprising a coating. Such compositions may result in a controlled increase in peroxide ions over a period of time. This may beneficially allow any released peroxide (or other oxidising agent) to be consumed at a similar rate to its release. This effect may be prolonged and allow for a sustained reduction in methane production from the animal without negatively impacting digestibility.

ORP measurements in an animal can conveniently be made by using a remote ORP electrode protected in a bolus which is added to the rumen by oral administration which can transmit data to a remote data logger. For instance the Example 3 provides an exemplary assay which can be used.

Alternatively, ORP can be measured by taking a stomach fluid (e.g. rumen fluid) sample via mouth using a conventional oral vacuum sampler and then ORP measured with a conventional ORP probe and meter.

Alternatively, an ORP meter and probe can be directly inserted into the stomach (e.g. rumen) via fistula.

The dose of the composition can be adjusted accordingly until a particular ORP change is achieved (e.g. increase in rumen ORP of +100 mV).

The quoted numerical figures and limits for ORP are normally with reference to the figure in an *in vitro* model, such as the RUSITEC system of Example 1 (even where stated to be ORP achieved in an animal). For instance a method said to provide an ORP increase of about +100mV in the rumen of an animal can be said to meet this requirement if an increase of about +100mV is observed in the RUSITEC model. For animals other than cows the model can be adapted accordingly, for example the temperature, saliva, and digesta can be adjusted to model the digestive tract of other animals. In addition or alternatively, conventional bioreactors which replicate the large intestine contents of pigs or other monogastrics are known in the art. Alternatively, the numerical measurement can refer to an average from an *in vivo* study, again taking an average from a group of animals (e.g. 8 animals) to reduce inter-subject variability in the conventional way.

### *Optional phases of the ORP profile in use*

In some embodiments, when a composition of the invention is administered to a ruminant, the ORP profile in the rumen comprises two phases, a first phase ("correction phase"), and a second phase ("maintenance phase").

In the correction phase, ORP may be increased following administration of the composition of the invention. During this phase there may be temporary periods between administrations, where ORP is below a baseline value (e.g. as measured in an untreated control ruminant, for example from about -500 mV to about -200 mV, particularly about -425 mV in a beef cow), for example this might occur in the interval(s) between administration of a composition of the invention to the ruminant (e.g. in the interval(s) between feedings of a composition of the invention to the ruminant).

In the maintenance phase (which may immediately follow the correction phase), ORP may no longer fall substantially below the baseline value between administrations (e.g. ORP does not fall under the baseline value by more than about 100 mv).

The ORP baseline value might vary, depending on for example, the particular species and/or breed of ruminant and/or the diet of the ruminant. ORP in the rumen can be measured by conventional means, e.g. using an ORP meter. See below for an illustrative assay method in animals.

Temporary dips in ORP (e.g. in the correction phase) below baseline may be observed, but the invention still provides increases in ORP from the outset after administration and any temporary dips may reduce or cease entirely over time and with repeated feed cycles (e.g. in the maintenance phase). The invention provides an overall increase in ORP to the extent that a reduction in methanogenic activity is achieved.

In some embodiments, the effects on ORP described elsewhere throughout the specification may be achieved in a correction phase. In some preferred embodiments, the effects on ORP described elsewhere throughout the specification may be achieved in a maintenance phase. In some preferred embodiments, the effects on ORP described elsewhere throughout the specification may be achieved in both correction and maintenance phases.

In some preferred embodiments, when the composition is administered to a ruminant at substantially regular intervals, the correction phase lasts less than about 350 hours from first administration of a composition of the invention to the ruminant. More preferably, the correction phase lasts less than about 300 hours from first administration of a composition of the invention to the ruminant. In particularly preferred embodiments, the correction phase lasts less than about 275 hours from first administration of a composition of the invention to the ruminant. Most preferably, the correction phase lasts less than about 250 hours from first administration of a composition of the invention to the ruminant. This might be achieved, for example, where the composition of the invention is administered at a frequency of once about every 24 hours.

In some preferred embodiments, when the composition is administered to a ruminant at substantially regular intervals, the correction phase might last no longer than from the first administration of a composition of the invention to a ruminant until the 14th administration of a composition of the invention to said ruminant (e.g. no longer than 14 days). More preferably, the correction phase might last no longer than from the first administration of a composition of the invention to a ruminant until the 12th administration of a composition of the invention to said ruminant (e.g. no longer than 12 days). Most preferably, the correction phase might last no longer than from the first administration of a composition of the invention to a ruminant until the 10th administration of a composition of the invention to said ruminant (e.g. no longer than 10 days).

In preferred embodiments, a maintenance phase follows the correction phase (as described above). Preferably, during the maintenance phase, ORP in the rumen of the treated ruminant does not dip below a baseline value (as measured in an untreated control animal, for example from about -500 mV to about -200 mV, particularly about -425 mV in a beef cow) by more than about 150 mV. More preferably, ORP in the rumen of the treated animal does not dip below a baseline value (as measured in an untreated control animal, for example from about -500 mV to about -200 mV, particularly about -425 mV in a beef cow) by more than about 100 mV. More preferably, ORP in the rumen of the treated animal does not dip below a baseline value (as measured in an untreated control animal, for example from about -500 mV to about -200 mV, particularly about -425 mV in a beef cow) by more than about 50 mV. Most preferably, ORP in the rumen of the treated animal does not substantially dip below a baseline value (as measured in an untreated control animal, for example from about -500 mV to about -200 mV, particularly about -425 mV in a beef cow). However, in some embodiments, during the maintenance phase a transient (e.g. for a period of less than an hour) dip below baseline exceeding a preferred tolerance stated above (e.g. of about 100 mV), may also be tolerated. In the context of the maintenance and correction phases "does not dip below a baseline value by more than X mV" generally means that ORP does not become more negative than the baseline value by more than X mV.

*Methane reduction:*

The invention provides methods which reduce methane production from the animal. In preferred embodiments, methods of the invention reduce the volume of methane produced from animals by at least about 5%; more preferably at least about 10%; more preferably at least about 15%; more preferably at least about 20%; more preferably at least about 25%; more preferably at least about 30%; more preferably at least about 35%; more preferably at least about 40%; more preferably at least about 45%; more preferably at least about 50%; more preferably at least about 55%. For example the reduction might be at least about 40%.

*Ammonia production:*

In one aspect, the invention provides a method of increasing ammonia production in the animal. Such methods that increase ammonia production generally employ a urea-containing oxidising agent.
The invention provides a method of reducing methane production from an animal while increasing ammonia production in the animal, the method comprising orally administering a composition comprising an oxidising agent to the animal, wherein the oxidising agent comprises urea hydrogen peroxide.
In preferred embodiments, methods of the invention lead to increase in ammonia production in the animal of at least about 5%; more preferably at least about 10%; more preferably at least about 15%; more preferably at least about 20%; more preferably at least about 25%; more preferably at least about 30%; more preferably at least about 35%; more preferably at least about 40%; more preferably at least about 45%; more preferably at least about 50%; more preferably at least about about 55%; more preferably at least about 60%; more preferably at least about 65%; more preferably at least about 70%; more preferably at least about 75%; more preferably at least about 80%; more preferably at least about 85%; more preferably at least about 90%; more preferably at least about 95%; more preferably at least about 100%. For example the increase might be at least about 40%.
In preferred embodiments, the methods of the invention reduce methane production from the ruminant and also increase in ammonia production in the animal.

*Administration:*

The methods of the invention normally comprise oral administration of a composition. The composition can be administered with food as a supplement to the animal's diet (e.g. as a feed additive), or as a bolus whereby the animal is administered a composition separately from food; or the composition may itself be an animal food product.
When the composition is a bolus, the methods of the invention comprises the step of oral administration of a composition. When the composition is a food additive, the methods of the invention comprise the steps of (i) adding a composition to food and (ii) oral administration of the food.
When the composition is in the form of animal feed, the administration in the methods of the invention comprises the step of feeding the animal (which encompasses any normal feeding techniques such as allowing the animal to feed itself).
In some embodiments the methods of the invention involve administration of a composition (e.g. bolus), in a manner similar to administration of a medicament. Sometimes the animal's diet consists of (or essentially of) feed including a composition of the invention. Sometimes, the animal's diet consists of (or essentially of) a composition of the invention. In some embodiments the administration step is the animal feeding.
In some embodiments the animals' diet consists of a combination of grazing and feeding, and the compositions and methods of the invention are administered during the feeding.
In some preferred embodiments, the compositions of the invention are administered at substantially regular intervals. For example, the interval between subsequent administration of compositions might be about 24 hours.
In some preferred embodiments, the compositions of the invention are administered once-daily. In some such embodiments, when administered daily, the interval between subsequent administration of compositions might be about 24 hours.
In some preferred embodiments, the compositions of the invention are administered more frequently than once-daily. In some particularly preferred embodiments, compositions of the invention are administered are administered at least twice per day. Preferably, compositions of the invention are administered twice per day. Based on the Examples described herein, it is expected that administering additives of the invention to the animals twice per day rather than once per day may result in an improved ORP profile, along with the associated benefits of reduced methanogenesis and improved animal performance.

***Doses and amounts in use:***

The dose can be adjusted based on the amount of feed given to the animal. For instance, a particular amount of composition might be added per kg of feed, in order to control the amount of composition orally administered to the animal. Therefore in some embodiments, the methods include steps of (i) mixing the composition with food and (ii) oral administration of the food and composition to the animal. The composition can itself comprise food. In some embodiments, the composition forms part of the animal's diet. In some embodiments, the composition includes enough food to constitute the animal's entire diet.

Methods of the invention may comprise oral administration of a composition of the invention to the animal in a particular dosage, wherein the dosage is adjusted to provide a particular change in ORP of the digestive tract of the animal (e.g. rumen of the ruminant). One aspect of the invention is method of determining a dose comprising administering a composition, measuring the effect on ORP, and adjusting the dose of the composition to achieve a desired ORP. As described herein, ORP in the digestive tract (e.g. stomach, e.g. rumen) can be measured by conventional means, such as the use of an ORP electrode and meter.

The compositions of the invention may be dosed according to the total dry-matter intake in an animal's diet. Advantageously, dosing according to dry-matter intake of the animal accounts for variations in diet due to age, species, and/or activity of the animal to which the composition is administered. In preferred embodiments, the dose of oxidising agent is from about 0.01% to about 5% of the animal's dry-matter intake; preferably from about 0.05% to about 4%; preferably from about 0.1% to about 3%; preferably from about 0.1% to about 2%. For instance, the dose of oxidising agent administered to the animal might be from about 0.1% to about 2% of the animal's dry-matter intake. For example, in some embodiments wherein the composition is administered daily, the daily dose of composition administered to the animal might be from about 0.1% to about 2% of the animal's daily dry-matter intake. Preferably the % figure is the % of dry matter intake by weight.

In some preferred embodiments, compositions of the invention may be administered at higher doses. For example, preferred doses of oxidising agent might be from about 0.01% to about 20% of the animal's dry-matter intake (by weight); preferably from about 0.1% to about 15%; preferably from about 0.5% to about 10%; preferably from about 1% to about 5%.

The invention provides a method comprising orally administering a composition comprising an oxidising agent to an animal (e.g. a ruminant), in a dose which provides about 0.01 to about 70 mg of hydrogen peroxide per kg of body weight to the animal. Preferably the composition in this method is administered to the animal in a dose which provides about 0.01 to about 60 mg of hydrogen peroxide per kg of body weight to the animal (or an oxidising agent which provides the same amount of oxygen as this amount of hydrogen peroxide); preferably wherein the composition is administered to the animal in a dose which provides about 0.01 to about 50 mg of hydrogen peroxide per kg of body weight to the animal (or an oxidising agent which provides the same amount of oxygen as this amount of hydrogen peroxide); preferably wherein the composition is administered to the animal in a dose which provides about 0.01 to about 40 mg of hydrogen peroxide per kg of body weight to the animal (or an oxidising agent which provides the same amount of oxygen as this amount of hydrogen peroxide); preferably wherein the composition is administered to the animal in a dose which provides about 0.01 to about 35 mg of hydrogen peroxide per kg of body weight to the animal (or an oxidising agent which provides the same amount of oxygen as this amount of hydrogen peroxide).

- For example methods of the invention may comprise administration of a composition comprising an oxidising agent to an animal (e.g. a ruminant) in a dose which provides about 0.01 to about 35 mg of hydrogen peroxide per kg of body weight to the animal.

- In some preferred embodiments of this aspect, the dose provides 0.1 to 70 mg; preferably 0.1 to 60 mg; preferably 0.1 to 50 mg; preferably 0.1 to 40 mg; preferably 0.1 to 35 mg, of hydrogen peroxide per kg of body weight to the animal (or an oxidising agent which provides the same amount of oxygen as this amount of hydrogen peroxide). For example a method of the invention may comprise administration of a composition comprising an oxidising agent, to an animal (e.g. a ruminant) in a dose which provides 0.1 to 35 mg of hydrogen peroxide per kg of body weight to the animal (or an oxidising agent which provides the same amount of oxygen as this amount of hydrogen peroxide).

- In some preferred embodiments of this aspect, the dose provides 1 to 70 mg; preferably 1 to 60 mg preferably 1 to 50 mg; preferably 1 to 40 mg; preferably 1 to 35 mg of hydrogen peroxide per kg of body weight to the animal (or an oxidising agent which provides the same amount of oxygen as this amount of hydrogen peroxide). For example a method of the invention may comprise administration of a composition comprising an oxidising agent, to an animal (e.g. a ruminant) in a dose which provides 1 to 35 mg of hydrogen peroxide per kg of body weight to the animal (or an oxidising agent which provides the same amount of oxygen as this amount of hydrogen peroxide).

- In a preferred embodiment, a method of the invention comprises oral administration of a composition of the invention to a ruminant (e.g. a cow) in a dose which provides 8 to 35 mg of hydrogen peroxide per kg of body weight to the ruminant (or an oxidising agent which provides the same amount of oxygen as this amount of hydrogen peroxide).

- In a preferred embodiment, a method of the invention comprises oral administration of a composition of the invention to a ruminant (e.g. a sheep) in a dose which provides 3 to 15 mg of hydrogen peroxide per kg of body weight to the ruminant (or an oxidising agent which provides the same amount of oxygen as this amount of hydrogen peroxide).

In some preferred embodiments, compositions of the invention may be administered at higher doses. For example, preferred doses of oxidising agent might be from 0.01 to about 1050 mg of oxidising agent per kg of body weight to the animal; preferably from about 0.01 to about 500 mg of oxidising agent per kg of body weight; preferably from about 0.01 to about 350 mg of oxidising agent per kg of body weight; preferably from about 0.1 to about 250 mg of oxidising agent per kg of body weight; preferably from about 0.5 to about 150 mg of oxidising agent per kg of body weight; preferably from about 1 to about 100 mg of oxidising agent per kg of body weight.

It is preferred that the dose is based on % of total dry-matter intake in an animal's diet rather than hydrogen peroxide per kg of body weight to the animal. This might be because, dosing according to dry-matter intake of the animal may account for variations in diet due to age, species, breed, and/or activity of the animal to which the composition is administered.

The invention provides a method comprising oral administration of a composition to a ruminant in a dose which provides 1 to 250 mg of hydrogen peroxide per L of rumen volume to the ruminant (or an oxidising agent which provides the same amount of oxygen as this amount of hydrogen peroxide).

- Preferably the composition is administered to the animal in a dose which provides 1 to 200 mg; preferably 1 to 150 mg; preferably 5 to 150 mg; preferably 10 to 150 mg; preferably 20 to 150 mg; preferably 30 to 150 mg; preferably 35 to 150 mg; of hydrogen peroxide per L of rumen volume to the ruminant (or an oxidising agent which provides the same amount of oxygen as this amount of hydrogen peroxide).
- For example methods of the invention may comprise oral administration of a composition to a ruminant in a dose which provides 35 to 150 mg of hydrogen peroxide per L of rumen volume to the ruminant (or an oxidising agent which provides the same amount of oxygen as this amount of hydrogen peroxide).

In some preferred embodiments, compositions of the invention may be administered at higher doses. For example, the invention provides a method comprising oral administration of a composition to a ruminant in a dose which provides 0.01 to 2250 mg of hydrogen peroxide per L of rumen volume to the ruminant (or an oxidising agent which provides the same amount of oxygen as this amount of hydrogen peroxide); preferably, in a dose which provides 0.1 to 1500 mg of hydrogen peroxide per L of rumen volume to the ruminant (or an oxidising agent which provides the same amount of oxygen as this amount of hydrogen peroxide); preferably, in a dose which provides 0.1 to 750 mg of hydrogen peroxide per L of rumen volume to the ruminant (or an oxidising agent which provides the same amount of oxygen as this amount of hydrogen peroxide); preferably, in a dose which provides 0.1 to 500 mg of hydrogen peroxide per L of rumen volume to the ruminant (or an oxidising agent which provides the same amount of oxygen as this amount of hydrogen peroxide); preferably in a dose which provides 1 to 300 mg of hydrogen peroxide per L of rumen volume to the ruminant (or an oxidising agent which provides the same amount of oxygen as this amount of hydrogen peroxide).

As discussed, the invention does not normally aim to provide significant concentrations of peroxide (e.g. hydrogen peroxide) per se. The dosages and amounts are normally suitable for providing a source of oxygen and therefore raising ORP. The peroxide may be decomposed at a rate such that the peroxide acts as a source of oxygen rather than a reactive species, particularly if the peroxide is released in a controlled fashion from the formulation.

In preferred embodiments, methods of the invention do not substantially increase the concentration of peroxide in the digestive tract of the animal (e.g. the rumen of a ruminant). For example, the methods of the invention preferably do not increase the concentration of peroxide ions in the rumen above a particular value (i.e. $C_{max}$ of about 10 mM) for a particular period following administration of the composition (e.g. 1 hour). In some embodiments the methods of the invention preferably do not increase the concentration of peroxide ions in the rumen above a $C_{max}$ of about 15 mM for a particular period following administration of the composition (e.g. 1 hour). In some embodiments the methods of the invention preferably do not increase the concentration of peroxide ions in the rumen above a $C_{max}$ of about 20 mM for a particular period following administration of the composition (e.g. 1 hour).

In some embodiments, a method of the invention comprises oral administration of a composition of the invention to a ruminant in a dose which provides $C_{max}$ of 0.01 to 100 mM of hydrogen peroxide or peroxide ions in the rumen of the ruminant; or a $C_{max}$ of 0.01 to 50 mM; or a $C_{max}$ of 0.01 to 40 mM; or a $C_{max}$ of 0.01 to 30 mM; or a $C_{max}$ of 0.01 to 20 mM; ; or a $C_{max}$ of 0.01 to 15 mM or a $C_{max}$ of 0.01 to 10 mM. For example, a method of the invention may comprise oral administration of a composition of the invention to a ruminant in a dose which provides $C_{max}$ of 0.01 to 10 mM of hydrogen peroxide or peroxide ions in the rumen of the ruminant.

In some embodiments, a method of the invention comprises oral administration of a composition of the invention to a ruminant in a dose which provides $C_{max}$ of 0.1 to 100 mM of hydrogen peroxide or peroxide ions in the rumen of the ruminant; or a $C_{max}$ of 0.1 to 50 mM; or a $C_{max}$ of 0.1 to 40 mM; or a $C_{max}$ of 0.1 to 30 mM; or a $C_{max}$ of 0.1 to 20 mM; or a $C_{max}$ of 0.1 to 15 mM; or a $C_{max}$ of 0.1 to 10 mM. For example, a method of the invention may comprise oral administration of a composition of the invention to a ruminant in a dose which provides $C_{max}$ of 0.1 to 10 mM of hydrogen peroxide or peroxide ions in the rumen of the ruminant.

In preferred embodiments, methods of the invention comprise oral administration of a composition of the invention to a ruminant in a dose which provides $C_{max}$ of less than 100 mM of hydrogen peroxide or peroxide ions in the rumen of the ruminant; preferably less than 50 mM; preferably less than 40 mM; preferably less than 30 mM; preferably less than 20 mM; preferably less than 15mM; preferably less than 10 mM. For example, methods of the invention may comprise oral administration of a composition of the invention to a ruminant in a dose which provides $C_{max}$ of less than 10 mM of hydrogen peroxide or peroxide ions in the rumen of the ruminant.

In some embodiments, methods of the invention may comprise a dose ramping period in which the dose is gradually increased to a target dose. The target dose might be any of the doses described herein. For example, methods of the invention might involve a dose ramping period in which the composition is administered to the animal at an initial dose of at least about 10% of the target dose for the first 7-14 administrations to a given animal (e.g. 14). Preferably methods of the invention might involve a dose ramping period in which the composition is administered to the animal at an initial dose of at least about 50% of the target dose for the first 7-14 (e.g. 7) administrations. Preferably methods of the invention might involve a dose ramping period in which the composition is administered to the animal at an initial dose of at least about 75% of the target dose for the first 3-7 (e.g. 7) administrations.

In a particularly preferred embodiment, methods of the invention comprise administering a composition to an animal which provides about 25-300 g of calcium peroxide to the animal (for example, 25 g, 50 g, 100 g, 150 g, 200 g, or 300 g, particularly 200 g). For example, methods of the invention might comprise once-daily administration a composition to an animal which provides about 25-300 g of calcium peroxide to the animal (for example, 25 g, 50 g, 100 g, 150 g, 200 g, or 300 g, particularly 200 g). In some such embodiments, the composition comprises about 25-300 g of calcium peroxide (for example, 25 g, 50g, 100 g, 150 g, 200 g, or 300 g, particularly 200 g). In some such embodiments, the composition comprises feed and calcium peroxide in a proportion such that daily feeding provides about 25-300 g of calcium peroxide (for example, 25 g, 50 g, 100 g, 150 g, 200 g, or 300 g, particularly 200 g).

*Digestibility:*

The invention provides a method of reducing methane production in ruminants without negatively impacting digestibility (e.g. without substantially reducing digestibility) of the animal (e.g. ruminant).

Digestibility, in particular dry-matter digestibility can be calculated from the feed material present after digestion as a proportion of total feed material prior to digestion (for example by dry weight). Preferably, methods of the invention do not reduce digestibility by more than about 25%; more preferably, methods of the invention do not reduce digestibility by more than about 20%; more preferably, methods of the invention do not reduce digestibility by more than about 15%; methods of the invention do not reduce digestibility by more than about 10%; more preferably, methods of the invention do not reduce digestibility by more than about 7%; more preferably, methods of the invention do not reduce digestibility by more than about 6%; more preferably, methods of the invention do not reduce digestibility by more than about 5%; more preferably, methods of the invention do not reduce digestibility by more than about 4%; more preferably, methods of the invention do not reduce digestibility by more than about 3%; more preferably, methods of the invention do not reduce digestibility by more than about 1%. For example the reduction might be less than about 5%. The term "without substantially reducing digestibility" can mean that digestibility is not reduced by these amounts.

The invention provides methods of reducing methane production in ruminants which avoid substantial defaunation of the digestive tract (e.g. rumen in ruminants). Preferably, the methods of the invention avoid substantial defaunation even after prolonged treatment (e.g. 1 week, preferably 1 month). Some minor transient defaunation may occur.

In some preferred embodiments, methods of the invention do not reduce rumen protozoa populations in the ruminant by more than about 40%. Preferably, methods of the invention do not reduce rumen protozoa populations in the ruminant by more than about 25%. Preferably, methods of the invention do not reduce rumen protozoa populations in the ruminant by more than about 20%. Preferably, methods of the invention do not reduce rumen protozoa populations in the ruminant by more than about 10%. In particularly preferred embodiments, methods of the invention do not substantially reduce rumen protozoa populations in the ruminant. In some embodiments protozoa populations are measured by conventional means, for example, taking samples, and using microscopy to count protozoa.

The invention provides methods of reducing methane production without substantially reducing microbiome function in the digestive tract (e.g. rumen in ruminants), wherein microbiome function is making nutrients available to the animal (and does not include methanogenesis). Preferably, methods of the invention do not reduce microbiome function by more than about 20%; preferably not more than about 15%; preferably not more than about 10%; preferably not more than about 5%; preferably not more than about 4%; preferably not more than about 3%; preferably not more than about 2%; preferably not more than about 1%; most preferably methods of the invention do not reduce the population of protozoa in the digestive tract (e.g. rumen in ruminants).

The invention provides methods of reducing methane production without substantially reducing feed efficiency. Generally, the proportion of feed converted into digestible carbon which is made available to the animal is not reduced by methods of the present invention. In particular, the invention might not reduce the proportion of feed converted into digestible carbon which is made available to the animal by more than about 30%; preferably not more than about 20%; preferably not more than about 10%; most preferably the proportion of feed converted into digestible carbon which is made available to the animal is substantially not reduced. In some embodiments methods of the invention might increase the proportion of feed converted into digestible carbon which is made available to the animal by at least about 5%; preferably at least about 10%; preferably at least about 15%; preferably at least about 20%; preferably at least about 25%. For instance methods of the invention might not substantially reduce the proportion of feed converted into digestible carbon which is made available to the animal (e.g. ruminant).

Previous methods of reducing methane production in animals have often involved defaunation, at the cost of reduced digestibility particularly due to impaired microbiome function. This type of approach may be characterised in that the level of methane production is correlated with a reduction in the methanogen and/or protozoa populations in the digestive tract (e.g. rumen). Such prior methods may not be suitable for long term use (e.g. for days or weeks) and prior experiments have often been terminated before long term use is investigated.

Preferably the methods of the invention do not substantially acidify the digestive tract, for example methods of the invention might not substantially reduce the pH of the rumen. Preferably the methods of the invention do not reduce the pH of the rumen below about 6; preferably the methods of the invention do not reduce the pH of the rumen below about 6.1; preferably the methods of the invention do not reduce the pH of the rumen below about 6.2; preferably the methods of the invention do not reduce the pH of the rumen below about 6.3; preferably the methods of the invention do not reduce the pH of the rumen below about 6.4. For example, the methods of the invention might not reduce the pH of the rumen below about 6.4. The pH of the digestive tract might not drop below about 6.4 in the initial hours (e.g. about 2 hours) after administration of the composition.

In some embodiments, methods of the invention, and compositions of the invention when administered to animals, do not substantially reduce the weight of the animal to which the composition is administered. Instead, methods of the invention, and compositions of the invention when administered to animals, preferably cause an increase in weight of the animal, which may, for example, be due to improved digestibility.

*Safety:*

Preferably, the methods of the invention are safe. In particular, methods of the invention preferably involve administration of compositions which are substantially free of toxic components. Any toxic components, if present, are preferably in non-toxic amounts and/or provide non-toxic concentrations over time. In some embodiments, toxic components are entirely absent.

In preferred embodiments, methods of the invention do not substantially increase the concentration of iodide in the meat and/or milk produced by or from the animal (e.g. ruminant).

Preferably, the compositions of the invention are safe for administration to animals. In particular, compositions of the invention preferably are substantially free of toxic components. Any toxic components, if present, are preferably in non-toxic amounts and/or provide non-toxic concentrations over time. In some embodiments, toxic components are entirely absent.

In preferred embodiments, compositions of the invention when administered to animals do not substantially increase the concentration of iodide in the meat and/or milk produced by or from the animal (e.g. ruminant).

*Further components in use:*

The methods of the invention may further comprise administration of additional anti-methanogenic agents (i.e. substances or compositions which reduce the production of methane from an animal). Some additional anti-methanogenic agents are known in the art, for example 3-nitrooxypropanol (3NOP), Coenzyme M analogs, halogenated aliphatic C1-C2 hydrocarbons e.g. bromoforms; Pterin compounds, Hydroxymethylglutaryl-CoA (HMG-S-CoA) reductase inhibitors, fats and fatty acids, plant secondary metabolites e.g. tannins, flavonoids, organosulphur compounds, essential oils, allicin, alternative hydrogen sinks e.g. nitrate and sulfate, nitrocompounds, propionate and butyrate enhancers, unsaturated organic acids, inhibitors to hydrogen-producing bacteria e.g. ionophores or bacteriocins. A preferred anti-methanogenic agent is 3NOP. Such additional methanogenic agents can be administered (i) before; (ii) after; or (iii) concomitantly with, administration of a composition disclosed herein. As mentioned below such anti-methanogenic agents can also be present within the composition.

In some preferred embodiments, the methods of the invention comprise administering only one anti-methanogenic agent. In some embodiments the methods do not comprise administration of additional anti-methanogenic agents.

For example, methods of the invention may comprise administering compositions which are substantially free of 3NOP (e.g. comprise no 3NOP).

In a further aspect, the invention relates to a method of reducing methane production from an animal, the method comprising orally administering a composition to the animal, wherein the composition comprises: (i) a source of peroxide; and (ii) catalase. In a further aspect, the invention relates to a method of reducing methane production from an animal, the method comprising orally administering a composition to the animal, wherein the composition comprises: (i) a source of peroxide; and (ii) a peroxidase. The inventors have discovered that compositions which comprise both a source of peroxide and a peroxidase or catalase exhibit accelerated decomposition of peroxide into oxygen. The presence of a peroxidase or catalase has the advantage of increasing ORP (by improving release of oxygen) whilst reducing peroxide accumulation (by decomposing peroxide) and any negative effects associated with peroxide accumulation, for example defaunation of the rumen.

*Further methods of the invention:*

In one aspect, the invention provides a method of reducing methane production from an animal by increasing the oxidation-reduction potential in the digestive tract of the animal without substantially reducing digestibility, the method comprising orally administering a composition comprising an oxidising agent to the animal. The inventors have discovered that increasing the oxidation-reduction potential while avoiding substantially reducing digestibility is a new and effective approach for reducing methane production. This approach can contrast previous approaches which may have focused on defaunation, at the price of reductions in digestibility.

In one aspect, the invention relates to a method of reducing methane production from an animal by increasing the oxidation-reduction potential in the digestive tract of the animal without substantially reducing digestibility, the method comprising orally administering a composition comprising an oxidising agent to the animal; wherein about 0.05% (by weight) of the composition is the oxidising agent. Preferably the oxidising agent is about 0.1 %; preferably about 0.15%; preferably about 0.3%; preferably about 0.6%; preferably about 1.2%; preferably about 2%; preferably about 2.5% of the composition.

*Iodides, nitrates, and thiocyanates:*

Preferably compositions of the invention are substantially free of iodide or a source of iodide. Preferably compositions of the invention are free of iodide or a source of iodide. Absence of iodide is generally desirable for animal food products, e.g. since iodide can be found in subsequent animal products such as milk. In particular, compositions of the invention may comprise less than 1% by weight of iodide or a source of iodide; more preferably compositions of the invention may comprise less than 0.5% by weight of iodide or a source of iodide; more preferably compositions of the invention may comprise less than 0.1% by weight of iodide or a source of iodide; more preferably compositions of the invention may comprise less than 0.01% by weight of iodide or a source of iodide; most preferably compositions of the invention are free of iodide or a source of iodide. For example, compositions of the invention may contain less than 0.01 % by weight of iodide or a source of iodide.

In preferred embodiments, any iodide or sources of iodide in the compositions of the invention are at a level such that in use, the compositions of the invention do not substantially increase the concentration of iodide in the meat and/or milk produced by or from the animal (e.g. ruminant) to which they are orally administered. The increase in concentration of iodide can be measured as relative to a normal level of iodide for an animal which is on substantially the same diet but which does not receive a composition of the invention as part of or in addition to the diet.

Preferably, compositions of the invention can be substantially free of nitrate or a source of nitrate. In particular, compositions of the invention may comprise less than 1% by weight of nitrate or a source of nitrate; more preferably compositions of the invention may comprise less than 0.5% by weight of nitrate or a source of nitrate; more preferably compositions of the invention may comprise less than 0.1% by weight of nitrate or a source of nitrate; more preferably compositions of the invention may comprise less than 0.01% by weight of nitrate or a source of nitrate; most preferably compositions of the invention are free of nitrate or a source of nitrate. For example, compositions of the invention may contain less than 0.01% by weight of nitrate or a source of nitrate. Preferably compositions of the invention are free of nitrate.

Preferably compositions of the invention are substantially free of thiocyanate or a source of thiocyanate. Preferably compositions of the invention are free of thiocyanate or a source of thiocyanate. In particular, compositions of the invention may comprise less than 1% by weight of thiocyanate or a source of thiocyanate; more preferably compositions of the invention may comprise less than 0.1% by weight of thiocyanate or a source of thiocyanate; more preferably compositions of the invention may comprise less than 0.01% by weight of thiocyanate or a source of thiocyanate; most preferably compositions of the invention are free of thiocyanate or a source of thiocyanate. For example, compositions of the invention may contain less than 0.01% by weight of thiocyanate or a source of thiocyanate.

*Formulation:*

Compositions of the invention are preferably formulated for oral administration.

Preferred compositions of the invention are free of enteric formulation features, e.g. an enteric coat. Preferred compositions of the invention are capable of delivering the oxidising agent(s) to (at least) the stomach (e.g. rumen) of the animal. Preferred compositions of the invention are capable of delivering the oxidising agent(s) to (at least) the mouth and oesophagus of the animal, for exposure to saliva. In some preferred embodiments, compositions are free of hydroxypropyl methylcellulose. In some preferred embodiments, compositions of the invention are formulated to avoid delivery of the oxidising agent to the intestine of the animal to which the composition is administered (e.g. the hind gut or colon). In some embodiments, this may be achieved by compositions which are free of enteric formulation features, e.g. an enteric coat. In some preferred embodiments, compositions are free of hydroxypropyl methylcellulose.

In some embodiments the composition of the invention may be in the form of a bolus. A bolus is normally a single unit dosage form comprising the oxidising agent which is adapted to be orally administered and to remain in the digestive tract and to release oxidising agent for a period of time.

In some embodiments, the composition of the invention may comprise one or more oxides, such as (but not limited to) phosphorus (V) oxide ($P_2O_5$), sodium oxide ($Na_2O$), iron oxide (e.g. iron (II) oxide, iron (III) oxide, or combinations thereof), zinc oxide (ZnO), and/or magnesium oxide (MgO); waxes; carbonates; trace elements; magnesium stearate; hydrogenated fat; salts; conventional excipients for a bolus for animals; or combinations thereof (e.g. all of these).

In some embodiments, the composition of the invention may comprise one or more oxides, such as (but not limited to) phosphorus (V) oxide ($P_2O_5$), sodium oxide ($Na_2O$), and/or magnesium oxide (MgO); waxes; carbonates; trace elements; salts; conventional excipients for a bolus for animals; or combinations thereof (e.g. all of these).

In some embodiments, the composition may comprise iron oxide (e.g. iron (II) oxide, iron (III) oxide, or combinations thereof), zinc oxide, magnesium stearate, hydrogenated fat, or combinations thereof (e.g. all of these).

In some embodiments where the invention is in the form of a bolus, the composition may comprise conventional excipients for a bolus for animals. In some embodiments where the invention is in the form of a bolus, the composition may comprise iron oxide, zinc oxide, magnesium stearate, hydrogenated fat, or combinations thereof (e.g. all of these).

When the composition is administered as a bolus, administration may be on a regular basis, for example the bolus may be administered on a weekly, and/or monthly basis. Normally the bolus will be administered weekly or less frequently. For example the bolus may be administered every two weeks. A bolus may optionally include a casing, and/or a coating. The composition of the invention can be formulated as a bolus using conventional means.

Compositions of the invention may be an animal food product.

In preferred embodiments, compositions of the invention are formulated as feed additives. Such compositions are suitable for adding to food prior to consumption.

The feed additives of the invention may further comprise animal feed components. Various acceptable animal feed components are known in the art, and preferred animal feed components are maize, barley, soya, and/molasses. In some embodiments the compositions of the invention may be provided as an animal food product including (i.e. further comprising) food, for example food suitable for the animal's entire diet. In some embodiments the invention as provided as a feed additive suitable for adding to animal feed prior to feeding. In this case the composition may optionally comprise food, or the composition may be free of food.

Compositions of the invention may also comprise a pharmaceutically acceptable carrier and/or diluent such as water, saline, an emulsion, a gel, a hydrogel, or a solid for pellets.

The compositions and compositions for use in methods of the invention may further comprise excipients. Various suitable excipients are known in the art. Excipients may include (but are not limited to) antiadherents, binders, coatings, colouring, disintegrants, flavourings, glidants, lubricants, preservatives, sorbents, sweeteners, and vehicles.

In some embodiments, further excipients may include (but are not limited to) density adjusters, water soluble bulking agents, water insoluble bulking agents, and polymers.

Compositions of the invention may be provided in aqueous solution, in saline solution, as an emulsion, as a gel, as a hydrogel, as a paste, as a pellet, or as a powder. In preferred embodiments, the feed additive is provided as a pellet. In another preferred embodiment, the feed additive is provided as a powder.

In some embodiments, compositions of the invention may be provided as a powder incorporated into a concentrate ration. In other embodiments, compositions of the invention may be provided as a compacted powder incorporated into a feed nut.

Compositions of the invention may generally be in solid or liquid form. Preferably the composition of the invention is a solid form. Alternatively, compositions of the invention may be in liquid form, for example compositions of the invention may comprise a liquid oxidising agent (e.g. a liquid source of peroxide, e.g. encapsulated liquid source of peroxide).

In some preferred embodiments, the compositions and compositions for use in methods of the invention are not nibbling or lick stones. In some embodiments the compositions of the invention are for feed and not for nibbling, licking or for administration via fistulation and/or cannulation.

*Optional coating:*

In one aspect the invention provides a composition for oral administration to an animal comprising an oxidising agent, and a coating.

The compositions of the invention described herein may preferably comprise a coating. The compositions may alternatively be a capsule.

In one aspect the invention provides a composition for oral administration to an animal comprising an oxidising agent, and a coating, wherein the composition is preferably free of a source of iodide.

Coatings may be suitable for improving storage stability of the composition. Thus in some preferred embodiments the composition is a coated, storage stable composition. For instance in some embodiments the composition of the invention comprises a coating which reduces degradation of the oxidising agent and improves storage stability (e.g. reducing degradation in air).

Coatings may also be suitable for preventing degradation in the presence of organic feed materials. For instance in some preferred embodiments the composition of the invention comprises a coating which reduces degradation of the oxidising agents in the presence of animal feed. Such coatings may reduce degradation in the period between mixing the composition with food and the mixture being consumed. Alternatively or in addition such coatings may improve storage stability by reducing degradation when the composition is mixed and stored with food.

Coatings may also improve release the profile of the composition. For instance in some preferred embodiments the composition of the invention comprises a coating which slows release of the oxidising agent into the digestive tract. Such coatings can for instance provide controlled release, e.g. delayed and/or sustained release.

A composition with a coating can be prepared by formulating another composition of the invention (e.g. a composition of Examples 1 and 2) as a solid form (e.g. pellets), and then coating the pellets with a coating material.

Preferred coated compositions deliver the increase in ORP to the intended site (e.g. the stomach, e.g. the rumen of ruminants). This feature can be advantageous to avoid the oxidising agent degrading in the mouth. For example a preferred coating is saliva resistant (e.g. reducing degradation or release of the oxidising agent by saliva during oral administration). A coating can also preferably avoid irritation in the mouth. It can also be preferably target the main site of methanogenesis (e.g. the stomach, e.g. the rumen of ruminants). In alternative embodiments, the composition comprises an enteric coating. Enteric coatings can be used to allow for passage through the stomach and small intestine and release in the large intestine.

Preferred coated compositions extend the delivery of ORP. An extended release coating can be particularly preferable when the composition comprises a source of peroxide as the oxidising agent. In particular this can help to keep peroxide concentrations low and avoid a substantial negative impact on digestibility, for example by defaunation of the rumen potentially caused by higher concentrations. In some preferred embodiments the composition comprises a extended release coating.

For example, where the oxidising agent is a source of peroxide, it may be desirable to avoid releasing high concentrations of peroxide into the rumen, for example an elevated $C_{max}$ of peroxide above a particular value (e.g. about 10 mM, about 15 mM, or about 20 mM) may be avoided. Accordingly, the compositions of the invention can preferably be coated in a coating which controls or extends release when administered to an animal, thus avoiding a sudden and/or substantial increase in the peroxide concentration in the digestive tract of the animal (e.g. the stomach such as the rumen of a ruminant).

Preferred coatings are also capable of protecting the composition from pressure. Preferred coatings are also capable of protecting the composition from moisture. Preferred coatings are also capable of protecting the composition from heat. Such feature may advantageously prevent degradation of the composition (e.g. decomposition of a source of peroxide) before administration, for example during processing, production, and/or storage.

Preferred coatings comprise at least one of the following: oils (e.g. coconut oil), gylcerin, glycerol, silica hydrogels, poly(methyl methacrylate) capsulation, poly(D,L-lactide-co-glycolide) (PLGA), alginate, poly(vinyl pyrrolidone) (PVP), hard fat, ethyl cellulose, N-isopropylacrylamide (NIPAAm), acrylic acid, hydroxyethyl methancrylate-oligo(hydroxybutyrate), polydimethylsiloxane (PDMS), methacrylamide chitosan, cyanoacrylate, sodium alginate, polyisobutylene, isobutyleneisoprene copolymers, styrene-butadiene copolymers, polyvinyl acetate, polyisoprene, polyethylene, vinyl acetate, and combinations thereof. Such coatings can be used individually, or in combination. For example, hydrogen peroxide may be bound to PVP and encapsulated further in PLGA. Hydrophobic materials for encapsulation/entrapment may be preferably employed to slow down rate of oxygen release; whilst hydrophilic materials maybe employed to increase the oxygen release/diffusion rate.

Preferred coatings are non-enteric coatings. For example preferred coatings may be free of adaptation which prevent delivery to (at least) the stomach (e.g. rumen) of the animal. For example preferred coatings may not include features which are adapted to prevent release in low-pH environment (e.g. below pH 4).

*Dosages and amounts in compositions:*

Compositions of the invention may comprise a dose of oxidising agent based on the body weight of the animal. For example, in some preferred embodiments the composition comprises a dose which provides about 1 to about 35 mg (e.g. about 8 to about 35mg or about 3 to about 15 mg) of hydrogen peroxide per kg of body weight to the animal. Alternatively the composition may comprise a dose of an oxidising agent which provides the same amount of peroxide ions (e.g. delivered to the digestive tract) as about 1 to about 35 mg (e.g. about 8 to about 35mg or about 3 to about 15 mg) of hydrogen peroxide per kg of body weight.

Compositions of the invention may comprise higher or lower doses of oxidising agent. For example, in some embodiments compositions of the invention may comprise a dose of oxidising agent of about 0.01 to about 1050 mg per kg of body weight of the animal. Preferably, the compositions of the invention comprise a dose of oxidising agent of about 0.01 to about 500 mg per kg of body weight of the animal. Preferably, the compositions of the invention comprise a dose of oxidising agent of about 0.01 to about 350 mg per kg of body weight of the animal. Preferably, the compositions of the invention comprise a dose of oxidising agent of about 0.1 to about 250 mg per kg of body weight of the animal. Preferably, the compositions of the invention comprise a dose of oxidising agent of about 0.5 to about 150 mg per kg of body weight of the animal. Preferably, the compositions of the invention comprise a dose of oxidising agent of about 1 to about 100 mg per kg of body weight of the animal.

In preferred embodiments, for example where the animal is a cow, compositions of the invention may comprise a dose which provides about 8 to about 35 mg of hydrogen peroxide per kg of body weight to the animal. Alternatively the composition may comprise a dose of an oxidising agent which provides the same amount of peroxide ions (e.g. delivered to the digestive tract) as about 8 to about 35 mg of hydrogen peroxide per kg of body weight.

In preferred embodiments, for example where the animal is a cow, compositions of the invention may comprise a dose which provides about 0.01 to about 2250 mg of hydrogen peroxide per kg of body weight to the animal (for example, about 0.01 to 1500 mg of hydrogen peroxide per kg of body weight, about 0.01 to 750 mg of hydrogen peroxide per kg of body weight, about 0.1 to 350 mg of hydrogen peroxide per kg of body weight, or about 8 to 100 mg of hydrogen peroxide per kg of body weight). Alternatively the composition may comprise a dose of an oxidising agent which provides the same amount of peroxide ions (e.g. delivered to the digestive tract) as about 0.01 to about 2250 mg of hydrogen peroxide per kg of body weight (for example, about 0.01 to 1500 mg of hydrogen peroxide per kg of body weight, about 0.01 to 750 mg of hydrogen peroxide per kg of body weight, about 0.1 to 350 mg of hydrogen peroxide per kg of body weight, or about 8 to 100 mg of hydrogen peroxide per kg of body weight).

In preferred embodiments, for example where the animal is a sheep, the compositions of the invention may comprise a dose which provides about 3 to about 15 mg of hydrogen peroxide per kg of body weight to the animal. Alternatively the composition may comprise a dose of oxidising agent which provides the same amount of peroxide ions (e.g. delivered to the digestive tract) as about 3 to about 35 mg of hydrogen peroxide per kg of body weight.

In preferred embodiments, for example where the animal is a sheep, the compositions of the invention may comprise a dose which provides about 0.01 to about 750 mg of hydrogen peroxide per kg of body weight to the animal (for example, about 0.1 to 350 mg of hydrogen peroxide per kg of body weight or about 8 to 100 mg of hydrogen peroxide per kg of body weight). Alternatively the composition may comprise a dose of oxidising agent which provides the same amount of peroxide ions (e.g. delivered to the digestive tract) as about 0.01 to about 750 mg of hydrogen peroxide per kg of body weight (for example, about 0.1 to 350 mg of hydrogen peroxide per kg of body weight or about 8 to 100 mg of hydrogen peroxide per kg of body weight).

Compositions of the invention may comprise a dose based on the stomach volume of the animal. For instance compositions of the invention may comprise a dose based on the rumen volume of a ruminant.

Compositions of the invention may comprise higher doses of oxidising agent. For example, in some embodiments compositions of the invention comprise a dose which provides about 150 to 450 mg of hydrogen peroxide per L of rumen volume to the ruminant. Alternatively the composition may comprise a dose of oxidising agent which provides the same amount of peroxide ions (e.g. delivered to the digestive tract) as about 150 to about 450 mg of hydrogen peroxide per L of rumen volume to the ruminant.

For example, in some embodiments the composition comprises a dose which provides about 35 to about 150 mg of hydrogen peroxide per L of rumen volume to the ruminant. Alternatively the composition may comprise a dose of oxidising agent which provides the same amount of peroxide ions (e.g. delivered to the digestive tract) as about 35 to about 150 mg of hydrogen peroxide per L of rumen volume to the ruminant.

Compositions of the invention may comprise a dose adapted to provide a particular Cmax of hydrogen peroxide or peroxide ions achieved in the digestive tract (e.g. stomach, e.g. rumen). For example, a dose which provide Cmax of about 0.5 to about 30 mM of hydrogen peroxide or peroxide ions in the rumen of the ruminant is preferred in some embodiments.

Compositions of the invention may comprise a dose adapted to provide a particular $C_{max}$ of hydrogen peroxide or peroxide ions achieved in the digestive tract (e.g. stomach, e.g. rumen). For example, a dose which provide $C_{max}$ of

about 0.1 to about 10 mM of hydrogen peroxide or peroxide ions in the rumen of the ruminant is preferred in some embodiments.

In some embodiments, compositions of the invention are formulated to provide a dose of oxidising agent which elevates ORP by a particular amount in the digestive tract of an animal (e.g. in the stomach, e.g. in the rumen of a ruminant). In particular, a composition of the invention may comprise a dose of oxidising agent which elevates ORP by at least about +10mV; preferably at least about +50mV; preferably at least about +100 mV; preferably at least about +150 mV; preferably at least about +200 mV; preferably at least about +250 mV; preferably at least about +300 mV. For example, a composition of the invention may comprise a dose of oxidising agent which elevates ORP in the digestive tract of an animal (e.g. in the stomach, e.g. in the rumen of a ruminant) by at least about +100 mV.

In some embodiments, compositions of the invention are formulated to provide a dose of oxidising agent which maintains a particular ORP range in the digestive tract of an animal (e.g. the stomach, e.g. the rumen of a ruminant) for a period (e.g. for at least about 1 hour). In particular, a composition of the invention may be formulated to provide a dose of oxidising agent which maintains an ORP in the digestive tract of an animal (e.g. the rumen of a ruminant) from -250 mV to +250 mV; preferably from -200 mV to +200 mV; preferably from -200 mV to +150 mV; preferably from -200 mV to +100 mV; preferably from -200 mV to +50 mV; preferably from -200 mV to 0 mV; preferably from -200 mV to -50 mV; most preferably from about -150 mV to about -50 mV for a period of time. This period of time may be at least about 1 hour. Alternatively it may be at least about 2 hours, about 3 hours, about 4 hours, about 5 hours or about 6 hours. For example, a composition of the invention may be formulated to provide a dose of oxidising agent which provides an ORP range in the digestive tract of an animal (e.g. the stomach, e.g. the rumen of a ruminant) of from about -150 mV to about -50 mV for a period (e.g. for at least about 1 hour).

In some embodiments, compositions of the invention may be formulated to provide a dose of oxidising agent which elevates average ORP above a particular potential in the digestive tract of an animal (e.g. the stomach, e.g. the rumen of a ruminant). In particular, a composition of the invention may increase average ORP to at least about -200 mV; preferably at least about -150 mV; preferably at least about -100 mV; preferably at least about -50 mV; preferably at least about -0 mV; preferably at least about +50 mV; most preferably at least about +100 mV. The average ORP is preferably taken in the period from administration to about 1 hour after administration. In some alternative embodiments the average ORP is taken in the period from administration to about 2 hours, about 3 hours, about 4 hours, about 5 hours, or about 6 hours after administration.

For example, a composition of the invention may be formulated to provide a dose of oxidising agent which increases average ORP in the digestive tract of an animal (e.g. the stomach, e.g. the rumen of a ruminant) to at least about -150 mV in the period from administration to about 1 hour after administration.

The compositions are suitable for elevating ORP for a period of time after administration of the composition. Normally the compositions of the invention are formulated to be capable of elevating ORP to least about -150 mV. The compositions are preferably formulated to provide ORP at or above the target level for at least about 1 hours, preferably for at least about 2 hours, preferably for at least about 3 hours, preferably for at least about 4 hours, preferably for at least about 4 hours, preferably for at least about 5 hours, preferably for at least about 6 hours. For example, the ORP may be elevated to at least -200 mV for at least 6 hours. The ORP is elevated in the main stomach component of the digestive tract, e.g. the rumen. This might be the first chamber of the alimentary canal. The increase could be measured at a single point in time or over a period of time, e.g. taking an average. ORP can be measured by conventional means, for example by the method described in Example 3.

In some embodiments, compositions of the invention may be formulated to provide a transient elevation of ORP in the digestive tract of an animal (e.g. the stomach, e.g. the rumen of a ruminant). In particular, a composition of the invention may elevate ORP to at least about 0 mV; preferably to at least about +50 mV; preferably at least about +100 mV; most preferably at least about +150 mV for a period of time. The period of time might be about 1 hour, or about 2 hours, or about 3 hours. The period of time might be less than about 4 hours, or less than about 3 hours, or less than about 2 hours. For example, a composition of the invention may be formulated to provide an elevation of ORP to at least +50 mV for a period of less than 4 hours.

In some embodiments, compositions of the invention provide a particular ORP profile in the digestive tract of an animal (e.g. the stomach, e.g. the rumen of a ruminant). The compositions of the invention may be formulated to provide a dose of oxidising agent which increases ORP to at least a certain potential above the initial value (the average), for at least a certain period of time. For example in some preferred embodiments the composition is formulated to provide a dose of oxidising agent which increases ORP by about +50 mV (from initial to the peak ORP) and the ORP may remain elevated by about +50 mV for an initial period of at least about 1 hour; preferably the invention increases ORP by about +100 mV (from initial to the peak ORP) and the ORP remains elevated by about +100 mV for an initial period of at least about 1 hour; preferably the invention increases ORP by about +150 mV (from initial to the peak ORP) and the ORP remains elevated by about +150 mV for an initial period of at least about 1 hour; preferably the invention increases ORP by about +200 mV (from initial to the peak ORP) and the ORP remains elevated by about +200 mV for an initial period of at least about 1 hour; preferably the invention increases ORP by about +250 mV (from initial to the peak ORP) and the ORP remains elevated by about +250 mV for an initial period of at least about 1 hour; preferably the invention increases ORP by about +300 mV (from initial to the peak ORP) and the ORP remains elevated by about +300 mV for an initial period of at least about 1 hour; preferably the invention increases ORP by about +350 mV (from initial to the peak ORP) and the ORP remains elevated by about +350 mV for an initial period of at least about 1 hour. For example a composition of the invention may be formulated to provide a dose of oxidising agent which increases ORP (e.g. in the rumen of a ruminant) by about +100 mV (from initial to the peak ORP) and the ORP may remain elevated by about +100 mV for an initial period of at least about 1 hour.

The compositions of the invention may be formulated to provide a dose of oxidising agent which increases ORP by about +50 mV (from initial to the peak ORP) and the ORP may remain elevated by about +50 mV for an initial period of at least about 4 hours; preferably the invention increases ORP by about +100 mV (from initial to the peak ORP) and the ORP remains elevated by about +100 mV for an initial period of at least about 4 hours; preferably the invention increases ORP by about +150 mV (from initial to the peak ORP) and the ORP remains elevated by about +150 mV for an initial period of at least about 4 hours; preferably the invention increases ORP by about +200 mV (from initial to the peak ORP) and the ORP remains elevated by about +200 mV for an initial period of at least about 4 hours; preferably the invention increases ORP by about +250 mV (from initial to the peak ORP) and the ORP remains elevated by about +250 mV for an initial period of at least about 4 hours; preferably the invention increases ORP by about +300 mV (from initial to the peak ORP) and the ORP remains elevated by about +300 mV for an initial period of at least about 4 hours; preferably the invention increases ORP by about +350 mV (from initial to the peak ORP) and the ORP remains elevated by about +350 mV for an initial period of at least about 4 hours. For example a composition of the invention may be formulated to provide a dose of oxidising agent which increases ORP (e.g. in the rumen of a ruminant) by about +100 mV (from initial to the peak ORP) and the ORP may remain elevated by about +100 mV for an initial period of at least about 2 hours.

The invention might increase ORP by about +50 mV (from initial to the peak ORP) and the ORP may remain elevated by about +50 mV for an initial period of at least about 8 hours; preferably the invention increases ORP by about +100 mV (from initial to the peak ORP) and the ORP remains elevated by about +100 mV for an initial period of at least about 8 hours; preferably the invention increases ORP by about +150 mV (from initial to the peak ORP) and the ORP remains elevated by about +150 mV for an initial period of at least about 8 hours; preferably the invention increases ORP by about +200 mV (from initial to the peak ORP) and the ORP remains elevated by about +200 mV for an initial period of at least about 8 hours; preferably the invention increases ORP by about +250 mV (from initial to the peak ORP) and the ORP remains elevated by about +250 mV for an initial period of at least about 8 hours; preferably the invention increases ORP by about +300 mV (from initial to the peak ORP) and the ORP remains elevated by about +300 mV for an initial period of at least about 8 hours; preferably the invention increases ORP by about +350 mV (from initial to the peak ORP) and the ORP remains elevated by about +350 mV for an initial period of at least about 8 hours. For example a composition of the invention may be formulated to provide a dose of oxidising agent which increases ORP (e.g. in the rumen of a ruminant) by about +100 mV (from initial to the peak ORP) and the ORP may remain elevated by about +100 mV for an initial period of at least about 3 hours.

Many embodiments described herein mention minimum ORP increases. An additional preferred feature of such embodiments is a maximum ORP increase, such that the compositions and methods provide an ORP increase that falls within a target range, thus avoiding excessive increases. For example in some preferred embodiments the ORP increase is not more than about 100 mV, about 150 mV, about 200 mV, about 250 mV, about 300 mV, about 350 mV, or about 400mV above the target minimum value. In a preferred embodiment the ORP increase is not more than about 200 mV above the target minimum value. For instance, in an embodiment which provides an increase in ORP of about +50 mV (from initial to the peak ORP) for a period of time, then in some preferred embodiments the ORP increase is from about +50 mV to about +150 mV. Setting a maximum ORP is a preferred embodiment for all ORP increases mentioned (e.g. about 100 mV, about 150 mV, about 200 mV, about 250 mV, about 300 mV, about 350 mV, or about 400mV above the target minimum value).

In one aspect, the invention provides a composition comprising an oxidising agent, wherein the composition is formulated to provide a dose of oxidising agent which increases and/or maintains the ORP in the digestive tract of an animal (e.g. the rumen of a ruminant) as described in any of preceding embodiment.

In preferred embodiments, about 0.05% (by weight) of a composition of the invention is the oxidising agent; preferably about 0.1%; preferably about 0.15%; preferably about 0.3%; preferably about 0.6%; preferably about 1.2%; preferably about 2% preferably about 2.5%;. For example, about 1.2% (by weight) of a composition of the invention might be the oxidising agent.

In preferred embodiments, at least about 0.05% (by weight) of a composition of the invention is the oxidising agent; preferably at least about 0.1%; preferably at least about 0.15%; preferably at least about 0.3%; preferably at least about 0.6%; preferably about 1.2%; preferably at least about 2%; preferably at least about 2.4%. For example, at least about 1.2% (by weight) of a composition of the invention might be the oxidising agent.

In some embodiments, about 0.05% to about 10% (by weight) of a composition of the invention is the oxidising agent; preferably about 0.1% to about 5%; preferably about 0.5% to about 2.5%; preferably about 1% to about 2%.

In other embodiments, substantially all of the composition of the invention is the oxidising agent. For example, greater than about 90%, greater than about 95%, greater than about 99% or about 100% (by weight) of a composition of the invention might be the oxidising agent.

In a particularly preferred embodiment, compositions of the invention comprise about 25 g of calcium peroxide. In another particularly preferred embodiment, compositions of the invention comprise about 50 g of calcium peroxide. In another particularly preferred embodiment, compositions of the invention comprise about 100 g of calcium peroxide. In another particularly preferred embodiment, compositions of the invention comprise about 150 g of calcium peroxide. In another particularly preferred embodiment, compositions of the invention comprise about 200 g of calcium peroxide. In another particularly preferred embodiment, compositions of the invention comprise about 300 g of calcium peroxide.

*Further components in compositions:*

Compositions of the invention may further comprise a peroxidase or catalase and/or a source of peroxidase, such as a peroxidase-producing organism (e.g. yeast). Introduction of a peroxidase or catalase to compositions of the invention may allow for the accelerated decomposition of peroxide into oxygen. This has the advantage of maximising ORP increase whilst minimising peroxide accumulation and any negative effects associated with peroxide accumulation, for example defaunation of the rumen. Alternatively, this might be achieved using non-biological catalysts (e.g. metal ions such as iron) which accelerate the decomposition of peroxide into oxygen. Accordingly, compositions of the invention may further comprise a peroxidase, a source of peroxidase, a peroxidase-producing organism, and/or a non-biological catalyst (which accelerates the decomposition of peroxide into oxygen).

The compositions of the invention may further comprise additional active ingredients, for example additional anti-methanogenic agents (i.e. substances or compositions which reduce the production of methane from an animal). Some additional anti-methanogenic agents are, for example, 3-nitrooxypropanol (3NOP), Coenzyme M analogs, halogenated aliphatic C1-C2 hydrocarbons e.g. bromoforms; Pterin compounds, Hydroxymethylglutaryl-CoA (HMG-S-CoA) reductase inhibitors, fats and fatty acids, plant secondary metabolites e.g. tannins, flavonoids, organosulphur compounds, essential oils, allicin, alternative hydrogen sinks e.g. nitrate and sulfate, nitrocompounds, propionate and butyrate enhancers, unsaturated organic acids, inhibitors to hydrogen-producing bacteria e.g. ionophores or bacteriocins. A preferred anti-methanogenic agent is 3NOP. In some embodiments the further active ingredients are same formulation as the present composition (e.g. mixed in a unit dosage formulation). However in other embodiments the composition comprises one part comprising an oxidising agent as described herein and at least one other part comprising the further active ingredient(s) as described herein, and preferably the two parts can be administered separately, simultaneously, or sequentially.

In some preferred embodiments, the compositions of the invention comprise only one anti-methanogenic agent. In some embodiments the compositions do not comprise additional anti-methanogenic agents. For example, methods of the invention may be substantially free of 3NOP. In one aspect, the invention provides a composition for oral administration to an animal comprising an oxidising agent and catalase (preferably wherein the composition is free of a source of iodide).

In preferred embodiments the compositions of the invention comprise an oxidising agent which is a source of peroxide and further comprise a peroxidase. Various peroxidases are known in the art and generally include (but are not limited to) haem peroxidases, haloperoxidases, cytochrome c peroxidases, glutathione peroxiredoxin, vanadium bromoperoxidase, manganese peroxidase, NADH peroxidase.

In some preferred embodiments, the oxidising agent and/or the composition of the invention is substantially free of peroxidase.

The inventors have discovered that compositions which comprise both an oxidising agent (e.g. a source of oxygen, particularly a source of peroxide) and a peroxidase or catalase exhibit accelerated decomposition into oxygen. Such compositions have the advantage of increasing ORP by rapid release of oxygen whilst minimising oxidising agent concentration, e.g. peroxide accumulation. This can be favourable for avoiding, for example, negative effects associated with high peroxide levels or peroxide accumulation, for example defaunation of the rumen and negative effects on digestibility.

However, in some preferred embodiments, the natural decomposition of the oxidising agent in the absence of peroxidase or catalase is sufficient to achieve the effect of increasing ORP by rapid release of oxygen whilst minimising oxidising agent concentration, e.g. peroxide accumulation. Therefore, in some preferred embodiments, compositions of the invention are substantially free of catalase and/or peroxidase.

*The oxidising agent:*

Generally, methods of the invention involve administering an oxidising agent to an animal (e.g. a ruminant).

Normally, the oxidising agent is a source of oxygen.

The oxidising agent is preferably a source of peroxide. Sources of peroxide as sources of oxygen.

A source of peroxide can be selected from the list comprising (but not limited to) hydrogen peroxide, urea hydrogen peroxide, sodium percarbonate, magnesium peroxide, sodium peroxide, lithium peroxide and calcium peroxide, or a combination thereof. In some preferred embodiments a source of peroxide can be selected from the list comprising hydrogen peroxide, urea hydrogen peroxide, magnesium peroxide, sodium peroxide, lithium peroxide and calcium peroxide, or a combination thereof. In some preferred embodiments a source of peroxide can be selected from the list comprising hydrogen peroxide, urea hydrogen peroxide, magnesium peroxide, sodium peroxide and lithium peroxide or a combination thereof. In preferred embodiments a source of peroxide can be selected from the list comprising urea hydrogen peroxide, magnesium peroxide, sodium peroxide and lithium peroxide or a combination thereof. Metal ions may catalyse the decomposition of peroxide to oxygen.

In some preferred embodiments, thr source of peroxide (a source of oxygen) can be selected from the list comprising (but not limited to) hydrogen peroxide, urea hydrogen peroxide, sodium percarbonate, magnesium peroxide, sodium peroxide, zinc peroxide, lithium peroxide and calcium peroxide, or a combination thereof.

In particularly preferred embodiments, the source of peroxide (a source of oxygen) is selected from the list comprising (but not limited to) urea hydrogen peroxide, sodium percarbonate, magnesium peroxide, sodium peroxide, lithium peroxide and calcium peroxide, or a combination thereof.

A particularly preferred source of peroxide is urea hydrogen peroxide. The inventors have found urea hydrogen peroxide to be particularly effective and to have additional advantages e.g. on ammonia production.

Another particularly preferred source of peroxide is calcium peroxide. The inventors have discovered that calcium peroxide provides a desirable ORP increase in the rumen, particularly when administered orally.

The source of peroxide (a source of oxygen), is preferably in solid form. In some embodiments, this means that the source of peroxide is incorporated into the animal's feed as a solid ingredient. In some other embodiments, this means that the source of peroxide is formulated as a solid bolus for oral administration.

Other potential sources of oxygen include perfluorocarbons and endoperoxides.

Alternatively, enzymatic sources of oxygen can be used. Other biological catalysts, such as yeasts, may also be present.

In one aspect, the invention provides a method of reducing methane production from an animal, the method comprising orally administering a composition comprising urea hydrogen peroxide, magnesium peroxide, or a combination thereof to the animal; preferably, wherein the composition is substantially free of iodide or a source of iodide. For example, the invention preferably provides a method of reducing methane production from an animal, the method comprising orally administering a composition comprising urea hydrogen peroxide to the animal, wherein the composition is substantially free of iodide or a source of iodide. The inventors have discovered that oral administration of compositions comprising urea hydrogen peroxide, magnesium peroxide, or a combination thereof is particularly effective in reducing methane production, particularly in ruminants.

***Enzymatic oxidising agent systems:***

In some embodiments, the oxidising agent may be an enzymatic oxidising agent system. For instance the composition might comprise an enzyme and further components which collectively produce an oxidising effect (e.g. a source of oxygen, or a source of peroxide). In some embodiments, the composition comprises an enzymatic oxidising agent system which reacts with endogenous components in an animal to provide an oxidising effect (e.g. a source of oxygen, or a source of peroxide).

In preferred embodiments the oxidising agent is non-enzymatic. However, for embodiments where the oxidising agent in the composition of the invention is an enzymatic oxidising agent system, the enzymatic oxidising agent system preferably does not comprise a peroxidase, for example an animal heme-dependent peroxidase. Preferably, compositions of the invention are free of lactoperoxidase.

Enzymatic oxidising agent systems are known in the art. One preferred enzymatic oxidising agent system comprises an oxidoreductase enzyme and an appropriate substrate for that enzyme to produce hydrogen peroxide. Alternatively the substrate may be endogenous (and not present in the composition).

In some embodiments, the enzymatic oxidising agent system provides a reaction between glucose and glucose oxidase, and results in the production of gluconic acid and peroxide. Such systems are discussed in WO2012140272 which is incorporated by reference in its entirety.

In some embodiments the enzymatic oxidising agent system comprises an oxidoreductase enzyme and an appropriate substrate for that enzyme to produce hydrogen peroxide. The oxidoreductase may be glucose oxidase. Glucose oxidase can react with glucose to produce hydrogen peroxide. Similarly, galactose oxidase could also be used, reacting with galactose to produce hydrogen peroxide.

In some embodiments, peroxide may be produced from glucose by the action of glucose oxidase.

In some embodiments, the compositions of the invention may further comprise a disaccharide sugar. The disaccharide could be hydrolysed by its corresponding glycoside hydrolase, for example sucrose and sucrase, allowing the release of monosaccharide sugars, which in turn could act as a source of additional hydrogen peroxide by the use of a corresponding oxidoreductase enzyme. Additionally, oligosaccharides or polysaccharides containing more than two sugar molecules, and that are cleaved to produce a source of hydrogen peroxide may be added.

In some embodiments, the compositions of the invention comprise two enzymes to derive a source of hydrogen peroxide; a glycoside hydrolase to break down disaccharide sugars into constituent monosaccharides, and a further oxidoreductase enzyme that reacts with the monosaccharide sugars to release hydrogen peroxide.

In some embodiments, compositions may further comprise additional sources of hydrogen peroxide. For example, an additional source of hydrogen peroxide is the exogenous addition of a solution of hydrogen peroxide or its release by a suitable perhydrate, such as sodium percarbonate. Alternatively, or in addition, a number of enzymes can be used to produce hydrogen peroxide. Xanthine oxidoreductase/oxidase reacts with either hypoxanthine or xanthine to produce hydrogen peroxide. Therefore, xanthine oxidoreductase/oxidase and/or xanthine could be added to the composition producing hydrogen peroxide. Similarly, sugar alcohols can be reacted with their appropriate oxidase enzymes to produce a source of hydrogen peroxide. For example, glycerol oxidase reacts with glycerol to produce a source of hydrogen peroxide and, therefore, glycerol oxidase/glycerol could be added to the composition. Another example would be mannitol reacting with mannitol oxidase. Further to this, citric acid is known to release hydrogen peroxide, and therefore could also be added to the composition.

Similarly, L-amino acid oxidase is an enzyme that reacts with free amino acids to produce hydrogen peroxide. Likewise, its addition (with or without L-amino acid supplementation) could provide a source of hydrogen peroxide.

Alternative methods of producing hydrogen peroxide could be used by the varying of the oxidoreductase and sugar, by other enzymatic reactions (L-amino acid oxidase and L-amino acids, xanthine oxidoreductase/oxidase and xanthine/hypoxanthine), by the addition of a perhydrate such as sodium percarbonate, or the direct addition of a solution of hydrogen peroxide.

In some embodiments, the source of peroxide could be rumen microbes such as lactic acid bacteria which secrete hydrogen peroxide. Various other bacteria in the rumen, such as streptococci, could act to provide an endogenous source of hydrogen peroxide. The peroxide produced by such bacteria can in turn act as a source of oxygen by decomposition of the peroxide.

In some preferred embodiments the source of peroxide is not a rumen microbe or bacteria. For example, in some preferred embodiments the source of peroxide (a source of oxygen) is not lactic acid bacteria.

*Animal subjects:*

Compositions and methods of the invention are for use on animals. The invention is particularly useful in ruminants, and the most preferable subjects are cattle. Examples of ruminants include cattle, sheep, goats, deer, giraffes, antelopes, and camels. Preferably, the ruminants are cows or sheep.

In some embodiments, the animal is a ruminant which is silage-fed, i.e. the ruminant has been silage fed prior to consuming a composition of the invention or being subject to a method of the invention. In some embodiments, the animal (e.g. ruminant) is grazing livestock, i.e. the animal (e.g. ruminant) has been pasture-fed prior to consuming a composition of the invention or being subject to a method of the invention. In some embodiments, the animal is grown intensively, i.e. the animal (e.g. ruminant) is being fed a balanced ration or a concentrated feed prior to consuming a composition of the invention or being subject to a method of the invention. In some embodiments, the animal is grown extensively, i.e. the animal (e.g. ruminant) is being fed by outdoor grazing on land prior to consuming a composition of the invention or being subject to a method of the invention. The invention is also suitable for use in other non-ruminant animals, in particular pigs. Such animals are non-ruminants but still produce significant quantities of methane.

In some preferred embodiments, the animal is not fistulated or cannulated. In particularly preferred embodiments, the animal is a ruminant (e.g. cow) which is not fistulated. For example, this might mean that the animal is a cow which has not been surgically fitted with a fistula or cannula.

*Animal Performance Enhancement*

In a further aspect, the invention provides a composition comprising an oxidising agent for improving animal performance. The composition may be any composition of the invention as described elsewhere herein. In preferred embodiments, the methods of reducing methane production from an animal described herein may further comprise improving animal performance.

In some embodiments, the invention encourages non-methanogenic microbiological pathways, which generate volatile fatty acids from excess hydrogen, rather than methane. In some embodiments, the non-methanogenic microbiological pathways may include fumarate reduction and/or fermentation. In some embodiments, reducing methanogenic activity may be accompanied by an increase in non-methanogenic activity which generates volatile fatty acids (e.g. acetogenic activity) and/or an increase in non-methanogenic microorganism populations (e.g. fermentative and fumarate-reducing microorganism populations) in the rumen. As a consequence of the increased non-methanogenic activity which generates volatile fatty acids (e.g. acetogenic activity) and/or increase in non-methanogenic microorganism populations (e.g. fermentative and fumarate-reducing microorganism populations) in the rumen, an improvement in animal performance may be observed, as more hydrogen may be diverted into producing volatile fatty acids rather than waste methane gas. The invention can encourage non-methanogenic microbiological pathways which generate acetate from excess hydrogen, rather than methane. In some embodiments, the non-methanogenic microbiological pathways may include acetogenic (e.g. homoacetogenic) microbiological pathways. Therefore a reduction in methanogenic activity may be accompanied by an increase in acetogenic (e.g. homoacetogenic) activity and/or an increase in acetogen (e.g. homoacetogen) populations in the rumen. As a consequence of the increased acetogenic (e.g. homoacetogenic) activity and/or increase in acetogen (e.g. homoacetogen) population, an improvement in animal performance may be observed, as more hydrogen may be diverted into producing acetate rather than waste methane gas.

The invention can encourage non-methanogenic microbiological pathways, which generate propionate and/or medium-chain fatty acids (e.g. valerate and/or caproate) from excess hydrogen, rather than methane. Therefore a reduction in methanogenic activity may be accompanied by an increase in microbial activity producing propionate and/or medium-chain fatty acids (e.g. valerate and/or caproate) and/or an increase in propionate- and/or medium-chain fatty acid-producing (e.g. valerate-producing and/or caproate-producing) microorganism populations in the rumen.

In a further aspect, the invention provides a method of improving animal performance, the method comprising orally administering a composition comprising an oxidising agent to an animal. The composition may be any composition of the invention as described elsewhere herein.

In preferred embodiments, improving animal performance is increasing milk production, increasing animal weight gain, improving milk quality from the animal, increasing volatile fatty acid content in the stomach (e.g. rumen) of the animal, reducing finishing times for the animal (e.g. beef cattle), improving carcass quality, improving feed efficiency, and/or improving a feed recuperation ratio. In preferred embodiments, any such improvement might be measured relative to an animal which is not subject to the methods, compositions, or uses of the invention.

In preferred embodiments, the term "increasing milk production" may include increasing milk volume yield; and/or increasing milk solids yield. In preferred embodiments, the term "increase in milk volume yield" means an increase in milk volume compared to the average volume produced by a ruminant which is not subject to the invention. In preferred embodiments, the term "increase in milk solid yield" means an increase in milk solid mass compared to the average mass produced by a ruminant which is not subject to the invention.

In an embodiment, the method of the invention increases milk volume yield by at least 1%. In an embodiment, the method of the invention increases milk volume yield by at least 2%. In an embodiment, the method of the invention increases milk volume yield by at least 5%. In an embodiment, the method of the invention increases milk volume yield by at least 10%. In an embodiment, the method of the invention increases milk volume yield by at least 15%. In an embodiment, the method of the invention increases milk volume yield by at least 20%. In an embodiment, the method of the invention increases milk volume yield by or at least 25%.

In another embodiment, the method of the invention increases milk volume yield by about 1% to about 2%. In an embodiment, the method of the invention increases milk volume yield by about 1% to about 5%. In an embodiment, the method of the invention increases milk volume yield by about 1% to about 10%. In an embodiment, the method of the invention increases milk volume yield by about 1% to about 15%. In an embodiment, the method of the invention increases milk volume yield by or about 1% to about 20%. In an embodiment, the method of the invention increases milk volume yield by about 1 % to about 25%.

In an embodiment, the method of the invention increases milk solids yield by at least 1%. In an embodiment, the method of the invention increases milk solids yield by at least 2%. In an embodiment, the method of the invention increases milk solids yield by at least 5%. In an embodiment, the method of the invention increases milk solids yield by at least 10%. In an embodiment, the method of the invention increases milk solids yield by at least 15%. In an embodiment, the method of the invention increases milk solids yield by at least 20%. In an embodiment, the method of the invention increases milk solids yield by at least 25%.

In embodiment, the method of the invention increases milk solid yield by about 1% to about 2%. In embodiment, the method of the invention increases milk solid yield by about 1% to about 5%. In embodiment, the method of the invention increases milk solid yield by about 1% to about 10%. In embodiment, the method of the invention increases milk solid yield by about 1% to about 15%. In embodiment, the method of the invention increases milk solid yield by or about 1% to about 20%. In embodiment, the method of the invention increases milk solid yield by about 1 % to about 25%.

In some embodiments, "reducing finishing time" means a reduction in the time taken to reach a final pre-determined weight prior to slaughter, compared to the average finishing time of an animal which is not subject to a method of the invention. The method of the invention may reduce the finishing time by at least 1 day, at least 2 days, at least 5 days, at least 10 days, or at least 20 days.

In some embodiments the invention may also provide an increase in volatile fatty acids (VFAs) in the rumen. In preferred embodiments the concentration of one or more VFAs selected from acetate, propionate, butyrate, valerate, and/or caproate may increase when a method of the invention is used. In preferred embodiments, the method of the invention provides an increase in VFA concentration of at least about 1%. Preferably, the method of the invention provides an increase in VFA concentration of at least about 5%. Preferably, the method of the invention provides an increase in VFA concentration of at least about 10%. These VFAs, which may be produced from microbial digestion in animals such as ruminants, might be important indicators of VFA production because they are the most rapidly metabolized. In some embodiments, an increase in VFA is an indication of an increase in digestion in an animal, and is thus a desirable result.

In preferred embodiments, the invention may comprise administering a second component which accelerates an increase in acetogenic activity and/or an increase in acetogen population. In preferred embodiments the second component comprises a probiotic.

In preferred embodiments, the second component may comprise a live culture of acetogens. For example the second component may comprise at least one of the following, or combinations thereof: acetitomaculum ruminis, acetoanaerobium noterae, acetobacterium bakii, acetobacterium carbinolicum, acetobacterium dehalogenans, acetobacterium fimetarium, acetobacterium malicum, acetobacterium paludosum, acetobacterium tundrae, acetobacterium wieringae, acetobacterium woodii, acetohalobium arabaticum, acetonema longum, alkalibaculum bacchi, blautia coccoides ga-1a, blautia hydrogenotrophica, blautia producta u-1a, blautia schinkii, butyribacterium methylotrophicum a, calderihabitans maritimus, carboxydothermus ferrireducens, carboxydothermus hydrogenoformans, carboxydothermus pertinax, clostridium aceticum, clostridium autoethanogenum a, clostridium carboxidivorans, clostridium coskatii a, clostridioides, difficile 630a, clostridium drakei, clostridium formicaceticum, clostridium ljungdahlii, clostridium magnum, clostridium methoxybenzovorans, clostridium ragsdalei, clostridium scatologenes, desulfotomaculum thermobenzoicum subsp. thermosyntrophicum, eubacterium aggregans, eubacterium limosum, fuchsiella alkaliacetigena, fuchsiella ferrireducens, holophaga foetidac, marvinbryantia formatexigens, moorella glycerinic, moorella mulderi, moorella thermoacetica, moorella thermoautotrophica, oxobacter pfennigii, sporomusa acidovorans, sporomusa aerivorans, sporomusa malonica, sporomusa ovata, sporomusa paucivorans, sporomusa rhizae, sporomusa silvacetica, sporomusa sphaeroides, sporomusa termitida, terrisporobacter glycolicus rd-1a, terrisporobacter mayombei, thermacetogenium phaeum, thermoanaerobacter kivui, and treponema primitia. The second component may also comprise ingredients which stimulate acetogen growth.

In preferred embodiments, the second component may comprise a live culture of fumarate reducers. For example the second component may comprise microorganisms of at least one of the following phyla: proteobacteria, fusobacteria, and firmicutes. For example, the second component may comprise at least one of the following, or combinations thereof: mitsuokella jalaludinii, enterococcus faecalis, enterococcus faecium, fibrobacter succinogenes, selenomonas lactilyca, selenomonas ruminantium, veillonella parvula and wolinella succinogenes. The second component may also comprise ingredients which stimulate fumarate reducer growth (e.g. fumarate).

In a further aspect, the invention provides a method of improving animal performance, the method comprising orally administering to an animal an additional anti-methanogenic agent and/or an oxidising agent and a second component which accelerates an increase in non-methanogenic activity which generates volatile fatty acids and/or an increase in non-methanogenic microorganism populations (e.g. fermentative and fumarate-reducing microorganism populations). In a preferred embodiment, the invention provides a method of improving animal performance, the method comprising orally administering to a ruminant an additional anti-methanogenic agent and/or an oxidising agent and a second component which accelerates an increase in acetogenic activity and/or an increase in acetogen population. Some additional anti-methanogenic agents are known in the art, for example 3-nitrooxypropanol (3NOP), Coenzyme M analogs, halogenated aliphatic C1-C2 hydrocarbons e.g. bromoforms; Pterin compounds, Hydroxymethylglutaryl-CoA (HMG-S-CoA) reductase inhibitors, fats and fatty acids, plant secondary metabolites e.g. tannins, flavonoids, organosulphur compounds, essential oils, allicin, alternative hydrogen sinks e.g. nitrate and sulfate, nitrocompounds, propionate and butyrate enhancers, unsaturated organic acids, inhibitors to hydrogen-producing bacteria e.g. ionophores or bacteriocins, seaweeds and seaweed extracts. A preferred anti-methanogenic agent is 3NOP.

In a preferred embodiment, the invention provides a method of improving animal performance, the method comprising orally administering 3NOP and optionally a second component which accelerates an increase in acetogenic activity and/or an increase in acetogen population (e.g. a probiotic) to an animal (e.g. a ruminant).

In some preferred embodiments, methods of the invention increase acetogen (e.g. homoacetogen) populations in the stomach (e.g. rumen), e.g. by more than about 10%. Preferably, methods of the invention increase acetogen (e.g. homoacteogen) populations in the stomach (e.g. rumen) by more than about 20%. Preferably, methods of the invention increase rumen acetogen populations in the stomach (e.g. rumen) by more than about 25%. Preferably, methods of the invention increase acetogen populations in the stomach (e.g. rumen) by more than about 30%. In some embodiments acetogen populations are measured by conventional means, for example, taking samples, and using microscopy to count acetogens.

In some embodiments the invention achieves an improvement in animal performance (e.g. weight gain) over time after repeated administration of the invention. For example the improvement in animal performance (e.g. weight gain) may be achieved in a maintenance phase.

In some embodiments there is a temporary reduction in animal performance (e.g. weight loss), for example, during a correction phase, followed by a long term improvement in animal performance (e.g. weight gain), for example, during a maintenance phase.

Without wishing to be bound by theory, any temporary or transient reduction in animal performance (e.g. weight loss) may be caused by hydrogen accumulation. In some embodiments a period of hydrogen accumulation is followed by a period of increased non-methanogenic microbiological pathways, and a long term reduction in methane production.

The invention also relates to the following additional numbered embodiments:

1. A composition comprising an oxidising agent for improving animal performance.

2. A method of improving animal performance, the method comprising orally administering a composition comprising an oxidising agent to an animal.

3. A method of improving animal performance, the method comprising orally administering to an animal an additional anti-methanogenic agent and/or an oxidising agent and a second component which accelerates an increase in non-methanogenic activity which generates volatile fatty acids and/or an increase in non-methanogenic microorganism populations.

4. The method according to embodiment 3, wherein the increase in non-methanogenic activity comprises an increase in acetogenic activity.

5. The method according to embodiment 3 or 4, wherein the increase in non-methanogenic microorganism populations comprises an increase in acetogen population.

6. The method or composition according to any preceding embodiment wherein the composition is substantially free of iodide or a source of iodide.

7. The method or composition according to any preceding embodiment wherein the composition comprises a peroxidase or catalase, a peroxidase-producing organism, or a non-biological catalyst which catalyses the decomposition of peroxide.

8. The method or composition according to any preceding embodiment wherein the oxidising agent is an enzymatic oxidising agent system.

9. The method of any preceding embodiment wherein the animal is a ruminant.

10. The method of any preceding embodiment wherein the animal is a cow.

11. The method of embodiment 2 or 3 wherein the animal is a ruminant and the stomach is the rumen.

12. The method or composition according to any preceding embodiment wherein improving animal performance comprises one or more of: increasing milk production, increasing animal weight gain, improving milk quality from the animal, increasing volatile fatty acid content in the stomach (e.g. rumen) of the animal, reducing finishing times for the animal (e.g. beef cattle), improving carcass quality, improving feed efficiency, and improving a feed recuperation ratio.

13. The method or composition of any preceding embodiment wherein the oxidising agent is a source of oxygen ($O_2$).

14. The method or composition of embodiment 13 wherein the source of oxygen is a source of peroxide.

15. The method or the composition of any preceding embodiment, wherein the oxidising agent is a source of oxygen selected from hydrogen peroxide, urea hydrogen peroxide, sodium percarbonate, magnesium peroxide, sodium peroxide, lithium peroxide and calcium peroxide, or a combination thereof.

16. The method or composition of any preceding embodiment, wherein the oxidising agent is urea hydrogen peroxide.

17. The method or composition of any preceding embodiment, wherein the oxidising agent is calcium peroxide

18. The method or composition of any preceding embodiment, wherein the oxidising agent is peroxide-producing bacteria; optionally wherein the peroxide-producing bacteria is lactic acid bacteria.

19. The method of any preceding embodiment, wherein the method comprises adding the composition to food prior to oral administration.

20. The method of any preceding claim wherein the method comprises the steps of: (i) sprinkling the composition onto food prior to feeding; and (ii) feeding the mixture of food and composition to the ruminants.

21. The method or composition of any preceding embodiment, wherein the composition further comprises food components, optionally wherein the food components comprise maize, barley, soya, and/or molasses.

22. The composition or method according to any preceding embodiment wherein the composition comprises a coating comprising coconut oil, gylcerin, glycerol, silica hydrogels, poly(methyl methacrylate) capsulation, poly(D, L-lactide-co-glycolide) (PLGA), alginate, poly(vinyl pyrrolidone) PVP, hard fat, ethyl cellulose, N-isopropylacrylamide (NIPAAm), acrylic acid, hydroxyethyl methancrylate-oligo(hydroxybutyrate), polydimethylsiloxane (PDMS), methacrylamide chitosan, cyanoacrylate, sodium alginate, polyisobutylene, isobutyleneisoprene copolymers, styrene-butadiene copolymers, polyvinyl acetate, polyisoprene, polyethylene, vinyl acetate, or a combination thereof.

23. The composition or method according to any preceding embodiment wherein the composition comprises a coating comprising coconut oil.

24. The composition according to any preceding embodiment wherein the composition comprises iron oxide, zinc oxide, magnesium stearate, hydrogenated fat, or combinations thereof.

25. The method of any preceding embodiment wherein the composition increases the oxidation-reduction potential in the stomach of the animal at least about +100 mV.

26. The method of any preceding embodiment wherein oral administration of the composition elevates oxidation-reduction potential in the stomach of the animal for a period of at least about 1 hour following oral administration.

27. The method of any preceding embodiment wherein oral administration of the composition induces and maintains an oxidation-reduction potential in the stomach of from about -200 mV to about -100 mV for at least about 1 hour.

28. The method of any preceding embodiment wherein oral administration of the composition does not reduce pH of the stomach by more than 10%.

29. The method of any preceding embodiment, wherein the method further comprises administration of an additional anti-methanogenic agent.

30. The method of any preceding embodiment wherein the additional anti-methanogenic agent is 3-nitrooxypropanol.

31. The composition or method of any preceding embodiment wherein about 0.01 to about 0.1% by weight of the composition is the oxidising agent.

32. The composition or method of any preceding embodiment wherein about 0.05% to about 10% by weight of the composition is the oxidising agent; preferably about 0.1% to about 5%; preferably about 0.5% to about 2.5%; preferably about 1% to about 2%.

33. The composition or method of any preceding embodiment wherein substantially all of the composition is the oxidising agent.

34. The method of any preceding embodiment wherein the composition used is the composition of any preceding embodiment.

35. Use of a composition of any preceding embodiment for improving animal performance.

## Examples

Compositions and methods of the invention are illustrated by the following non-limiting examples.

### Example 1 - RUSITEC Composition Screening 1st Run

A well-established assay for the investigation of rumen, known as the rumen simulation technique (RUSITEC), is described by Czerkawski & Breckenridge (British Journal of Nutrition, 1997, Volume 38(3), pp. 371-384). The present inventors employed the RUSITEC system to screen the effects of a series of compositions of the invention as feed additives ex *vivo.*

*Experimental details*: An 800 ml vessel was used for the RUSITEC setups and for each feed additive/control screened, four replicate systems were employed. The results given below are the mean results across the four replicate experiments. Fresh rumen fluid and digesta were collected from >3 fistulated donor animals that had been acclimatised to a similar diet to that being tested in the vessels. The contents collected were pooled to eliminate variation. A minimum of 500ml of rumen fluid and 90g digesta were collected per vessel. The purpose of using digesta is to allow the microbes on the fibre mats to replicate and attach themselves to the newly introduced feed bags. For each experiment the feed composition, comprising either a control composition or a composition containing a feed additive, was added to the RUSITEC system in a porous bag. In addition, a second bag, containing grass silage (the "forage bag") was added to the vessel. 450 ml of rumen fluid, 350 ml of anaerobic saliva, the appropriate bag containing the feed composition, the forage bag, and 80g of the pooled digesta were added to each vessel. Prior to addition, the dry weight of each of the feed composition and forage bags was recorded. Artificial saliva was prepared according to McDougall (Biochemical Journal, 1948, Volume 43(1), pp. 99-109) and pumped into the vessel at a rate of 27.5 ml per hour. Each vessel was kept at a constant temperature of 39 °C for the duration of the experimental procedure. Overflow vessels were kept at 2 °C. The bag containing the feed composition was removed and replaced with a fresh bag every 24 hours, the forage bag was replaced every 48 hours. The following feed compositions were added to the RUSITEC system:

Reference Composition CO: Control composition (20 g) consisting of toasted barley, wheat distillers dried grains, dried grains, dried beet pulp, soya(bean) hulls, cane molasses, flaked maize, soya, sodium chloride, and calcium carbonate.

Reference Composition A ("LARS 1X"): Feed composition consisting of 0.54 g urea hydrogen peroxide, 0.06 g potassium iodide and Composition CO (20 g).

**Invention Composition B:** Feed composition consisting of 0.54 g urea hydrogen peroxide and Composition CO (20 g).

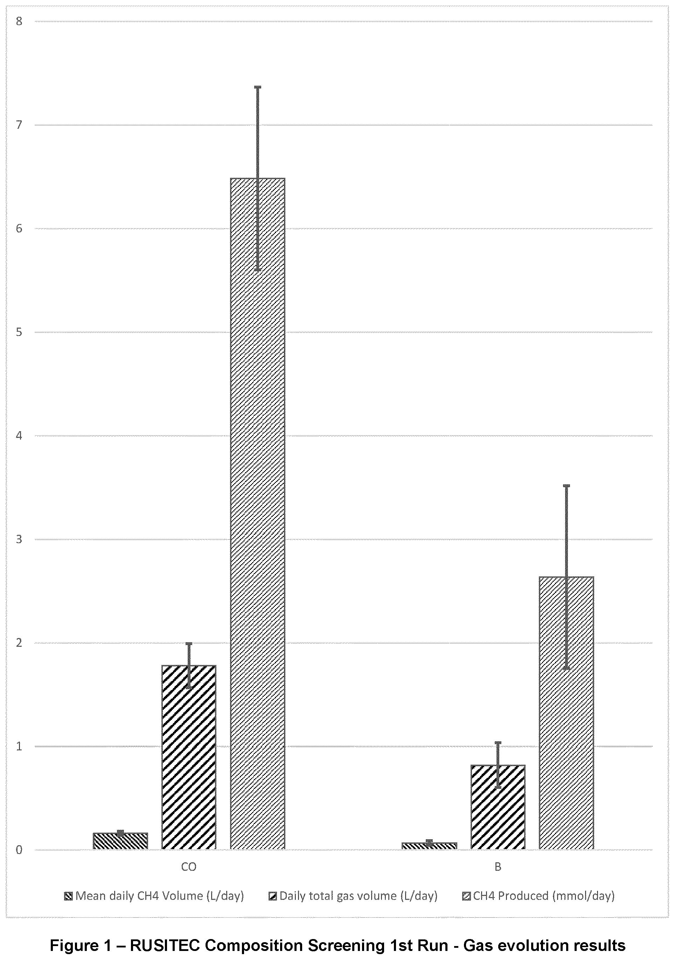

*Results 1.1:* Measurements included biogas production, in particular methane and ammonia production. The total volume of gas produced and in particular, the volumes of methane and ammonia which evolved from the system were monitored over 21 days. Additionally, the pH of the vessel and overflow rumen fluids were measured on a daily basis. Results are also shown in the appended Figure 1.

**Table 1.1 - RUSITEC Composition Screening 1st Run - Gas evolution & pH results**

| Composition | CO (Ref) | B | SEM | p-value |
|---|---|---|---|---|
| **Total daily gas volume evolved (L/day)** | 1.78 | 0.82 | 0.2148 | <.0001 |
| **Mean daily $CH_4$ volume evolved (L/day)** | 0.1585 | 0.0644 | 0.0216 | <.0001 |
| **$CH_4$ evolved (mmol/day)** | 6.4832 | 2.6339 | 0.8824 | <.0001 |
| **Vessel pH** | 6.48 | 6.46 | 0.0543 | <.0001 |
| **Overflow pH** | 6.6 | 6.79 | 0.0534 | <.0001 |
| **Reduction in daily $CH_4$ evolved relative to control (%)[2]** | 0 | 59 | n/a | n/a |

[1] Reduction in $CH_4$ proportion of total gas relative to control (%) = ($CH_4$ proportion of total gas for CO (%) - $CH_4$ proportion of total gas for X (%)) / $CH_4$ proportion of total gas for CO (%) x 100; wherein X is A, B, or C.

[2] Reduction in daily $CH_4$ produced relative to control (%) = ($CH_4$ produced for CO (mmol/day) - $CH_4$ produced for B (mmol/day)) / $CH_4$ produced CO (mmol/day) x 100.

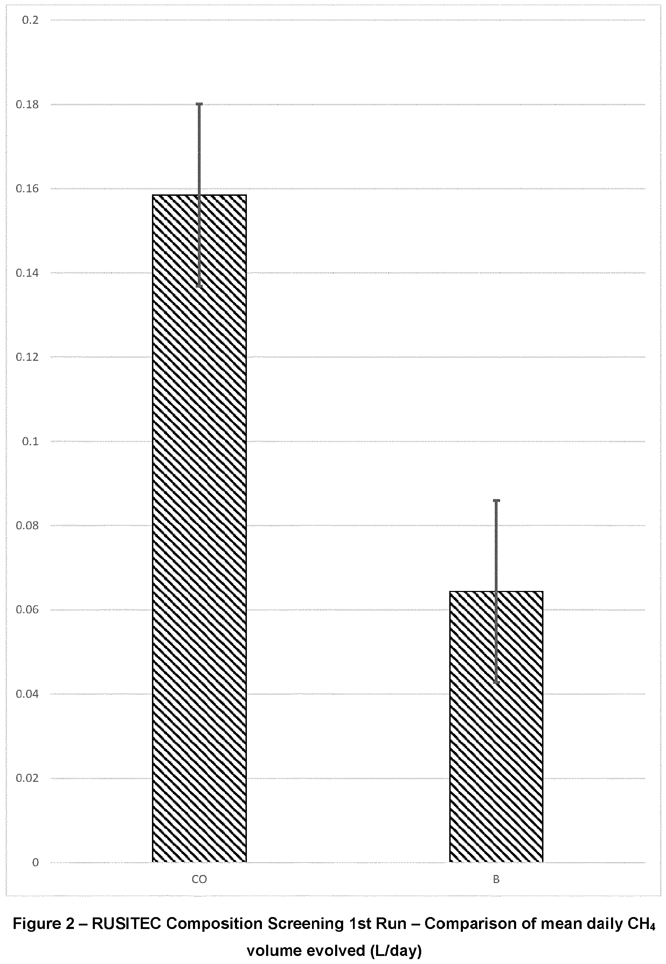

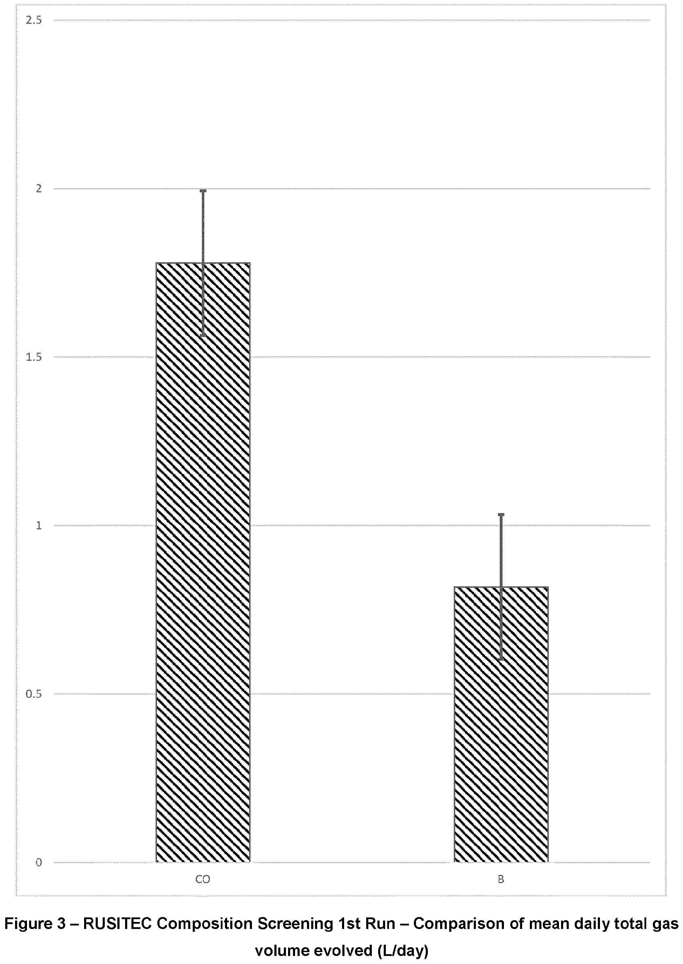

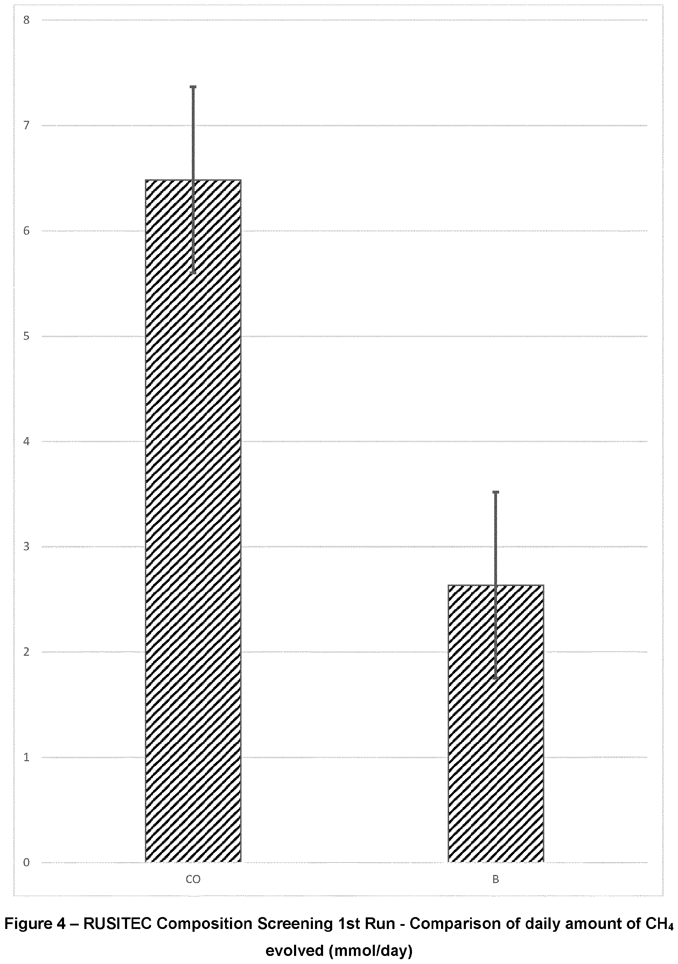

*Conclusions 1.1:* It was observed that Composition B, which is free of a source of iodide, achieved a significant reduction of total gas volume evolved relative to control (see Figure 3). Composition B also achieved a significant (greater than 50%) reduction in methane volume produced relative to control (see Figure 2). Composition B also achieved an overall reduction in the amount of methane produced in the rumen per day compared to control (see Figure 4).

*Results 1.2:* Cumulative volumes of $CH_4$ evolved from the RUSITEC over the 21 day experimental period were recorded.

**Table 1.2 - RUSITEC Composition Screening 1st Run - Gas evolution & pH results**

| Composition | Total cumulative $CH_4$ volume over 21 day period (L) | Standard Deviation | Decrease in total cumulative $CH_4$ volume produced relative to CO (%) |
|---|---|---|---|
| **CO** | 3.2902837 | 0.071317 | 0% |
| **B** | 1.291765 | 0.06701 | 61% |

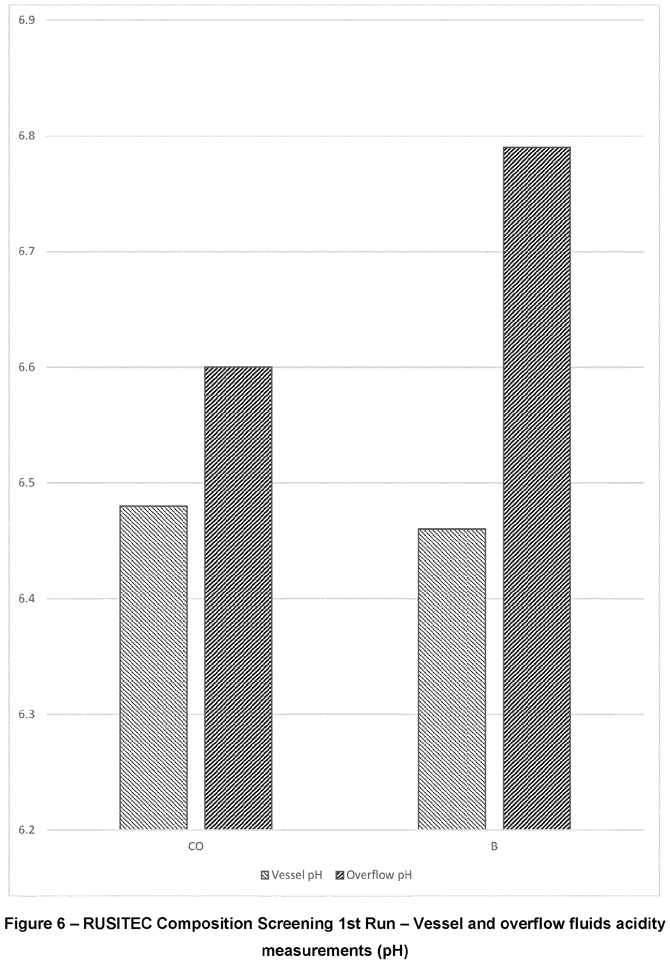

pH was also monitored and the results are shown in Figure 6.

*Conclusions 1.2:* As shown in Table 1.2, administration of Composition B significantly decreased the cumulative volume of methane evolved from the rumen over the 21 day trial period, relative to control. Notably, the decrease was in excess of 60% relative to control.

Notably, administration of Composition B did not lead to substantial acidification of the rumen compared to the control Composition CO, as shown in Figure 6. In particular, it was observed that Composition B, maintained a similar pH to the control Composition CO, in addition to the reduction in methane production described above.

This absence of substantial acidification is surprising. From the biogas production results, it is expected that the compositions which inhibit methane production in the rumen will also promote the metabolic formation of volatile fatty acids (without wishing to be bound by theory). A corresponding acidification of the rumen may therefore have been expected (which can negatively impact digestibility of feed in ruminants), but was not observed.

*Experimental 1.3:* The effect of each feed additive composition on dry-matter digestibility was assessed by measuring the change in dry weight of the feed composition, before and after the simulated digestion using the RUSITEC model as described above.

At each of the replacement intervals specified above, when a feed composition bag or forage bag was removed from the vessel, this was washed in 25ml saliva to detach as many microbes as possible and the washings returned to the vessel. For the final 7 days of the trial (15-21), subsequent to the saliva wash, the feed bag and forage bag were washed in a 30 minute rinse cycle in a washing machine with no heat, detergent, or spinning. Once washed, the bags were dried for 48 hours at a temperature of 55°C and the dry weight was recorded.

*Results 1.3:* Dry-matter digestibility was measured as described above for each of the screened compositions.

Table **1.3 - RUSITEC Composition Screening 1st Run - Dry-matter digestibility results**

| Composition | A (Ref) | B | CO (Ref) | SEM | P-Value |
|---|---|---|---|---|---|
| Forage digestibility[3] (%) | 62.28 | 69.42 | 75.39 | 4.591 | 0.242 |
| Pellet digestibility (%) | 70.22 | 71.79 | 69.75 | 1.643 | 0.913 |
| Average digestibility[4] (%) | 66.25 | 70.6 | 72.57 | 2.976 | 0.47 |
| [3] Digestibility (%) = (1 - (dry weight of feed material after digestion / dry weight of feed material prior to digestion)) x 100. [4] Average digestibility refers to the mean digestibility across both the pellet and forage compositions. | | | | | |

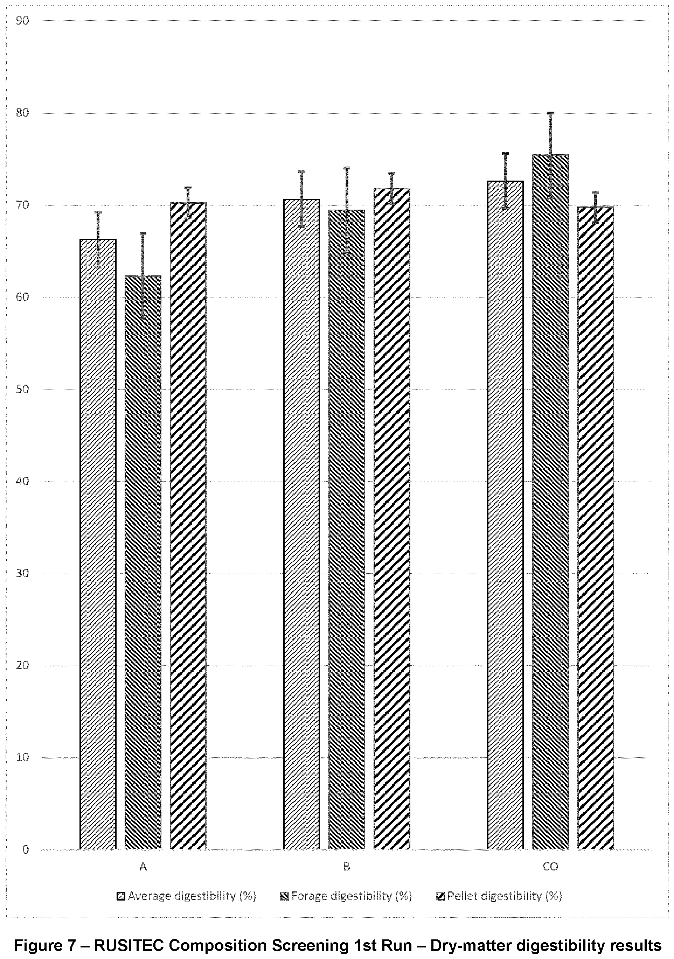

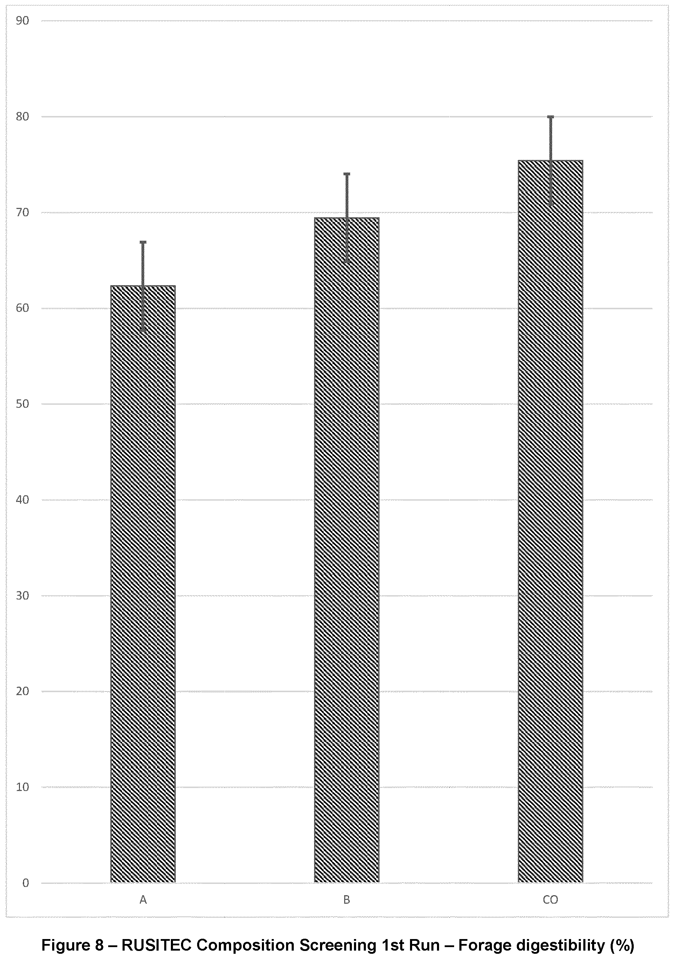

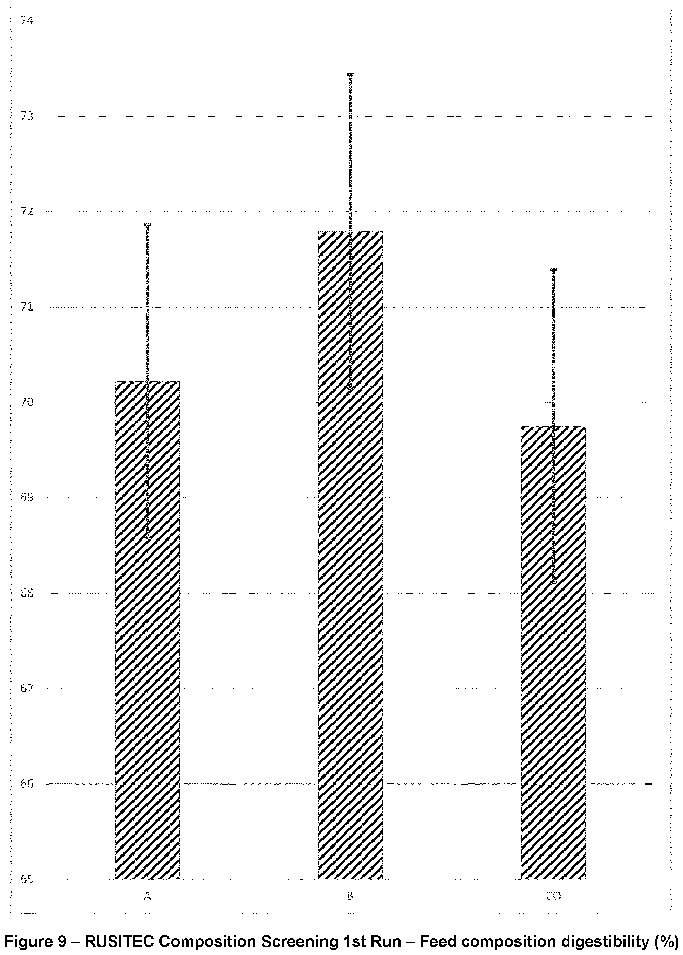

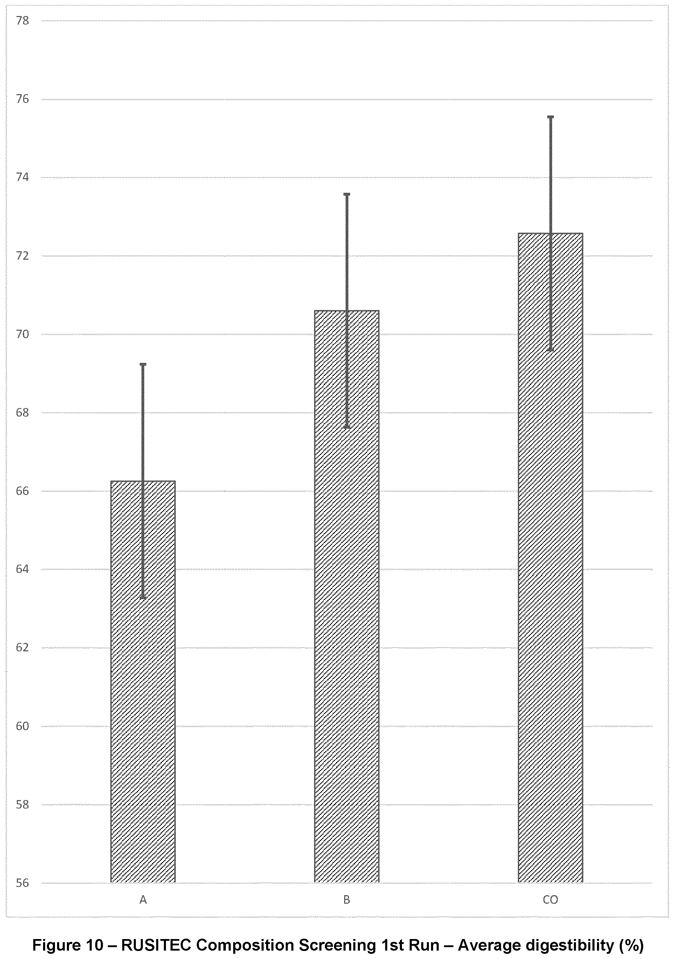

*Conclusions 1.3*: These results demonstrate that administration of Composition B does not appear to have a substantial negative impact on digestibility, as shown in Figures 7-10. Notably, it was observed that Composition B retains digestibility similar to the control Composition CO, and to a greater extent than the active control Composition A.

*Results 1.4:* The volume of ammonia which evolved from the system was monitored over 21 days.

Table **1.4** - **RUSITEC Composition Screening 1st Run - Ammonia evolution**

| Composition | A (Ref) | B | CO (Ref) | SEM | P-Value |
|---|---|---|---|---|---|
| Ammonia concentration per litre (mM) | 7.0556 | 8.0805 | 4.028 | 0.3661 | <0.001 |

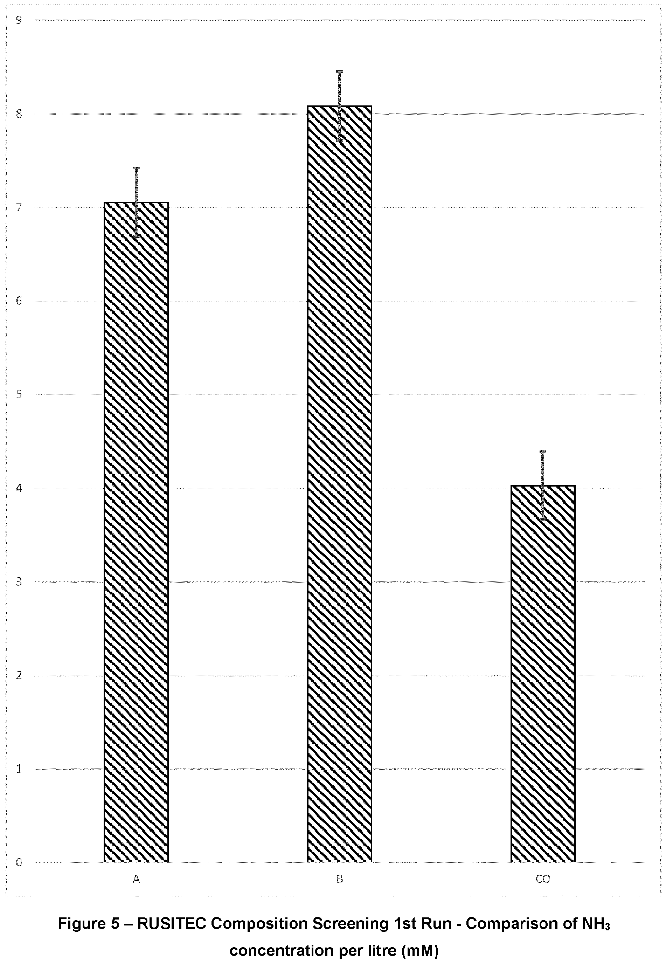

*Conclusions 1.4:* Administration of Compositions B achieved an overall increase in the amount of ammonia evolved from the rumen compared to the control Composition CO. Notably, it was observed that Composition B, achieved a greater increase relative to the control Composition CO, and than the active control Composition A, as shown in Figure 5.

Reduced ammonia production in the rumen has been linked to defaunation of the rumen. Accordingly the elevated levels ammonia production observed by the inventors for Composition B of the invention suggests that the methods and compositions of the invention avoid substantial defaunation of the rumen. Therefore, the inventors have shown that the methods and compositions of the invention can achieve a desirable reduction in methane production, surprisingly without causing substantial defaunation of the rumen, which may avoid some negative effects associated with rumen defaunation, for example reduction in digestibility.

*Results 1.5:* Amounts (millimoles per litre) of volatile fatty acids (VFAs) generated in the control composition experiment and the composition B experiment were measured on day 21.

Table **1.5** - **RUSITEC Composition Screening 1st Run** - **Volatile Fatty Acid Production**

| Composition | CO (Ref) | B | p-value |
|---|---|---|---|
| Caproate (mmol/L) | 1.3 | 1.9 | <.0001 |
| Valerate (mmol/L) | 6.2 | 9.5 | <.0001 |

## Example 2 - RUSITEC Composition Screening 2nd Run

The method of Example 1 was repeated using an additional series of compositions:

**Reference Composition CO:** Control composition (20 g) consisting of toasted barley, wheat distillers dried grains, dried grains, dried beet pulp, soya(bean) hulls, cane molasses, flaked maize, soya, sodium chloride, and calcium carbonate.

**Composition D** ("UHP 0.5X"): Feed composition consisting of 0.27 g urea hydrogen peroxide and Composition CO (20 g).

**Composition E** ("$MgO_2$ 0.5X"): Feed composition consisting of 0.162 g magnesium peroxide complex (wherein the complex comprises 24%-28% magnesium peroxide) and Composition CO (20 g).

**Composition G** ("UHP 0.25X"): Feed composition consisting of 0.135 g urea hydrogen peroxide Composition CO (20 g).

*Results 2.1:* Measurements included biogas production and pH, as described above.

**Table 2.1 - RUSITEC Composition Screening 2nd Run - Gas evolution results**

| Composition | CO (Ref.) | D | E | G | SEM | p-value |
|---|---|---|---|---|---|---|
| Total daily gas volume evolved (L/day) | 2.0813 | 1.0499 | 1.7291 | 1.7201 | 0.12 | <.0001 |
| Mean daily $CH_4$ volume evolved (L/day) | 0.2792 | 0.09205 | 0.2076 | 0.1532 | 0.0162 | <.0001 |
| $CH_4$ evolved (mmol/day) | 11.4174 | 3.7647 | 8.4916 | 6.2671 | 0.6626 | <.0001 |
| Vessel pH | 6.49 | 6.57 | 6.49 | 6.59 | 0.0316 | <.0001 |
| Overflow pH | 6.4 | 6.67 | 6.49 | 6.57 | 0.0304 | <.0001 |
| Reduction in daily $CH_4$ evolved relative to control (%) | 0 | 67 | 26 | 45 | | |

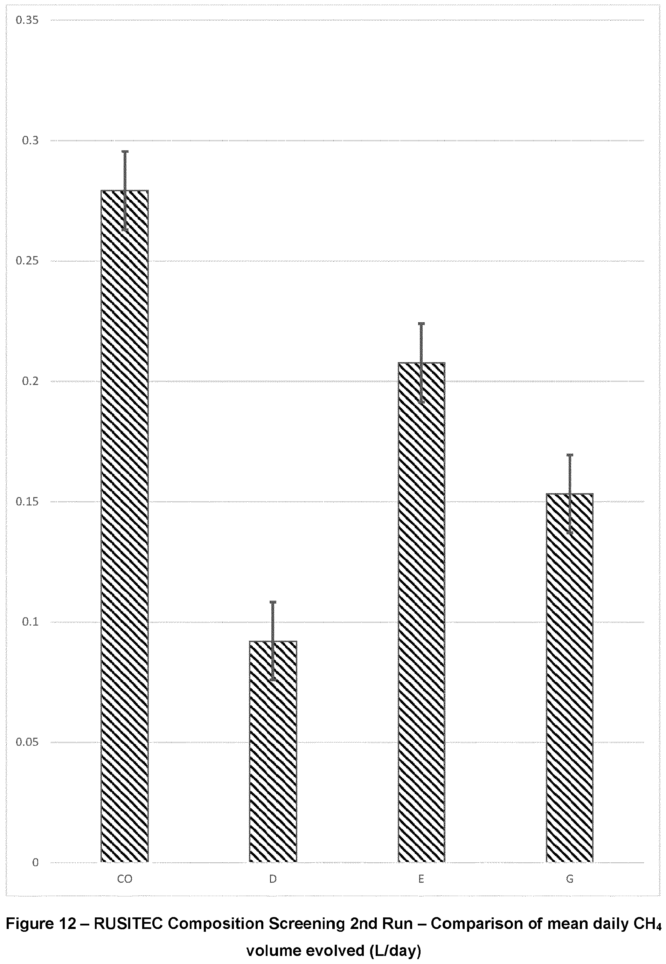

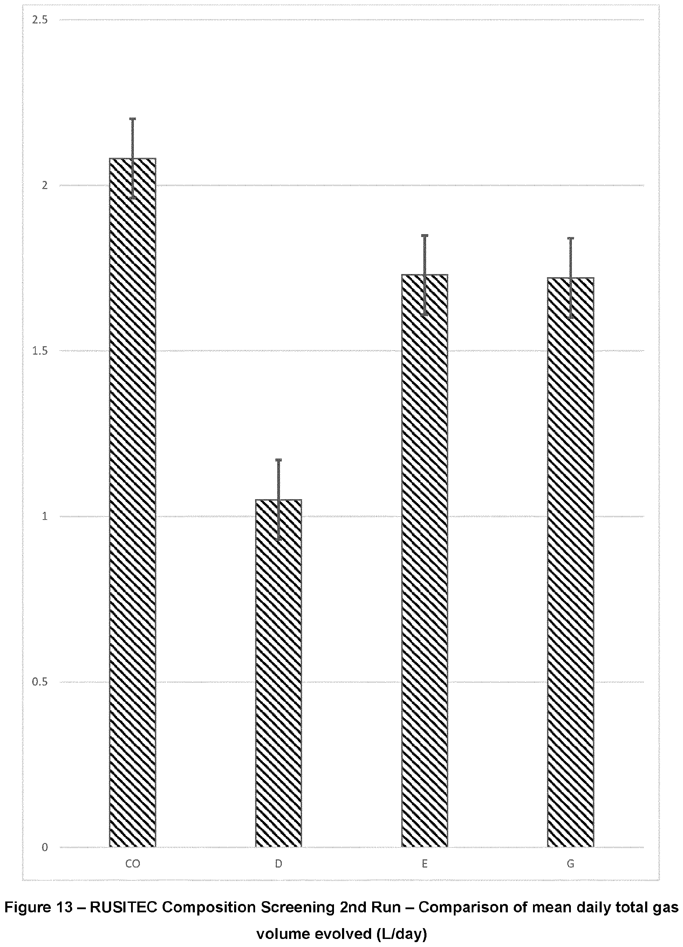

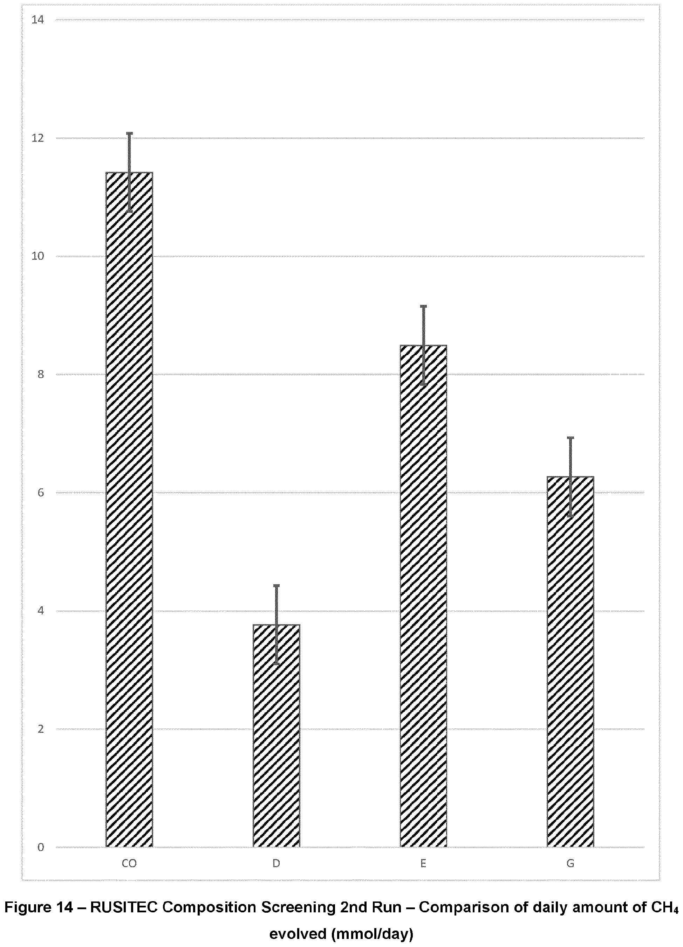

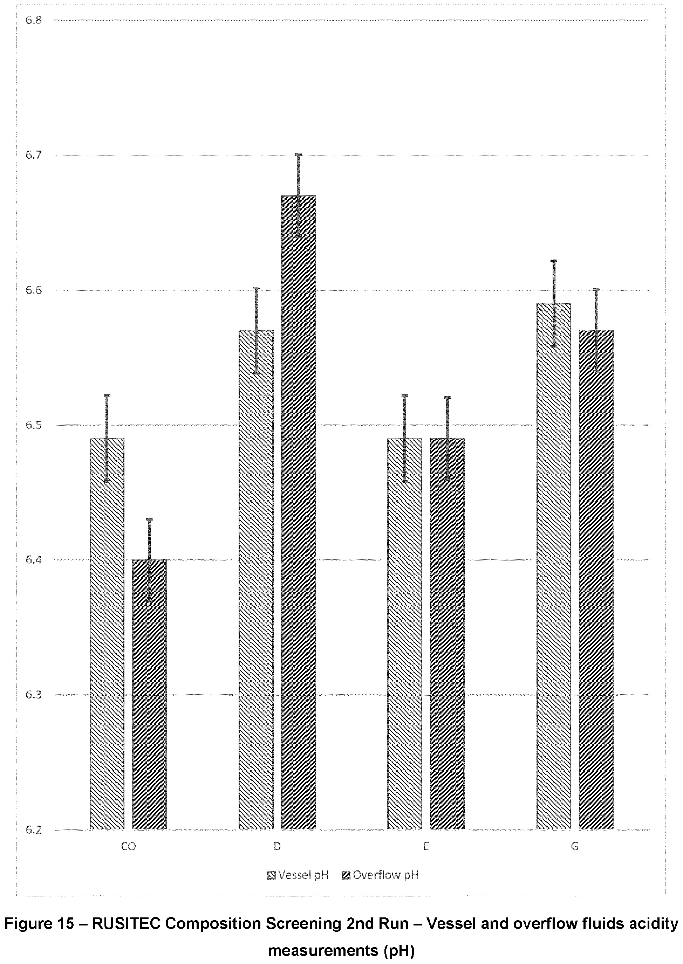

Administration of Compositions D, E, and G achieved a reduction relative to the control, in the total volume of gas evolved from the rumen. Composition D achieved the greatest reduction in total gas volume evolved relative to the control (see also Figure 13).
Administration of Compositions D, E, and G achieved an overall significant reduction in the volume of methane evolved from the rumen compared to the control Composition CO. Notably, Composition D achieved a greater than 50% reduction in methane volume produced relative to the control Composition CO (see also Figure 12).
Administration of Compositions D, E, and G achieved an overall significant reduction in the amount of methane produced in the rumen per day compared to the control Composition CO. Notably, Composition D, achieved a greater than 50% reduction in daily methane production relative to the control Composition CO (see also Figure 14).
Remarkably, administration of Compositions D, E, and G did not lead to significant acidification of the rumen compared to the control Composition CO, as shown in Figure 15. In particular, it was observed that Compositions D and G, resulted in an increase in pH relative to the control Composition CO, in addition to the reduction in methane production described above. These results suggest that Compositions D, E, and G may exhibit an inhibitory effect on methane production without causing acidification of the rumen and negatively impacting digestibility.
*Results 2.2:* Dry-matter digestibility was measured for each of the compositions screened as described above.

**Table 2.2 - RUSITEC Composition Screening 2nd Run - Dry-matter digestibility results**

| Treatment | D | E | G | CO | SEM | P-Value |
|---|---|---|---|---|---|---|
| Forage digestibility (%) | 68.64 | 78.37 | 71.01 | 77.44 | 1.6542 | <.0001 |
| Pellet digestibility (%) | 71.25 | 71.26 | 70.35 | 71.55 | 0.7059 | 0.0133 |
| Average digestibility (%) | 69.94 | 74.81 | 70.68 | 74.5 | 1.0823 | <.0001 |

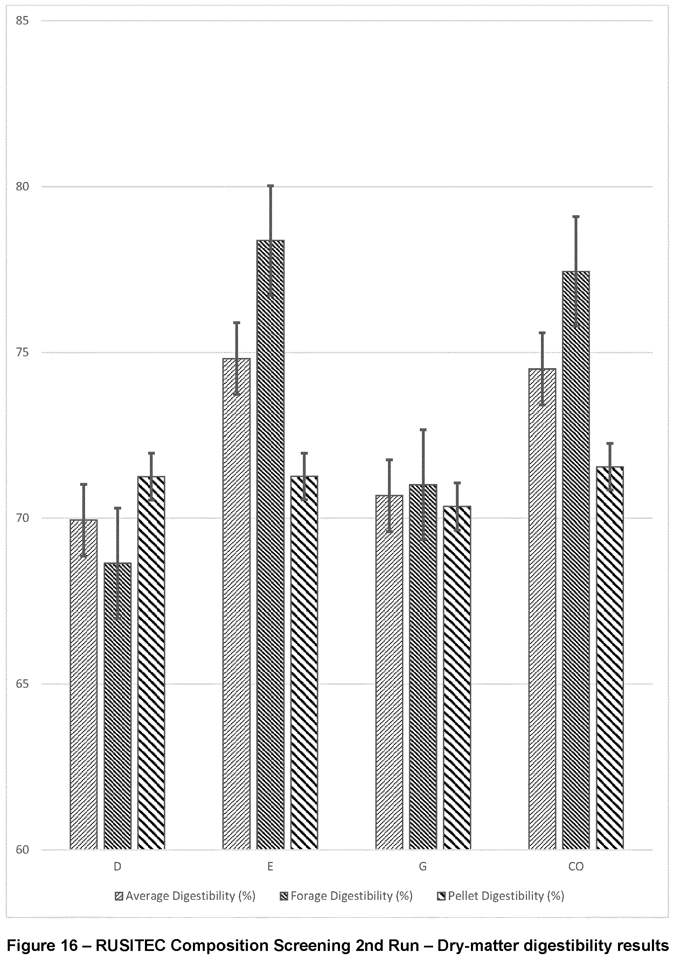

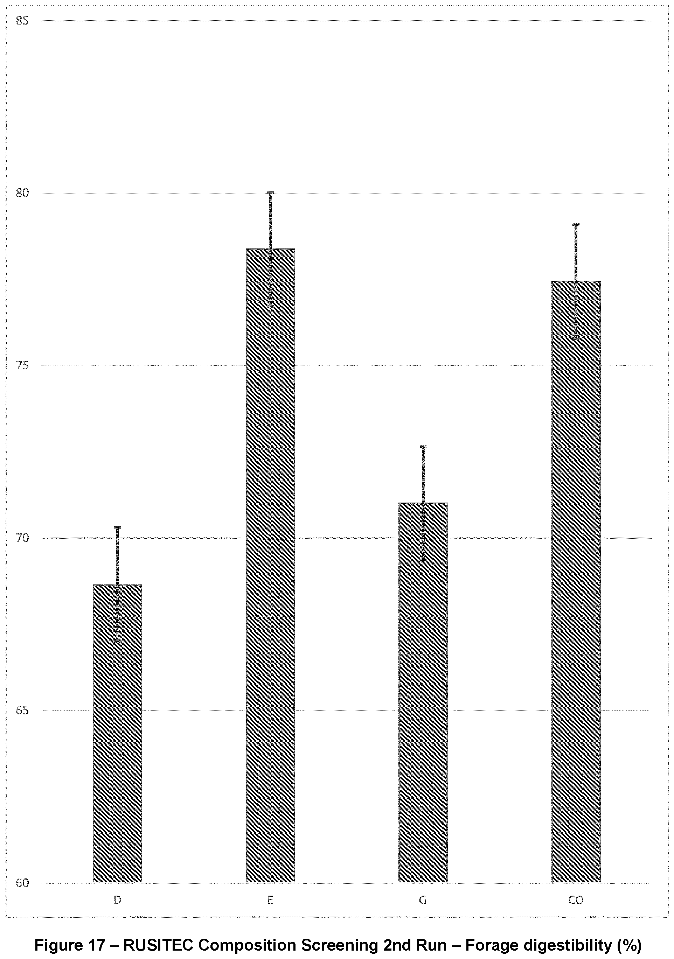

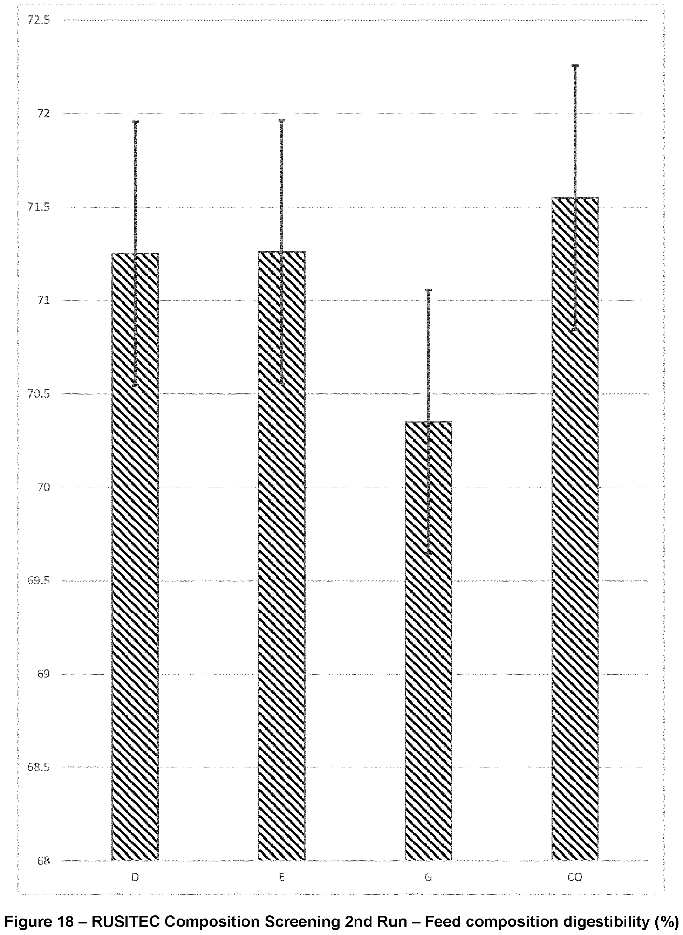

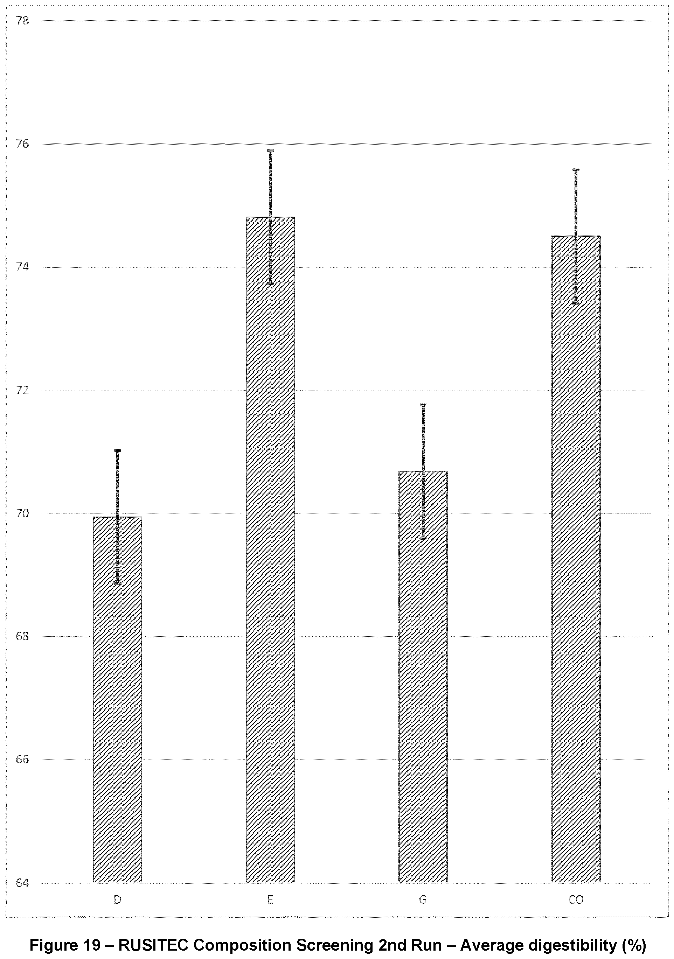

*Conclusions 2.2:* The dry-matter digestibility results demonstrate that administration of Compositions D, E, or G does not appear to have a substantial negative impact on digestibility, as shown in Figures 16-19.

***Illustrative Example 3 - ORP Assays***

ORP in the stomach of an animal (and the effect of a composition of the invention on ORP) can be measured by conventional means for ORP measurements. For instance, the following preferred method can be used: (i) an ORP meter bolus is prepared by adapting a commercially available pH bolus; (ii) the ORP meter bolus is administered; (ii) ORP measurements are initiated; (iii) the composition under investigation is administered; (iv) the effect of the composition on ORP over time is measured by taking ORP measurements at regular intervals.
For example, ORP in the stomach of an animal (e.g. rumen) can be measured using a bolus electrode, the bolus electrode being a capsule of about 100 mm x 20 mm that is inserted into the rumen via mouth, or via fistula. The bolus sends measurements to a control station data logger within a range of 500 m.

*Example 4 - ORP Data*

Initial experiments have demonstrated that compositions and methods of the invention increase ORP in the stomach of animals, particularly in the rumen of a ruminants. The inventors have observed that methanogenesis usually requires an ORP in the digestive tract (e.g. rumen) of about -200 mV or less. The inventors have also observed that compositions of the present invention (e.g. Compositions B, D, E, G of Examples 1 and 2) are capable of achieving an increase in ORP in the digestive tract of an animal of at least about +100 mV (i.e. elevating ORP to above about -100 mV) for a sustained period of time, e.g. greater than about 2-4 hours.

*Discussion:* Without wishing to be bound by theory, it is believed that these results confirm that the increase in ORP is responsible for the reduction in methanogenic activity without substantially reducing digestibility (and consequent retention of feed efficiency and animal performance). In particular, when using peroxide sources, the concentration of peroxide in the stomach remains low (since peroxide is released in a sustained fashion to ensure that rapid decomposition provides sustained supply of oxygen). The elevated ORP remains sustained, and therefore peroxide source compositions are acting as a source of oxygen, rather than any reduction in methanogenic activity caused by minor and negligible defaunation effects of the peroxide.

*Example 5 - In vivo methane emission studies*

*Experimental*

A methane emission study was carried out to determine the impact of feeding different doses of the feed additive would have on enteric fermentation. In general, animals (in this instance adult ewes, of approximate 80 kg body weight at trial commencement) were fed a diet of 500 g concentrate (30% maize meal, 30% barley, 16.5% soya hulls, 15.5%, soya bean meal, 5% molasses, 3% minerals and vitamins) at 8 am daily to which the feed additive was added. Any refused concentrate/additive was recorded. Sheep then had ad libitum access to grass silage and water throughout the day, with the amount of silage eaten recorded for each animal.

Body weights of all animals were recorded at the start of the experiment ('week 1') and after 49 days ('week 7').

Methane emissions were recorded through use of Portable Accumulation Chambers (PAC). Briefly: these are sealed chambers in which individual animals remain for a 1 hour time period. During this time period methane, oxygen and carbon dioxide measurements are taken at 0 minutes, 25 minutes and 50 minutes. These data points are adjusted for temperature and humidity to allow for an hourly and daily rate of emission of the 3 gases. Animals were placed in the PAC on week 1 and week 9 as defined above.

12 adult ewes, at least 2 years old, were divided into 2 groupings of 6 sheep. One group received a 2.355 g dose of Urea-Hydrogen Peroxide (termed Group 1) per day and the other received a 4.71 g dose of Urea-Hydrogen Peroxide (termed Group 2) per day.

**Control Group:** Sheep were fed 0.5 kg of feed concentrate comprising animal feed, on a daily basis over a 7 week period.

**Treatment Group 1 (UHP 0.5X):** Sheep were fed 0.5 kg of feed concentrate comprising the same animal feed as the control group and 0.15% (by weight) of urea hydrogen peroxide, on a daily basis over a 7 week period.

**Treatment Group 2 (UHP 1X):** Sheep were fed 0.5 kg of feed concentrate comprising the same animal feed as the control group and 0.3% (by weight) of urea hydrogen peroxide, on a daily basis over a 7 week period.

Methane emissions per day (CH4/day) and methane emissions per body weight of animal (CH4/BW) can be seen in the following tables.

*Results*

The mean methane production for each animal is given below for weeks 1 and 7 of the study. Methane production is also given relative to the body weight (BW) of the animal (i.e. litres of methane per kg of animal body weight).

**Treatment Group 1**

|  |  | CH4/day | CH4/BW |
|---|---|---|---|
| Pen 17 | Week 1 | 41.5655 | 0.44218617 |
|  | Week 7 | 41.0679 | 0.408635821 |

(continued)

|  |  | CH4/day | CH4/BW |
|---|---|---|---|
| Pen 41 | Week 1 | 27.0445 | 0.321958333 |
|  | Week 7 | 25.5675 | 0.295578035 |
| Pen 71 | Week 1 | 27.233 | 0.358328947 |
|  | Week 7 | 28.2914 | 0.338819162 |
| Pen 90 | Week 1 | 32.3602 | 0.420262338 |
|  | Week 7 | 19.3246 | 0.238575309 |
| Pen 107 | Week 1 | 22.5221 | 0.317212676 |
|  | Week 7 | 23.5307 | 0.311664901 |
| Pen 112 | Week 1 | 25.215 | 0.362805755 |
|  | Week 7 | 27.9034 | 0.379638095 |

|  | CH4/day | Difference week 7 versus week 1 | CH4/BW | Difference week 7 versus week 1 |
|---|---|---|---|---|
| Week 1 | **29.32338333** |  | **0.370459037** |  |
| Week 7 | **27.61425** | 5.828% | **0.328818554** | 11.24% |

**Treatment Group 2**

|  |  | CH4/day | CH4/BW |
|---|---|---|---|
| Pen 21 | Week 1 | 33.0424 | 0.393361905 |
|  | Week 7 | 32.3578 | 0.371928736 |
| Pen 40 | Week 1 | 36.3662 | 0.420418497 |
|  | Week 7 | 29.5925 | 0.340143678 |
| Pen 51 | Week 1 | 32.9273 | 0.404015951 |
|  | Week 7 | 28.2905 | 0.330883041 |
| Pen 55 | Week 1 | 22.5261 | 0.3128625 |
|  | Week 7 | 10.0579 | 0.135005369 |
| Pen 93 | Week 1 | 26.9555 | 0.352359477 |
|  | Week 7 | 26.357 | 0.310082353 |
| Pen 128 | Week 1 | 27.1224 | 0.369012245 |
|  | Week 7 | 19.9919 | 0.254673885 |

|  | CH4/day | Difference week 7 versus week 1 | CH4/BW | Difference week 7 versus week 1 |
|---|---|---|---|---|
| Week 1 | **29.82331667** |  | **0.375338429** |  |
| Week 7 | **24.44126667** | 18.04% | **0.290452844** | 22.61% |

***Example 6 - In vivo feed efficiency studies***

*Experimental:* A study was conducted to determine what impact consumption of the feed additives would have on animal performance. It is estimated that between 2-12% of the energy content of animal feed is lost through enteric fermentation. This study examined whether though potential reductions in enteric fermentation additional energy would be diverted to the animal, with this being seen via an improved feed:gain metric, that is the ability of the animal to improve the rate of increase in its body weight per unit of feed eaten.

60 adult ewes (average starting body weight of approximately 80 kg) were divided into 3 equal groups of 20 sheep per group. Animals were fed as described in the Animal Study - Methane Emissions above. Animals were grouped into a control group (no feed additive given); treatment group 1 (2.355 g Urea-Hydrogen Peroxide given daily); treatment group 2 (4.71 g Urea-Hydrogen Peroxide given daily).

Animals were weighed at the start of the experiment (day 1) and at the end of the study (day 49). All feed (DMI) consumed was weighed and recorded throughout the study.

A feed:gain ratio was calculated as follows:

$$\text{weight gain} = \text{finishing weight} - \text{starting weight}$$

$$\text{feed:gain ratio} = \text{weight gain/daily DMI}$$

As seen in the following tables, this was calculated on an individual animal basis, and on a group basis.

*Results:* The weight of the animals at the start and end of the study are given below along with the average daily dry-matter intake (DMI) of the animal. The ratio of weight gain to DMI was then calculated to determine the feed efficiency of each animal.

| Control Grouping | | | |
|---|---|---|---|
| Sheep No. | Average Daily DMI (Kg) | Starting Weight (Kg) | Finishing Weight (Kg) |
| 1 | 2.03 | 85.5 | 89.5 |
| 2 | 1.68 | 98.5 | 97.5 |
| 3 | 1.89 | 86.5 | 92 |
| 4 | 1.48 | 96 | 100.5 |
| 5 | 1.61 | 83 | 87 |
| 6 | 1.3 | 81 | 83.5 |
| 7 | 1.57 | 88 | 92 |
| 8 | 1.83 | 82.5 | 85.5 |
| 9 | 1.37 | 77.5 | 81.5 |
| 10 | 1.81 | 78.5 | 84.5 |
| 11 | 1.51 | 74.5 | 83.5 |
| 12 | 1.71 | 78.5 | 87 |
| 13 | 1.28 | 72.5 | 75 |
| 14 | 1.6 | 79 | 84 |
| 15 | 1.7 | 74 | 80 |
| 16 | 1.79 | 70.5 | 73 |
| 17 | 1.55 | 69 | 74.5 |
| 18 | 1.54 | 70 | 74 |
| 19 | 1.57 | 65.5 | 76 |
| 20 | 1.4 | 66.5 | 72 |

| Treatment Group 1 | | | |
|---|---|---|---|
| Sheep No. | Average Daily DMI (Kg) | Starting Weight (Kg) | Finishing Weight (Kg) |
| 1 | 1.74 | 94 | 100.5 |
| 2 | 1.89 | 85 | 90 |
| 3 | 1.67 | 83.5 | 88 |
| 4 | 1.69 | 84.5 | 87 |
| 5 | 1.69 | 84 | 86.5 |
| 6 | 1.42 | 82 | 90.5 |
| 7 | 2.29 | 85.5 | 89.5 |
| 8 | 1.83 | 77.5 | 83.5 |
| 9 | 1.31 | 74.5 | 80 |
| 10 | 1.71 | 76 | 83.5 |
| 11 | 1.56 | 73 | 79.5 |
| 12 | 1.56 | 74 | 82 |
| 13 | 1.61 | 77 | 81 |
| 14 | 1.39 | 70.5 | 76 |
| 15 | 1.32 | 71 | 75.5 |
| 16 | 1.3 | 69.5 | 73.5 |
| 17 | 2.09 | 76.5 | 82 |
| 18 | 1.81 | 72 | 80.5 |
| 19 | 1.63 | 68 | 77.5 |
| 20 | 1.18 | 60 | 64.5 |

| Treatment Group 2 | | | |
|---|---|---|---|
| Sheep No. | Average Daily DMI (Kg) | Starting Weight (Kg) | Finishing Weight (Kg) |
| 1 | 1.28 | 93.5 | 98.5 |
| 2 | 1.41 | 87 | 90 |
| 3 | 1.48 | 84 | 92 |
| 4 | 1.48 | 84 | 87 |
| 5 | 1.37 | 86.5 | 87 |
| 6 | 1.28 | 85 | 85 |
| 7 | 1.6 | 81.5 | 85.5 |
| 8 | 1.05 | 72 | 74.5 |
| 9 | 1.27 | 58.5 | 73.5 |
| 10 | 2.14 | 80 | 90.5 |
| 11 | 1.68 | 75 | 81.5 |
| 12 | 1.72 | 76.5 | 85 |
| 13 | 1.2 | 71 | 76 |

(continued)

| Treatment Group 2 | | | |
|---|---|---|---|
| Sheep No. | Average Daily DMI (Kg) | Starting Weight (Kg) | Finishing Weight (Kg) |
| 14 | 1.65 | 65.5 | 72.5 |
| 15 | 1.84 | 72 | 79.5 |
| 16 | 1.49 | 68.5 | 75.5 |
| 17 | 1.57 | 73.5 | 78.5 |
| 18 | 1.72 | 62.5 | 77 |
| 19 | 1.83 | 60.5 | 65.5 |
| 20 | 1.26 | 62 | 71 |

| | Average Starting Weight (Kg) | Average Finishing Weight (Kg) | Average Weight Gain (Kg) | Average Daily DMI (Kg) | Gain: Feed Ratio |
|---|---|---|---|---|---|
| Control | 78.85 | 83.625 | 4.775 | 1.611 | 0.0741 |
| Treatment 1 | 76.9 | 82.55 | 5.65 | 1.6345 | 0.0864 |
| Treatment 2 | 74.95 | 81.275 | 6.325 | 1.516 | 0.1043 |

_Conclusions:_ The treatment groups which were administered a feed concentrate comprising urea hydrogen peroxide showed a significant decrease in methane production by week 7. The feed efficiency results demonstrate that the treatments did not negatively impact weight gain and therefore did not cause substantial negative impact digestibility of the feed. Notably, the animals in the treatment groups gained more weight on average than the control group, demonstrating that the invention has a positive impact on digestibility and feed efficiency. Without wishing to be bound by theory, by increasing ORP and reducing methanogenesis in the animal, the compositions and methods of the invention may divert more of the food weight to the animal rather than the methanogens in the digestive tract.

_Example 7 - Higher Dosage Animal Studies_

A further animal study based on the study described in Examples 5 and 6 is ongoing using higher doses of urea hydrogen peroxide in two further treatment groups. The higher dosage study involves trialling feed concentrate compositions comprising 0.6% urea hydrogen peroxide (UHP 2X) and 1.2% urea hydrogen peroxide (UHP 4X). It is expected that treatments comprising higher concentrations of urea hydrogen peroxide will be more effective in reducing methane production from ruminants whilst also retaining other beneficial properties such as not having a substantial negative impact on digestibility and potentially improving feed efficiency.

**_Example 8 - Ex vivo defaunation study_**

_Experimental:_ An ex _vivo_ study was conducted to investigate the effect of compositions of the invention on protozoa populations in rumen fluid. The study consisted of treating diluted fresh rumen fluid ("RF") with test compositions comprising urea hydrogen peroxide and potassium iodide ("UHP+KI") of increasing concentration and control compositions ("water" and "$N_2:CO_2$"), diluting the samples further, and then counting the protozoa population by microscopy. Experiments were repeated in triplicate. Experiments were carried out in sealed vials under an $N_2:CO_2$ (80:20) atmosphere.

_Results:_ As shown below, protozoa count was not substantially affected by treatments of the invention as compared to untreated samples. Furthermore, no trend was observed for decreased protozoa population with increasing test treatment concentration.

**Table 8.1 - Protozoa microscopy count results**

| Treatment | Dilution (1 in x) | Average (mean) count in 50 grids | Dilution factor | Protozoa count per ml |
|---|---|---|---|---|
| Time zero RF | 10 | 186.0 | 100 | 3.72E+05 |
| Time zero RF | 10 | 200.0 | 100 | 4.00E+05 |
| RF under $N_2$:$CO_2$ | 10 | 122.5 | 100 | 2.45E+05 |
| RF under $N_2$:$CO_2$ | 10 | 147.0 | 100 | 2.94E+05 |
| RF under $N_2$:$CO_2$ | 10 | 92.3 | 100 | 1.85E+05 |
| RF under $N_2$:$CO_2$ + water | 10 | 160.0 | 100 | 3.20E+05 |
| RF under $N_2$:$CO_2$ + water | 10 | 130.0 | 100 | 2.60E+05 |
| RF under $N_2$:$CO_2$ + water | 10 | 137.0 | 100 | 2.74E+05 |
| RF under $N_2$:$CO_2$ + 70mg/L UHP+KI | 10 | 143.3 | 100 | 2.87E+05 |
| RF under $N_2$:$CO_2$ + 70mg/L UHP+KI | 10 | 160.0 | 100 | 3.20E+05 |
| RF under $N_2$:$CO_2$ + 70mg/L UHP+KI | 10 | 170.3 | 100 | 3.41E+05 |
| RF under $N_2$:$CO_2$ + 80mg/L UHP+KI | 10 | 149.5 | 100 | 2.99E+05 |
| RF under $N_2$:$CO_2$ + 80mg/L UHP+KI | 10 | 155.5 | 100 | 3.11E+05 |
| RF under $N_2$:$CO_2$ + 80mg/L UHP+KI | 10 | 150.0 | 100 | 3.00E+05 |
| RF under $N_2$:$CO_2$ + 90mg/L UHP+KI | 10 | 143.0 | 100 | 2.86E+05 |
| RF under $N_2$:$CO_2$ + 90mg/L UHP+KI | 10 | 146.7 | 100 | 2.93E+05 |
| RF under $N_2$:$CO_2$ + 90mg/L UHP+KI | 10 | 173.7 | 100 | 3.47E+05 |
| RF under $N_2$:$CO_2$ + 130mg/L UHP+KI | 10 | 168.7 | 100 | 3.37E+05 |
| RF under $N_2$:$CO_2$ + 130mg/L UHP+KI | 10 | 151.0 | 100 | 3.02E+05 |
| RF under $N_2$:$CO_2$ + 130mg/L UHP+KI | 10 | 149.5 | 100 | 2.99E+05 |

*Conclusions:* Surprisingly, there is no reduction in protozoa count associated with the test treatments. Moreover, there is no relationship between increasing the concentration of test compositions and any reduction in protozoa count. Therefore, it would be expected that compositions of the invention do not appear to substantially defaunate the rumen at the tested concentrations.

In this experiment, potassium iodide was used. Without wishing to be bound by theory, potassium iodide is expected to facilitate the decomposition of the urea hydrogen peroxide and the resulting release of oxygen. Similar results are expected for treatment compositions of the invention which are substantially free of potassium iodide, since the other experiments herein demonstrate that similar release of oxygen is possible in the absence of potassium iodide.

This experiment also shows that exposure of the rumen fluid to a mixture of atmospheric gases ($N_2$ and $CO_2$)

36

was associated with a decrease in protozoa count. This suggests that exposure of the rumen fluid to atmospheric gases, for example via a fistula, may be linked to a reduction in protozoa count, i.e. defaunation.

***Example 9 - Further ORP assays and data***

*Experimental:* An *in vivo* study was conducted to investigate the effect of feed additives of the invention on rumen ORP in cattle. The study involved administering an ORP measurement bolus to each cow to allow for an ORP reading to be recorded every 10 minutes, dividing the cattle into three groups: a control group (receiving feed without any feed additive of the invention), low dose (receiving 100 g $CaO_2$ per day along with feed - with the additive being delivered at a single morning feeding) and a high dose group (receiving 200 g $CaO_2$ per day along with feed - with the additive being delivered at a single morning feeding). The rumen was opened after approximately 450 hours.

*Results:*

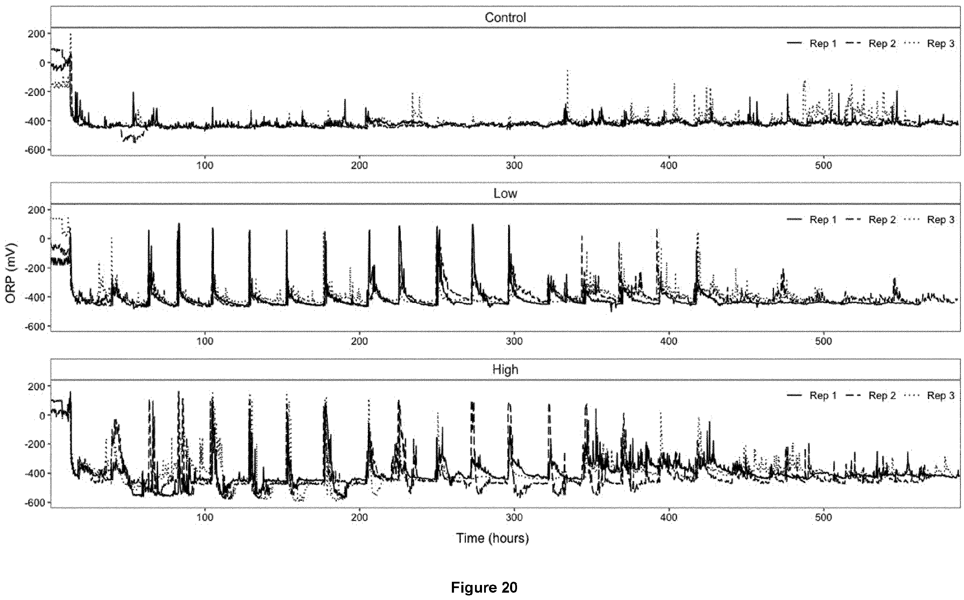

The results of this study are given in Figure 20 which charts the ORP level in the rumen (mV) over time measured at 10-minute intervals for the control group, the low-dose group, and the high-dose group respectively.
An average baseline ORP reading measured for the animals from the control group is about -440 mV. This average baseline ORP reading of about -440 mV may be useful to aid comparison between control ORP and increases in ORP due to the animals consuming the additive. After approximately 450 hours the rumen was opened and the ORP results after this time point should be disregarded.
After each morning feed, ORP in the rumen increases by at least +100 mV for a sustained period of at least one hour.
For the treated animals, after multiple feed cycles a cumulative effect was observed such that the duration of sustained ORP elevation became longer with each subsequent feeding.
In addition, with increasing number of feed cycles, the baseline for the treated animals gradually increased above the baseline observed in the control group. This effect is particularly pronounced in the high dosage group. A "correction phase" may be observed over the initial period of the first about 300-350 hours, since the ORP profile shows a general increase, with specific increases after each administration, while there are temporary reductions in ORP below baseline between administrations.
A "maintenance phase" may be observed after the initial period of the first about 300-350 hours until the end of the study, and in this phase the ORP profile shows a general increase, with specific increases after each administration, and there are no substantial reductions in ORP below baseline between administrations.
*Conclusions:* The observed ORP profile *in vivo* is consistent with ORP profiles generated with the RUSITEC *ex vivo* assay, in which a strong correlation between increased ORP and reduced methane emissions was also observed. Therefore, a corresponding reduction in methane emissions in the treated animals is expected.
The observation that the baseline ORP for treated animals gradually increased over time suggests that the treatments of the invention a sustained shift to a higher baseline ORP.

***Example 10- Comparison of*** **ex** ***vivo (RUSITEC) assays with in vivo ORP measurements***

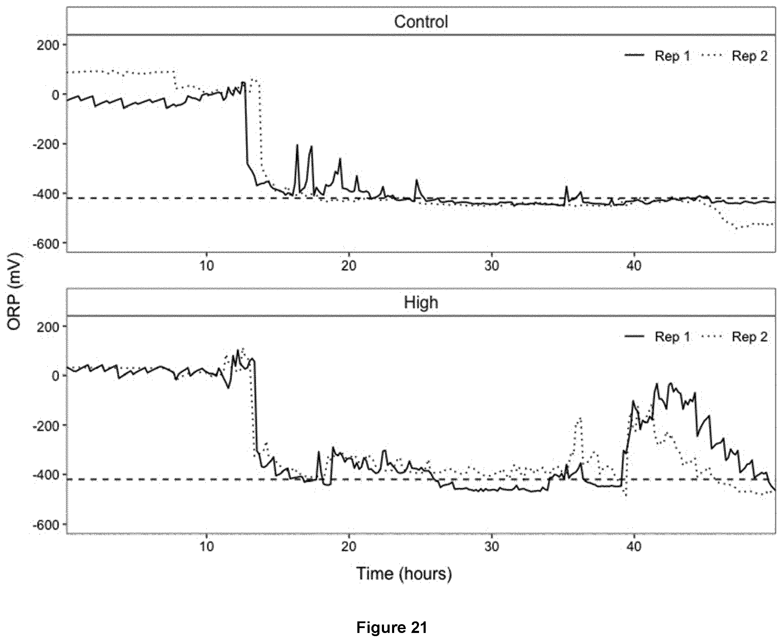

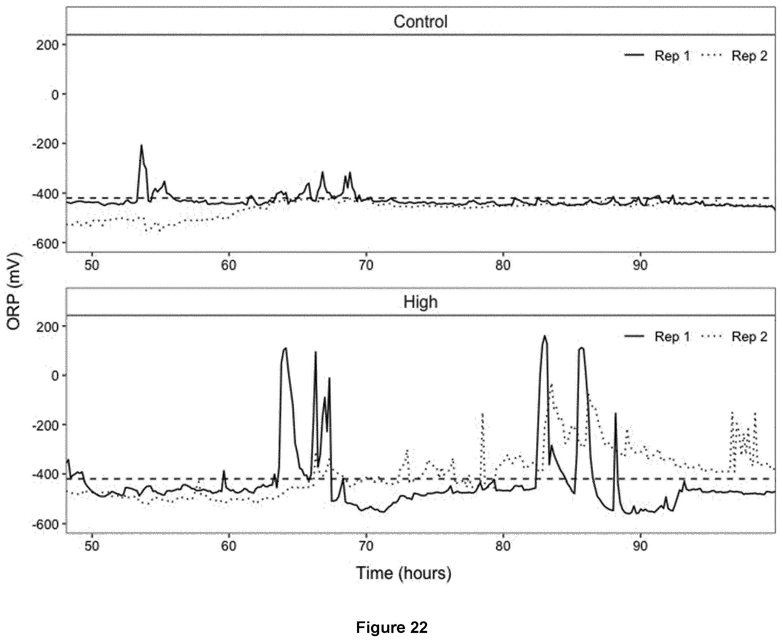

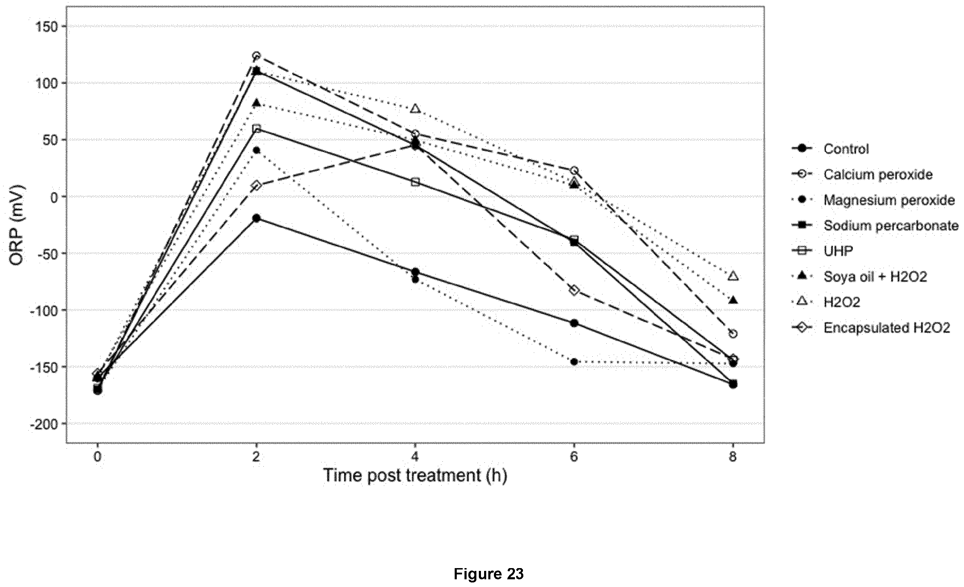

Further ex *vivo* (RUSITEC) and *in vivo* studies were conducted to investigate the correlation between the effects of the feed additives of the invention on rumen ORP in cattle and the effects observed in RUSITEC experiments (such as those described in Examples 1 and 2).
*Experimental 10.1:* The *in vivo* study involved administering the same ORP measurement bolus as described in Example 9, to four cows. The two cows designated as B423 and B419 were fed with 221.7 g calcium peroxide ($CaO_2$) per day incorporated into 3 kg of conventional feed concentrate mix. The two cows designated as B420 and B421 were control animals, which were fed only the 3 kg conventional feed concentrate mix per day, absent the additive of the invention. ORP measurement boluses entered the rumen at about hour 13, and administration of feed (with or without additive) to the animals commenced at about hour 18.
*Results 10.1:* Measured ORP levels of the rumen (y-axis) in the studied cattle are shown as a function of time (x-axis) in Figures 21 and 22.
*Experimental 10.2:* For comparison with the *in vivo* data, various RUSITEC experiments as described in Examples 1 and 2 were set up and ORP was measured for a 24-hour period on day 16 and several different oxidising agents were tested, including calcium peroxide. ORP in the RUSITEC rumen system was measured at regular intervals.
*Results 10.2:* For each of the tested oxidising agents, ORP (mV, x-axis) is plotted as a function of time (hours, y-axis) in Figure 23.
*Conclusions:* The ORP profile *in vivo* is comparable to the profile observed in the RUSITEC ex *vivo* model for the same inclusion rate (i.e. equivalent dose) of calcium peroxide This confirms that the ex *vivo* model (RUSITEC) is representative of rumen response.

Various modifications of the invention, in addition to those described herein, will be apparent to those skilled in the art from the foregoing description without departing from the spirit and scope of the invention. Such modifications are also intended to fall within the scope of the appended claims. All references, including all patent, patent applications, and publications, cited in the present application are incorporated herein by reference in their entirety.

The invention also relates to the following numbered clauses:

1. A method of reducing methane production from an animal by increasing the oxidation-reduction potential in the stomach of the animal, the method comprising orally administering a composition comprising an oxidising agent to the animal.

2. A method of reducing methane production from an animal, the method comprising orally administering a composition comprising an oxidising agent to the animal, wherein the composition is administered to the animal in a dose which provides an increase in the oxidation-reduction potential of the stomach of at least about + 100 mV for a time period of at least about 1 hour.

3. A composition in the form of a bolus for oral administration to an animal comprising:

    (i) an oxidising agent;
    (ii) excipients; and
    (iii) a coating.

4. A composition in the form of pellets comprising:

    (i) urea hydrogen peroxide or magnesium peroxide or a combination thereof;
    (ii) food components; and
    (iii) a coating.

5. The method of any preceding clause wherein the composition is substantially free of iodide or a source of iodide.

6. The method or composition of any preceding clause wherein the composition comprises a peroxidase or catalase, a peroxidase-producing organism, or a non-biological catalyst which catalyses the decomposition of peroxide.

7. The method or composition of any preceding clause in the second set wherein the oxidising agent is an enzymatic oxidising agent system.

8. The method of any preceding clause wherein the animal is a ruminant.

9. The method of any preceding clause in the second set wherein the animal is a cow.

10. The method of clause 1 wherein the animal is a ruminant and the stomach is the rumen.

11. The method of any preceding clause wherein the method does not substantially reduce digestibility of food in the stomach.

12. The method of any preceding clause wherein the method increases feed efficiency.

13. The method of any preceding clause wherein the method reduces methane by inhibiting methanogens without substantially defaunating the stomach.

14. The method or composition of any preceding clause wherein the oxidising agent is a source of oxygen ($O_2$).

15. The method or composition of clause 14 wherein the source of oxygen is a source of peroxide.

16. The method or the composition of any preceding clause, wherein the oxidising agent is a source of oxygen selected from hydrogen peroxide, urea hydrogen peroxide, sodium percarbonate, magnesium peroxide, sodium peroxide, lithium peroxide and calcium peroxide, or a combination thereof.

17. The method or composition of any preceding clause, wherein the oxidising agent is urea hydrogen peroxide.

18. The method or composition of any preceding clause, wherein the oxidising agent is calcium peroxide.

19. The method or composition of any preceding clause, wherein the oxidising agent is peroxide-producing bacteria; optionally wherein the peroxide-producing bacteria is lactic acid bacteria.

20. The method of any preceding clause, wherein the method comprises adding the composition to food prior to oral administration.

21. The method of any preceding clause wherein the method comprises the steps of: (i) sprinkling the composition onto food prior to feeding; and (ii) feeding the mixture of food and composition to the ruminants.

22. The method or composition of any preceding clause, wherein the composition further comprises food components, optionally wherein the food components comprise maize, barley, soya, and/or molasses.

23. The composition or method according to any preceding clause wherein the composition comprises a coating comprising coconut oil, gylcerin, glycerol, silica hydrogels, poly(methyl methacrylate) capsulation, poly(D,L-lactide-co-glycolide) (PLGA), alginate, poly(vinyl pyrrolidone) PVP, hard fat, ethyl cellulose, N-isopropylacrylamide (NIPAAm), acrylic acid, hydroxyethyl methancrylate-oligo(hydroxybutyrate), polydimethylsiloxane (PDMS), methacrylamide chitosan, cyanoacrylate, sodium alginate, polyisobutylene, isobutyleneisoprene copolymers, styrene-butadiene copolymers, polyvinyl acetate, polyisoprene, polyethylene, vinyl acetate, or a combination thereof.

24. The composition or method according to any preceding clause wherein the composition comprises a coating comprising coconut oil.

25. The composition according to any preceding clause wherein the composition comprises iron oxide, zinc oxide, magnesium stearate, hydrogenated fat, or combinations thereof.

26. The method of any preceding clause wherein the composition increases the oxidation-reduction potential in the stomach of the animal at least about +100 mV.

27. The method of any preceding clause wherein oral administration of the composition elevates oxidation-reduction potential in the stomach of the animal for a period of at least about 1 hour following oral administration.

28. The method of any preceding clause wherein oral administration of the composition induces and maintains an oxidation-reduction potential in the stomach of from about -200 mV to about -100 mV for at least about 1 hour.

29. The method of any preceding clause wherein oral administration of the composition does not reduce pH of the stomach by more than 10%.

30. The method of any preceding clause, wherein the method further comprises administration of an additional anti-methanogenic agent.

31. The method of clause 30 wherein the additional anti-methanogenic agent is 3-nitrooxypropanol.

32. The composition or method of any preceding clause wherein about 0.01 to about 0.1% by weight of the composition is the oxidising agent.

33. The composition or method of any preceding clause wherein about 0.05% to about 10% by weight of the composition is the oxidising agent; preferably about 0.1% to about 5%; preferably about 0.5% to about 2.5%; preferably about 1% to about 2%.

34. The composition or method of any preceding clause wherein substantially all of the composition is the oxidising agent.

35. The method of any preceding clause wherein the composition used is the composition of any preceding clause.

## Claims

1. Use of a composition comprising an oxidising agent for improving animal performance, the use comprising orally administering the composition comprising an oxidising agent to an animal;

   wherein the composition is substantially free of iodide or a source of iodide; and
   wherein the oxidising agent is a source of oxygen selected from urea hydrogen peroxide, sodium percarbonate, magnesium peroxide, sodium peroxide, lithium peroxide and calcium peroxide, or a combination thereof, or is an enzymatic oxidising agent system, or is peroxide-producing bacteria.

2. The use of any preceding claim wherein improving animal performance comprises one or more of: increasing milk production, increasing animal weight gain, improving milk quality from the animal, increasing volatile fatty acid content in the stomach of the animal, reducing finishing times for the animal, improving carcass quality, improving feed efficiency, and improving a feed recuperation ratio.

3. The use of any preceding claim wherein the animal is a ruminant, for example, a cow, and optionally wherein the stomach is the rumen.

4. The use of any preceding claim, wherein the oxidising agent is calcium peroxide.

5. The use of any preceding claim, wherein the use comprises adding the composition to food prior to oral administration; for example, wherein the method comprises the steps of: (i) sprinkling the composition onto food prior to feeding; and (ii) feeding the mixture of food and composition to the ruminants.

6. The use of any preceding claim, wherein (i) the use involves administration of a composition, for example, a bolus; or (ii) the animal's diet consists essentially of feed including a composition of the invention; or (iii) the animal's diet consists essentially of a composition of the invention; or (iv) the administration step is the animal feeding.

7. The use of any preceding claim, wherein the composition further comprises food components, optionally wherein the food components comprise maize, barley, soya, and/or molasses.

8. The use according to any preceding claim wherein the composition comprises a coating comprising coconut oil, gylcerin, glycerol, silica hydrogels, poly(methyl methacrylate) capsulation, poly(D,L-lactide-co-glycolide) (PLGA), alginate, poly(vinyl pyrrolidone) PVP, hard fat, ethyl cellulose, N-isopropylacrylamide (NIPAAm), acrylic acid, hydroxyethyl methancrylate-oligo(hydroxybutyrate), polydimethylsiloxane (PDMS), methacrylamide chitosan, cyanoacrylate, sodium alginate, polyisobutylene, isobutyleneisoprene copolymers, styrene-butadiene copolymers, polyvinyl acetate, polyisoprene, polyethylene, vinyl acetate, or a combination thereof; preferably, wherein the composition comprises a coating comprising coconut oil.

9. The use of any preceding claim wherein the animal is a ruminant and oral administration of the composition does not reduce pH of the rumen below about 6.

10. The use of any preceding claim, wherein the method further comprises administration of an additional anti-methanogenic agent; for example, wherein the additional anti-methanogenic agent is 3-nitrooxypropanol.

11. The use of any preceding claim wherein about 0.01 to about 0.1% by weight of the composition is the oxidising agent; for example, wherein about 0.05% to about 10% by weight of the composition is the oxidising agent; preferably about 0.1% to about 5%; preferably about 0.5% to about 2.5%; preferably about 1% to about 2%.

12. The use of any preceding claim wherein substantially all of the composition is the oxidising agent.

13. The use of any preceding claim wherein the use increases non-methanogenic activity which generates volatile fatty acids from excess hydrogen rather than methane.

14. The use of any preceding claim wherein reducing methanogenic activity is accompanied by an increase in non-methanogenic activity which generates volatile fatty acids, for example, acetogenic activity.

15. The use of any preceding claim wherein the use:

   (i) increases non-methanogenic microorganism populations in the rumen; and/or
   (ii) inhibits methanogens.

16. A method of reducing methane production from a ruminant, optionally in the use of and preceding claim, the method comprising orally administering a composition comprising an oxidising agent and substantially free of iodide or a source of iodide to the ruminant, wherein the composition is administered to the ruminant in a dose which provides an increase in the oxidation-reduction potential of the stomach of at least about + 10 mV for a time period of at least about 1 hour.

Figure 1 – RUSITEC Composition Screening 1st Run - Gas evolution results

Figure 2 – RUSITEC Composition Screening 1st Run – Comparison of mean daily $CH_4$ volume evolved (L/day)

Figure 3 – RUSITEC Composition Screening 1st Run – Comparison of mean daily total gas volume evolved (L/day)

**Figure 4 – RUSITEC Composition Screening 1st Run - Comparison of daily amount of CH₄ evolved (mmol/day)**

Figure 5 – RUSITEC Composition Screening 1st Run - Comparison of $NH_3$ concentration per litre (mM)

Figure 6 – RUSITEC Composition Screening 1st Run – Vessel and overflow fluids acidity measurements (pH)

**Figure 7 – RUSITEC Composition Screening 1st Run – Dry-matter digestibility results**

Figure 8 – RUSITEC Composition Screening 1st Run – Forage digestibility (%)

Figure 9 – RUSITEC Composition Screening 1st Run – Feed composition digestibility (%)

Figure 10 – RUSITEC Composition Screening 1st Run – Average digestibility (%)

Figure 11 – RUSITEC Composition Screening 2nd Run - Gas evolution results

Figure 12 – RUSITEC Composition Screening 2nd Run – Comparison of mean daily CH₄ volume evolved (L/day)

Figure 13 – RUSITEC Composition Screening 2nd Run – Comparison of mean daily total gas volume evolved (L/day)

Figure 14 – RUSITEC Composition Screening 2nd Run – Comparison of daily amount of $CH_4$ evolved (mmol/day)

Figure 15 – RUSITEC Composition Screening 2nd Run – Vessel and overflow fluids acidity measurements (pH)

**Figure 16 – RUSITEC Composition Screening 2nd Run – Dry-matter digestibility results**

Figure 17 – RUSITEC Composition Screening 2nd Run – Forage digestibility (%)

Figure 18 – RUSITEC Composition Screening 2nd Run – Feed composition digestibility (%)

Figure 19 – RUSITEC Composition Screening 2nd Run – Average digestibility (%)

Figure 20

Figure 21

Figure 22

Figure 23

63

**REFERENCES CITED IN THE DESCRIPTION**

**Patent documents cited in the description**

- WO 2020074672 A

- WO 2012140272 A

**Non-patent literature cited in the description**

- **CZERKAWSKI ; BRECKENRIDGE.** *British Journal of Nutrition,* 1997, vol. 38 (3), 371-384

- **MCDOUGALL.** *Biochemical Journal,* 1948, vol. 43 (1), 99-109